(19) Europäisches Patentamt
European Patent Office
Office européen des brevets

(11) EP 3 914 698 B1

(12) **EUROPEAN PATENT SPECIFICATION**

(45) Date of publication and mention of the grant of the patent:
**11.12.2024 Bulletin 2024/50**

(21) Application number: **20704611.1**

(22) Date of filing: **23.01.2020**

(51) International Patent Classification (IPC):
***C12N 5/0735*** *(2010.01)* ***C12N 5/074*** *(2010.01)*

(52) Cooperative Patent Classification (CPC):
**C12N 5/0606; C12N 5/0696;** C12N 2501/16; C12N 2501/235; C12N 2501/415; C12N 2501/42; C12N 2501/727; C12N 2501/999; C12N 2510/00

(86) International application number:
**PCT/IL2020/050095**

(87) International publication number:
**WO 2020/152686 (30.07.2020 Gazette 2020/31)**

(54) **CULTURE MEDIA FOR PLURIPOTENT STEM CELLS**

KULTURMEDIEN FÜR PLURIPOTENTE STAMMZELLEN

MILIEUX DE CULTURE POUR CELLULES SOUCHES PLURIPOTENTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.01.2019 US 201962795626 P**

(43) Date of publication of application:
**01.12.2021 Bulletin 2021/48**

(73) Proprietor: **Yeda Research and Development Co. Ltd**
**7610002 Rehovot (IL)**

(72) Inventors:
- **HANNA, Yaqub**
  **6803450 Tel Aviv-Yafo (IL)**
- **NOVERSHTERN, Noa**
  **7610002 Rehovot (IL)**
- **SHANI, Tom Haim**
  **7610002 Rehovot (IL)**
- **VIUKOV, Sergey**
  **7610002 Rehovot (IL)**
- **MASSARWA, Rada**
  **7610002 Rehovot (IL)**
- **BAYERL, Jonathan**
  **7610002 Rehovot (IL)**
- **AYYASH, Muneef**
  **7610002 Rehovot (IL)**

(74) Representative: **Dennemeyer & Associates S.A.**
**Postfach 70 04 25**
**81304 München (DE)**

(56) References cited:
**WO-A1-2017/164746 WO-A2-2015/196072**
**WO-A2-2016/027099**

- **BAYERL JONATHAN ET AL: "Principles of signaling pathway modulation for enhancing human naive pluripotency induction", CELL STEM CELL, ELSEVIER, CELL PRESS, AMSTERDAM, NL, vol. 28, no. 9, 28 April 2021 (2021-04-28), pages 1549, XP086761543, ISSN: 1934-5909, [retrieved on 20210428], DOI: 10.1016/J.STEM.2021.04.001**
- **THOROLD?W. THEUNISSEN ET AL: "Systematic Identification of Culture Conditions for Induction and Maintenance of Naive Human Pluripotency", CELL STEM CELL, vol. 15, no. 4, 1 October 2014 (2014-10-01), AMSTERDAM, NL, pages 471 - 487, XP055237395, ISSN: 1934-5909, DOI: 10.1016/j.stem.2014.07.002**
- **VORONKOV ET AL: "Wnt/beta-Catenin Signaling and Small Molecule Inhibitors", CURRENT PHARMACEUTICAL DESIGN,, vol. 19, 1 December 2012 (2012-12-01), pages 634 - 664, XP002764558**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MASAKI KINOSHITA ET AL: "Pluripotency Deconstructed", DEVELOPMENT GROWTH AND DIFFERENTIATION., vol. 60, no. 1, 1 January 2018 (2018-01-01), US, pages 44 - 52, XP055455321, ISSN: 0012-1592, DOI: 10.1111/dgd.12419**
- **GAO XUEFEI ET AL: "Establishment of porcine and human expanded potential stem cells", NATURE CELL BIOLOGY, MACMILLAN MAGAZINES LTD, GB, vol. 21, no. 6, 3 June 2019 (2019-06-03), pages 687 - 699, XP036796369, ISSN: 1465-7392, [retrieved on 20190603], DOI: 10.1038/S41556-019-0333-2**


Remarks:

•The complete document including Reference Table(s) and the Sequence Listing(s) can be downloaded from the EPO website

•The file contains technical information submitted after the application was filed and not included in this specification

## Description

The present disclosure, in some aspects thereof, relates to culture media for culturing pluripotent stem cells more particularly, but not exclusively, to naive pluripotent stem cells.

A continuum of pluripotent configurations represents changes occurring during *in vivo* transition of naive pre-implantation pluripotency toward that of primed post-implantation pluripotent state, can be captured *in vitro* to various extents. Many naive and primed pluripotency properties can be individually characterized and attributed to pluripotent stem cells expanded in distinct conditions. In mice, defined serum free 2i/LIF conditions have been extensively characterized where many naive molecular and functional properties are endowed by these conditions. The latter include global DNA hypomethylation, loss of bivalency over developmental genes, exclusive nuclear localization of TFE3 transcription factor, tolerance of lack of exogenous L-glutamine, tolerance for loss of repressors like DNMT1, METTL3 and DGCR8 (or DICER). Mouse ESCs expanded Fetal Bovine Serum (FBS)/Lif are also considered naive and possess features such as retention of pre-X inactivation state, ability to tolerate lack of repressors like Mettl3, Dnmt1 and Dgcr8. However, they do not retain a global hypermethylated epigenome, and acquire H3K27me3 over developmental genes consistent with retaining a relatively less naive state. Rodent EpiSCs expanded in Fgf2/Activin A show further consolidation and acquisition of their milieu of primed pluripotency characteristics, thus exemplifying how mouse naive and primed PSCs can have different mix of naive and primed pluripotent states. EpiSC lines are heterogeneous in their epigenetic and transcriptional patterns, and while they are pluripotent and give rise to differentiated cells from all three germ layers, they are epigenetically restricted as evident for example in their reduced ability, after long term/permanent maintenance in FGF2/ACTIVIN A conditions, to differentiate into primordial germ cells (PGCs) or contribute to chimera formation when injected in the pre-implantation ICM.

While conventional human embryonic stem cells (hESCs) and iPSCs (hiPSCs) growth conditions entailed FGF/TGFB as typical for murine EpiSC, these two cell types are not identical, and hESC share several molecular features with naive mESCs including expression of E-CADHERIN (rather than N-CADHERIN). Further, conventional human ESCs express high levels of PRDM14 and NANOG as murine naive ESCs, and they are functionally dependent on their expression. Still however, hESCs retain a variety of epigenetic properties that are consistent with possessing a primed pluripotent state. This includes inability to tolerate MEK/ERK signaling inhibition, predominant (yet non-exclusive) utilization of the proximal enhancer element to maintain OCT4 expression, tendency for initiation of X chromosome inactivation in most female ESC lines, pronounced increase in DNA methylation, prominent deposition of H3K27me3 and bivalency acquisition on lineage commitment regulators.

The ability of human zygotes to develop into blastocysts in the presence of MEK/ERKi and the proof of concept for the metastability between naive and primed state in rodents, have raised the possibility that the human genetic background is more "stringent" in regards to requirement for exogenous factors provided in allowing preservation of ground state-naive pluripotency in comparison to rodents.

Condition to derive naive MEK/ERK signaling-independent, genetically unmodified human pluripotent cells via iPSC generation, from established conventional ESC lines or directly from human blastocysts are described in WO2016/016894. Specifically, NHSM conditions do not require the use of exogenous transgenes or feeder cells, maintain teratoma formation competence and entail the following components: LIF, 2i, P38i/JNKi, PKCi, ROCKi, TGFB1/ACTIVIN A and FGF2. NHSM conditions endow human PSCs with variety with naïve features including maintain pluripotency while MEK/ERK signaling is inhibited, predominant TFE3 nuclear localization, resolution of bivalent domains over developmental regulators, *in vitro* reconstitution of human PGCLC and a mild reduction of demethylation. The latter effect was profoundly weaker than that seen in mouse pluripotent cells, suggesting sub-optimal human naïve pluripotency growth conditions.

Theunissen et al., 2014; Cell Stem Cell 1-17, describe alternative conditions that generate MEK independent human naïve cells and retain a more compelling milieu of transcriptional markers expressed in the human ICM. Several components found in NHSM conditions (2i, ROCK inhibitor, FGF/ACTIVIN) were supplemented with BRAF inhibitors, to generate MEF obligatory dependent naive cell lines (different conditions termed: 5iLA-, 5iLAF-, 6i/LA- and 4i/LA-MEF conditions). Globally these conditions generated more pronounced downregulation in DNA methylation and upregulation of naive pluripotent cell markers. However, the hypomethylation in these conditions is however accompanied by immediate and global deterministic loss of imprinting (Theunissen, 2016; Cell Stem Cell 1-49) and obligatory confounding chromosomal abnormalities in nearly 100% of the line generated by 10 passages only (Liu et al., 2017, Nat. Methods 14, 1-14).

Derivation of human naive ESC in t2iL-Go conditions has been reported, however these results have not yet been reproduced without exogenous transgenes. In both cases, the reported cell line do not form teratomas *in vivo* and can only differentiate *in vitro* after an extended 2-week transfer to primed conditions, thus questioning their pluripotent functionality and stability (Guo et al., 2016, Stem Cell Reports 1-19; Liu et al., 2017, Nat. Methods 14, 1-14; Takashima et al., 2014, Cell 158, 1254-1269). The latter is in striking difference from rodent ground state naïve PSCs, which are fully pluripotent and can initiate differentiation in vivo following autologous induction of the needed priming signals toward differentiation.

Additional background art includes WO2014/174470.

THOROLD W. THEUNISSEN ET AL, relates to "Systematic identification of Culture Conditions for Induction and Maintenance of Naive Human Pluripotency", CELL STEM CELL, AMSTERDAM, NL, (20141001), vol. 15, no. 4.

VORONKOV ET AL, relates to "Wnt/beta-Catenin Signaling and Small Molecule Inhibitors", CURRENT PHARMACEUTICAL DESIGN" (20121201), vol. 19, pages 634 - 664.

MASAKI KINOSHITA ET AL, relates to "Pluripotency Deconstructed", DEVELOPMENT GROWTH AND DIFFERENTIATION., US, (20180101), vol. 60, no. 1.

GAO XUEFEI ET AL, relates to "Establishment of porcine and human expanded potential stem cells", NATURE CELL BIOLOGY, MACMILLAN MAGAZINES LTD, GB, vol. 21, no. 6, pages 687 - 699.

## SUMMARY OF THE INVENTION

The present invention is set out in the appended set of claims.

## BRIEF DESCRIPTION OF THE SEVERAL VIEWS OF THE DRAWINGS

Some aspects of the disclosure are herein described, by way of example only, with reference to the accompanying drawings. With specific reference now to the drawings in detail, it is stressed that the particulars shown are by way of example and for purposes of illustrative discussion of aspects of the disclosure. In this regard, the description taken with the drawings makes apparent to those skilled in the art how aspects of the disclosure may be practiced.

In the drawings:

FIGs. 1A-I Defining enhanced human naive conditions compatible with expanding METTL3 depleted PSCs. A. Strategy for generating human ESCs with TET-OFF regulated expression of METTL3 $m^6A$ methyltransferase enzyme. B. Western blot analysis for correctly engineered human ESCs with TET-OFF METTL3 regulation, before and after DOX treatment. C. Representative images for human METTL3 TET-OFF engineered cells in different conditions with or without DOX addition. Previously described primed and naive conditions do not support maintain their pluripotency and viability when DOX is added (METTL3 is depleted). D. Scheme depicting strategy for conducting a screen for identifying a small molecule or cytokine additive to previously described human naive NHSM conditions that allow maintain pluripotency in TET-OFF METTL3 human stem cells after adding DOX. E. W3G4 cells were maintained in the presence of DOX for up to 4 passages in different conditions and stained for OCT4 to quantify percentage of cells that retained their pluripotency. Graph shows that supplementing NHSM conditions with an inhibitor for Tankyrase (that blocks WNT signaling) allows maintain pluripotency in majority of cells expanded (>75% positive OCT4 staining). F. OCT4+ pluripotency maintenance in NHSM conditions supplemented with various TNK inhibitors and DOX to repress METTL3 expression. G. Quantification of ΔPE-OCT4-GFP knock in naive pluripotency reporter, in variety of primed (red) and naive conditions (blue). Mean fluorescence intensity values (MFI) are indicated. H. Quantification of ΔPE-OCT4-GFP knock in naive pluripotency reporter in variety of conditions with various concentrations of ACTIVIN A recombinant cytokine. Figure shows that in NHSM+TNKi conditions the naivety of human ESCs is still dependent on ACTIVIN A supplementation. I. Representative phase contrast images in human ESCs expanded in NHSM+TNKi conditions showing their maintenance of pluripotent domed-like morphology even in the presence of FGFRi. However, upon blocking of TGF/ACTIVIIN A signaling (with A83-01 designated as TGFRi), the cells in NHSM+TNKi lose their pluripotent dome-like shaped morphology and differentiate.

FIGs. 2A-I. Optimizations for enhanced human naive pluripotency conditions. A. W3G4 METTL3 TET-OFF cells were maintained in the presence of DOX for up to 4 passages in different conditions and stained for OCT4 to quantify percentage of cells that retained their pluripotency. Graph shows that NHSM conditions without feeder cells and other previously described naive and primed conditions for human ESCs/iPSCs failed to maintain pluripotency in majority of cells expanded in the presence of Dox. B. Quantification of ΔPE-OCT4-GFP knock in naive pluripotency reporter, in variety of primed (red) and naive conditions (blue). Mean fluorescence intensity values (MFI) are indicated. Figure shows that supplementing NHSM conditions with TNKi like IWR1 small molecules boosts expression of GFP suggesting enhancement of naivety characteristics. C. Quantification of ΔPE-OCT4-GFP knock in naive pluripotency reporter, in optimized naive conditions and various concentrations of GSK3 inhibitor that leads to WNT activation (CHIR99021 is used as GSK3 inhibitor and is abbreviated as CHIR). Figure indicates that CHIR addition negatively influences human naive pluripotency as determined by ΔPE-OCT4-GFP intensity. D. Phase images showing how supplementation of 0.2% Geltrex (Life Technologies) in the growth media and SRCi additively enhance domed like morphology of human naive PSCs in ENHSM conditions optimized herein. E. RT-PCR analysis for naïve pluripotency markers in ENHSM conditions with and without P38i/JNKi (BIRB0796). Values were normalized to ACTIN and values in Primed conditions were set as 1. Figure indicates that use of BIRB0796 as P38i/JNKi boosts expression of naive pluripotency markers. F. W3G4 METTL3 TET-OFF cells were maintained in the presence of DOX for up to 4 passages in the optimized ENHSM conditions but without TNKi, in order to see if other molecules can substitute for TNKi after all optimizations were applies (e.g. adding of Geltrex, concentration optimization). G. FACS analysis for OCT4-GFP pluripotency reporter expression following addition of TGFRi. In optimized NHSM conditions that still lack SRCi, pluripotency is entirely and rapidly lost upon inhibition of TGFRi. H. Efficiency of generating KO and targeting DGCR8 in human primed (TeSR) and ENHSM naive conditions. 2 replicates for targeting were done for each growth conditions. Only in ENHSM conditions we recovered DGRC8 KO clones based on genotyping and western blot analysis. I. RT-PCR analysis for the indicated microRNA in DGCR8 WT and KO human ESCs. Figure shows loss of microRNA expression in DGCR8 KO human naive ENHSM conditions as expected following ablation of DGCR8.

FIGs. 3A-I. Defining enhanced human naive conditions compatible with blocking TGF/ACTIVIN signaling. A. Scheme depicting strategy for conducting a screen for identifying a small molecule or cytokine additive to optimized NHSM conditions after addition of TNKi, allow to maintain pluripotency in TET-OFF METTL3 human stem cells (clone W3G4) and without supplementing exogenous TGF or ACTIVIN A. B. OCT4+ pluripotency maintenance in optimized NHSM conditions without TGF/ACTIVIN, indicated that supplementing SRCi CGP77675 allows maintain OCT4+ cells in optimized HSM conditions and without METTL3 expression. C. Summary of small molecules and their concentrations used in the optimized ENHSM conditions used herein (ENHSM can be also named 6iL conditions because we use 6 inhibitors + LIF). D. Representative phase contrast images showing naive domed-like morphology of human ESCs expanded in ENHSM conditions, that is maintained even when TGFRi small molecule A83-01 is supplemented. E. Immunostaining of W3G4 cells in ENHSM conditions with and without DOX. Cells expressed canonical (OCT4-SSEA4) and naive pluripotency specific markers like KLF17 in both conditions. F. Mass spectrometry-based quantification of $m^6A$ on isolated mRNA from the indicated cell lines and conditions. Depletion of $m^6A$ in human cells was validated in ENHSM + DOX conditions. G. Mature teratoma obtained following injection of METTL3 TET-OFF human ESCs expanded for 15 passages (P15) in ENHSM + DOX conditions. Please note that no *in vitro* priming or media other than ENHSM+DOX was used before the cells were injected into the mice to test for teratoma formation. H. Scheme showing strategy for generating DGCR8 knockout clones in human cells, western validation of correctly targeted clones and sequencing of mutated alleles obtained.

ggggcccGCCTGCTCTTTCTGGGTGATccccgac - SEQ ID NO: 1
CCGGACGAGAAAGA-SEQ ID NO: 2
CCGGAACGAGAAAGA-SEQ ID NO: 3

I. FACS analysis showing preservation of ΔPE-OCT4-GFP naive marker expression in both WT and DGCR8 KO human ESCS expanded in ENHSM conditions.

FIGs. 4A-G. ENHSM conditions enable naive pluripotency maintenance in the absence of DNMT1 or L-Glutamine. A. Strategy for generating human ESCs with TET-OFF regulated expression of DNA methyltransferase enzyme, DNMT1. B. Western blot analysis for DNMT1 expression in ENHSM conditions supplemented with either DOX of BRAF inhibitor (SB590885 - 0.25μM). Please note that DOX ablates DNMT1 expression. Also, please note that BRAFi depletes DNMT1 expression to much lower levels than seen in ENHSM conditions (without DOX), yet still they retain residual DNMT1 expression that is necessary for their survival and viability when BRAFi is added to ENHSM conditions. C. Representative images for human DNMT1 TET-OFF engineered cells in different conditions with or without DOX addition. Previously described primed and naïve conditions do not support maintain their pluripotency and viability when DOX is added (DNMT1 is depleted). Only ENHSM and ENHSM-ACT conditions (with and without irradiated feeder cells - MEFs) maintain robust expansion of dome-like undifferentiated human PSCs in vitro even after extended passaging in the presence of DOX. D. DNMT1 TET-OFF ESC clone was maintained in the presence of DOX for up to 4 passages in different conditions and stained for OCT4 to quantify percentage of cells that retained their pluripotency. Graph shows that only ENHSM and ENHSM-ACT conditions (with and without irradiated feeder cells - MEFs) maintain robust expansion of dome-like undifferentiated human PSCs in vitro. Omitting TNKi and SRCi (in other words WNTi and SRCi) form ENHSM leads to loss of ability to maintain DNMT1 depleted human naive PSCs, as evident from loss of OCT4+ cells. E. WGBS validates global reduction in CG methylation in ENHSM conditions following DOX addition (shutdown of DNMT1 expression). F. Oxygen consumptions rate (OCR) measurement in different conditions. G. Mouse or human ESCs carrying NANOG-GFP pluripotency reporter were expanded in the indicated naive and primed conditions in the absence of exogenous L-Glutamine supplementation for 4 passages. Percentage of pluripotent cells was quantified based on GFP expression levels. Graph shows that only ENHSM and ENHSM-ACT conditions (with and without irradiated feeder cells - MEFs) maintain expansion and stability of human NANOG+ pluripotent cells when exogenous L-Glutamine is omitted, and that this is similar to 2iL conditions on mouse naive ESCs. Omitting TNKi and SRCi (in other words WNTi and SRCi) form ENHSM leads to loss of ability to maintain human naive PSCs without exogenous L-Glutamine supplementation, as evident from loss of NANOG-GFP+ cells.

FIGs. 5A-E. Optimized ENHSM conditions enable naive pluripotency maintenance in the absence of DNMT1 or L-Glutamine. A. Immunostaining validating OCT4+ expressed in DNMT1 TET-OFF cells expanded in ENHSM + DOX conditions at P10. B. Mitochondrial staining in primed and naïve ENHSM conditions. TMRE staining is dependent on mitochondrial membrane activity, and is much more enhanced in ENHSM conditions as compared to their isogenic primed cells expanded in TeSR. C. FACS based validation of pluripotency maintenance in human stem cells expanded in ENHSM conditions with and without exogenous L-Glutamine. Percentage of positive cells and intensity of naive marker expression were not compromised upon omitting GLUT in ENHSM based conditions. D. Representative phase contrast and fluorescent images of human cells expanded in ENHSM conditions with and without exogenous L-Glutamine (GLUT). TeSR primed human ESCs were used as controls. E. human ESCs expanded for 10 passages in ENHSM conditions without exogenous GLUT robustly formed teratomas (without any need for exogenously induced priming before they were injected).

FIGs. 6A-C. Source of stem cell liens used herein in ENHSM conditions. A. 5 new lines were derived in ENHSM or ENHSM-ACT conditions directly from human blastocysts. At P8, a small portion of the cells was taken and expanded in primed conditions (and thus are labeled as "primed cells". B. Representative images showing previously established human primed/conventional ESCS were transferred to ENHSM conditions, and after at least 5 passages a small portion of them were transferred back into primed conditions (thus are referred to as "reprimed" cells). C. Newly derived iPSC lines from dermal fibroblasts or peripheral blood mononuclear cells (PBMCs) were obtained following OSKM transduction and cell culturing in ENHSM conditions.

FIG. 7. Pluripotency marker expression and characterization in ENHSM conditions. Representative immunostaining for pluripotency markers in ENHSM conditions are shown. Primed cells expanded in TeSR conditions are used as controls. Note that KLF17 and TFCP2L1 are naive pluripotency specific markers and are expressed in ENHSM conditions and not in primed cells.

FIG. 8. ENHSM conditions maintain teratoma formation competence of PSCs. Mature teratoma images are shown following their derivation from the indicated cell lines expanded in the different indicated conditions. Please note that without exception, all teratomas were formed following direct subcutaneous injections after being expanded only in the indicated media condition and without the need for any expansion in other primed conditions in vitro before injection.

FIGs. 9A-D. ENHSM endows human PSCS with canonical naïve-like transcriptional features. A. Unbiased hierarchical clustering was performed on RNA-seq measurement obtained from different human ESC and iPSCs expanded in ENHSM, ENHSM-ACT, ENHSM+CHIR and TeSR primed conditions. The data was also clustered with previous independently generate RNA-seq on human PSCs expanded in 5iLA conditions (Theunissen et al. Cell Stem Cell 2016) or Rest conditions (Takashima et al. Cell 2014 - composed of NANOG-KLF2 transgenes and 2iLGo). Figure shows that ENHSM and ENHSM-ACT conditions (Dark blue) resemble 5iLA and Reset conditions (light blue) and cluster separately from primed cells (Red and orange). B. PCA analysis of samples represented in a, showing that ENHSM and ENHSM-ACT conditions (Dark blue) resemble 5iLA and Reset conditions (light blue) and cluster separately from primed cells (Red and orange). Please note that ENHSM supplementation of GSK3 inhibitor CHIR99021 (CHIR) compromises the naive of human PSCs and renders them more similar to primed cells at the transcriptional levels. C. RT-PCR analysis for naïve pluripotency markers. Values were normalized to ACTIN and GAPDH. Primed expression levels were set as 1. Please note that ENHSM-ACT (E19) show higher expression of naive pluripotency markers than ENHSM (E20), consistent with the notion that ACTIVIN A supports naive pluripotency in humans. D. Correspondence between transposable element expression (TE) in naïve/primed ESCs and single-cell human embryonic stages (Yan et al. 2013). For every stage of human embryonic development, a statistical test was performed to find the TEs that have a different expression level compared to other stages. The proportions of developmental stage-specific TEs that are upregulated ($p < 0.05$, 2-fold change) in naive or primed cells are indicated in orange and blue, respectively.

FIG. 10. Chromosomal stability following long term expansion in ENHSM based conditions. Metaphase chromosomal spreads are shown from the indicated human ESC and iPSC lines expanded in ENHSM based conditions. Passage numbers are indicated throughout.

FIGs. 11A-D. Differentially expressed genes highlights regulatory candidates. A. Volcano plot comparing change in expression of all 26,899 genes (log2(Naive ENHSM/primed Fold-Change) in x-axis), to their statistic (-log10(q-value) in y-axis). Differentially expressed genes (Fold-change>2(<0.5), p-adjusted<0.1, n=6121) are marked in red. Extreme genes are highlighted. B. Spearman correlation matrix of naive ENHSM (E20) and primed samples, along with previously published naive and primed samples (Takashima et al, 2014). C. Differentially expressed genes (Fold-change>2(<0.5), p-adjusted<0.1, n=7087 genes). Clustered expression profile in naive and primed samples, along with Takashima et al samples. D. FACS analysis for expression levels of the indicated surface markers. CD130 and CD77 are induced in ENHSM conditions consistent with their previous designation as markers of human naïve pluripotency (Collier et al. Cell Stem Cell 2017). CD24 is depleted in naive conditions.

FIGs. 12A-B. Expression profile of selected sets of genes in ENHSM naive and primed conditions. A. Expression profile of selected sets of genes in naive and primed conditions. B. RT-PCR validation of expression of primed pluripotency markers ZIC2 and OTX2. Both were significantly depleted in ENHSM based naive conditions.

FIGs. 13A-E. Characterization of STELLA and TFAP2C expression in function in human PSCS expanded in ENHSM conditions. A. Strategy for generating human STELLA-CFP knock in reporter cell line. B. Southern blot validation analysis for corrected targeting in selected clones. C. FACS analysis for STELLA-CFP expression levels in the indicated primed and naive conditions. Both ENHSM and ENNHSM-ACT conditions upregulated STELLA expression in comparison to primed cells and consistent with transcriptome data. D. Strategy for generating TFAP2C human KO for TFAP2C via simultaneous targeting of both alleles.

CGTTGTAAGCAAAGAGTGCG - SEQ ID NO: 4;
CTACCACAAATGTCCCACGC - SEQ ID NO: 5.

E. Western blot analysis for validation of TFAP2C KO generation in primed human WIBR3-ΔPE-O4G hESCs.

FIGs. 14A-B. Generation of TFAP2C KO human ESCS with reporter for human naive pluripotent state. A. Strategy for generating TFAP2C human KO in human ESC line carrying GFP/tdTomato reporters on each of the X chromosomes respectively. B. Immunostaining analysis for TFAP2C (also known as AP2gamma) expression in WT and KO human ESC clones (WIBR2-29-8 hESC line). OCT4 expression was not affected in primed KO cells in comparison to WT primed control cells.

FIGs. 15A-B. Human PSCs in ENHSM conditions have a transposon element (TE) transcription signature of the human pre-implantation embryo, a. Heatmap of RNA-seq expression data form primed human ESSCs and naïve cells expanded in ENHSM and ENHSM-ACT conditions. Data shown include 10000 TEs with the highest standard deviation between samples. Figure shows clear separation between naive ENHSM and primed datasets in TE expression and profile. b. Principal component analysis (PCA) pf primed (in TeSR or KSR/FGF2 conditions) or ENHSM naive conditions based on the differential expression of transposable elements.

FIG. 16. P53 (TP53) targeting in human iPSCs. Design of CRISPR/Cas9 targeting Exon 4 of hTP53 to generate knock-out with the guide RNA in red and the PAM sequence in green. Western blot analysis showing complete depletion of TP53 protein in various chosen clones. DNA sequence alignment showing out-of-frame insertions/deletions in clone C2 and a point mutation in clone E7. Staining and Karyotyping showed normal pluripotency marker expression and karyotype in representative clones.

CCTGGGTCTTCAGTGAACCATTGTTCAATATCGTCCGGGGACAGCATCAAATC ATCCAT- SEQ ID NO: 6;
ATGCTGTCCCCGGACGATATTGAACAATGGTTCACTGAAG - SEQ ID NO: 7;
ATGCTGTCCCCGTGAGCCACCGTGCCCACTGAAG - - SEQ ID NO: 8;
GTCCCCGGACGATATTGAA - SEQ ID NO: 9;
GTCCCCGGAACGATATTGAA - SEQ ID NO: 10.

FIGs. 17A-D. Chromosome X reactivation status and DNA methylation profile in ENHSM based conditions. A. Schematic and FACS results following using WIBR2 (female 46XX) 29-9 hESC line that carries GFP and mCherry on each of the X chromosomes in the MECP2 locus. Parental 29-9 clone has the X chromosome carrying mCherry allele in the active state, and thus is positive only for mCherry and negative for GFP in the primed state. Upon transfer to ENHSM conditions, all cells turn on both X chromosomes and thus become double positive for both fluorescent markers (GFP and mCherry). After transfer into primed conditions (i.e. repriming), cells start to inactivate the X chromosome again. B. RNA-FISH analysis for ATRX transcription in primed and ENHSM WIBR3 cells. Note ATRX is active on both X chromosomes only in ENHSM conditions. C. Western blot analysis for DNA methylation regulators, DNMT1 and UHRF enzymes. DNMT1 protein levels are maintained in all conditions. UHRF1 is partially depleted in ENHSM conditions, and this decrease is more enhanced when ERKi concentration is increased in ENSHM conditions. D. Global methylation histogram as calculated from primed samples, and naive samples that were maintained in various conditions, including titrated ERKi supplementation, along with previously published Reset-naïve and primed samples (Takashima et al), and human ICM samples (Smith et al, Nature 2014, Guo et al, Nature 2014). DNMT1$^{-/-}$(from TET-OFF lines) samples were used as negative control for methylation. Dark blue - percentage of highly methylated CpGs (>0.1 methylation level), light blue - percentage of lowly methylated CpGs (<0.1 methylation level). Average of each sample is indicated as a yellow mark.

FIG. 18. Chromosome X reactivation status and DNA methylation profile in ENHSM based conditions. a. Schematic and FACS results following using WIBR2 (female 46XX) 29-8 hESC line that carries GFP and mCherry on each of the X chromosomes in the MECP2 locus. Parental 29-8 clone has the X chromosome carrying GFP allele in the active state, and thus is positive only for GFP and negative for mCherry in the primed state. Upon transfer to ENHSM conditions, all cells turn on both X chromosomes and thus become double positive for both fluorescent markers (FGP and mCherry). Then we started depleting indicated components from ENHSM conditions, and cells were subjected to FACS analysis after 10 days. Figure shows that upon LIF omission, cells do not become primed and cells maintain b=XaXa state. Withdrawal of PKCi, SRCi or WNTi compromises XaXa state and cells start inactivating one of the X chromosomes, indicating loss of naive state identity.

FIGs. 19A-H. NOTCHi allows maintain human naïve cells in ENHSM without ERKi. A. FACS analysis showing status of X activation in female 29-9 cells following decreasing concentrations of ERKi in ENHSM conditions. Note that the fraction of cells inactivating X chromosome increases with depleting ERKi concentrations. B. Schematic showing screen strategy for finding small molecule supplements that could allow maintaining GFP+/mCherry+ cells in ENHSM conditions in which ERKi is completely omitted (OENHSM) or partially depleted (tENHSM). C. FACS analysis following supplementing OENHSM or tENHSM with ACTIVIN A or DBZ, a gamma secretase small molecule inhibitor that blocks NOTCH signaling (NOTCHi). D. Summary of small molecules and their concentrations used in the optimized tENHSM and OENHSM conditions used herein. E. Percentage of OCT4+ cells at P4 from TET-OFF-METTL3 cell lines in ENHSM, tENHSM and OENHSM conditions. Note that removal of DBZ from tENHSM or OENHSM results in loss of maintenance of pluripotency when METTL3 is depleted by DOX. F. Percentage of OCT4+ cells at P4 from TET-OFF-DNMT1 cell lines in ENHSM, tENHSM and OENHSM conditions. Note that removal of DBZ from tENHSM or OENHSM results in loss of maintenance of pluripotency when DNMT1 is depleted by DOX. G. Percentage of OCT4-GFP+ cells at P4 from WIBR3-OCT4-GFP cells in ENHSM, tENHSM and OENHSM conditions without L-Glutamine. Note that removal of DBZ from tENHSM or OENHSM results in loss of maintenance of pluripotency when exogenous L-Glutamine is omitted from the grow conditions. H. RT-PCR analysis for hESCs in Primed, ENHSM, OENHSM and tENHSM. Note that naive pluripotency markers like DPPA5, TFCP2L1, KLF17 are induced in all three ENHSM based conditions. DNMT3L which supports DNA hypomethylation in ENHSM conditions is not induced in OENHSM or tENHSM conditions.

FIGs. 20A-B. NOTCHi allows maintain human naïve cells in ENHSM without ERKi and without depleting global DNA methylation. A. Spearman correlation matrix of naïve, primed and titrated ERK conditions hESC samples, along with naive and primed samples from Takashima et al. Cell 2014. B. Principal component analysis (PCA) comparing naive (E20=ENHSM (green), E19=ENHSM-ACT (yellow)), primed (purple) and titrated ERK (oE20=0ENHSM (red), tE20=tENHSM (pink)) conditions with Wu.et.al naive (blue) and primed (jade) conditions.

FIG. 21. ENHSM-derived human naive pluripotent stem cells are competent for interspecies chimaera formation. Representative images of frozen tissue sections of E17.5 chimaeric embryos were stained for GFP and Human-Nuclei to confirm human identity. Non-injected embryos served as negative control. GFP, Human-Nuclei, overlap as well as merged are zoomed-in regions of lung tissue depicted in red squares in the tiles. White arrowheads in insets point out co-localization between GFP and Human-Nuclei. GFP, green fluorescent protein; WT, wild-type; iPSC, induced pluripotent stem cell.

Tile scale bar 500 μm. Zoomed-in scale bar 100 μm. Lung-E17.5-Frozen section-injected WT hiPSCs; 100 μm scale bars. Tile: scale bar 500 μm.

FIG. 22. ENHSM-derived human naive pluripotent stem cells integrate successfully into mouse embryos and acquire respective tissue identity. Representative images of frozen tissue sections of E17.5 chimaeric embryos were stained for GFP and Pro-Spc for lung-specific alveolar-surfactant secreting cells. Non-injected embryos served as negative control. GFP, Pro-Spc, overlap as well as merged are zoomed-in regions of lung tissue depicted in red squares in the tiles. White arrowheads in insets point out co-localization between GFP and Pro-Spc. GFP, green fluorescent protein; WT, wild-type; Pro-Spc, prosurfactant Protein C; iPSC, induced pluripotent stem cell. Tile scale bar 1000 μm. Zoomed-in scale bar 50 μm.

FIG. 23. Blocking apoptosis by depleting P53 endows P53KO hiPSCs with enhanced integration in cross-species humanized chimeric mouse embryos. Representative images depicting more extensive integration of P53KO GFP-labelled hiPSCs into different locations within developing E9.5/E10.5 mouse embryo in comparison to WT GFP+-cells and non-injected embryos. Hoechst was used for counterstaining. Red squares in the first column represent zoomed-in areas shown in the following images 1 and 2. GFP, green fluorescent protein; WT, wild-type; iPSC, induced pluripotent stem cell.

Tile scale bar 200 μm. Inset scale bar 50 μm.

FIG. 24. P53KO in human naïve pluripotent stem cells allows for establishment of next-generation cross-species chimaera. Representative images of whole-mount in-toto imaged mouse embryos are shown in comparison to non-injected wild-type embryos. White squares in tiles outline zoomed-in regions in subsequent panels. GFP staining was used to trace hiPSC-derived progeny and CellTracker and Hoechst as counterstaining. GFP, green fluorescent protein; p53, tumor protein p53; iPSC, induced pluripotent stem cell. FACS analysis for GFP is shown in middle Table. Bottom panel showed PCR detection for human mitochondrial specific RNA detection assay (Theunissen et al Cell Stem Cell 2016).

FIG. 25. NUMA immunofluorescence staining allows for tracing of human-specific cell cohorts. Representative images of immunofluorescence staining of mouse and human teratoma are shown with GFP and NUMA labelling. GFP, green fluorescent protein; NUMA, nuclear mitotic apparatus protein.

Tile scale bar 1000 μm. Zoomed-in region scale bar 100 μm.

FIG. 26. NUMA immunofluorescence staining allows for tracing of human-specific cell cohorts. Representative images of immunofluorescence staining of mouse and human teratoma are shown with GFP and NUMA labelling. GFP, green fluorescent protein; NUMA, nuclear mitotic apparatus protein.
Tile scale bar 1000 μm. Zoomed-in region scale bar 100 μm.

FIG. 27. NUMA immunofluorescence staining allows for tracing of human-specific cell cohorts. Representative images of immunofluorescence staining of mouse and human teratoma are shown with GFP and NUMA labelling. GFP, green fluorescent protein; NUMA, nuclear mitotic apparatus protein.
Tile scale bar 1000 μm. Zoomed-in region scale bar 100 μm.

FIG. 28. Contribution of GFP+ hiPSC-derived descendants to ectodermal-neural lineages within chimaeric humanized mouse embryos. Representative images of immunofluorescence staining of injected (upper panels) and non-injected E15.5 mouse embryos (lower panels) for each tissue type respectively are shown. GFP served as human cell tracer and TUJ1 as neural progenitor signature. GFP, TUJ1, overlap as well as merged constitute zoomed-in regions of tissues depicted in red squares in the tiles. White arrowheads in insets depict co-localization of GFP and TUJ1. GFP, green fluorescent protein; TUJ1, neuron-specific class III beta-tubulin; P53KO, knock-out of tumor protein p53. Tile scale bar 2000 μm. Non-injected scale bar 1 mm. Zoomed-in region scale bar 100 μm.

FIG. 29. Contribution of GFP+ hiPSC-derived descendants to ectodermal-neural lineages within chimaeric humanized mouse embryos. Representative images of immunofluorescence staining of injected (upper panels) and non-injected E15.5 mouse embryos (lower panels) for each tissue type respectively are shown. GFP served as human cell tracer and TUJ1 as neural progenitor signature. GFP, TUJ1, overlap as well as merged constitute zoomed-in regions of tissues depicted in red squares in the tiles. White arrowheads in insets depict co-localization of GFP and TUJ1. GFP, green fluorescent protein; TUJ1, neuron-specific class III beta-tubulin; P53KO, knock-out of tumor protein p53. Tile scale bar 1000 μm. Zoomed-in region scale bar 100 μm.

FIG. 30. Contribution of GFP+ hiPSC-derived descendants to ectodermal-neural lineages within chimaeric humanized mouse embryos. Representative images of immunofluorescence staining of injected (upper panels) and non-injected E15.5 mouse embryos (lower panels) for each tissue type respectively are shown. GFP served as human cell tracer and SOX2 as neural progenitor signature. GFP, SOX2, overlap as well as merged constitute zoomed-in regions of tissues depicted in red squares in the tiles. White arrowheads in insets depict co-localization of GFP and SOX2. GFP, green fluorescent protein; SOX2, sex determining region Y-box; P53KO, knock-out of tumor protein p53. Tile scale bar 1000 μm. Zoomed-in region scale bar 100 μm.

FIG. 31. Contribution of GFP+ hiPSC-derived descendants to endoderm and mesodermal lineages within chimaeric humanized mouse embryos. Representative images of immunofluorescence staining of injected (upper panels) and non-injected E15.5 mouse embryos (lower panels) for each tissue type respectively are shown. GFP served as human cell tracer and SOX17 as endoderm progenitor and mesoderm-progeny tissue marker. GFP, SOX17, overlap as well as merged constitute zoomed-in regions of tissues depicted in red squares in the tiles. White arrowheads in insets depict co-localization of GFP and SOX17. GFP, green fluorescent protein; SOX17, SRY-related HMG-box 17; P53KO, knock-out of tumor protein p53. Tile scale bar 2000 μm. Zoomed-in region scale bar 100 μm.

FIG. 32. Contribution of GFP+ hiPSC-derived descendants to endoderm and mesodermal lineages within chimaeric humanized mouse embryos. Representative images of immunofluorescence staining of injected (upper panels) and non-injected E15.5 mouse embryos (lower panels) for each tissue type respectively are shown. GFP served as human cell tracer and SOX17 as endoderm progenitor and mesoderm-progeny tissue marker. GFP, SOX17, overlap as well as merged constitute zoomed-in regions of tissues depicted in red squares in the tiles. White arrowheads in insets depict co-localization of GFP and SOX17. GFP, green fluorescent protein; SOX17, SRY-related HMG-box 17; P53KO, knock-out of tumor protein p53. Tile scale bar 2000 μm. Zoomed-in region scale bar 100 μm.

FIG. 33. P53KO in human naïve pluripotent stem cells allows for establishment of next-generation cross-species chimaera. Representative images of whole-mount in-toto imaged mouse embryos are shown in comparison to non-injected wild-type embryos. White squares in tiles outline zoomed-in regions in subsequent panels. GFP staining was used to trace hiPSC-derived progeny and CellTracker and Hoechst as counterstaining. GFP, green fluorescent protein; p53, tumor protein p53; iPSC, induced pluripotent stem cell.

FIG. 34 illustrates FACS analysis of WIBR3 human ES cells carrying OCT4-GFP or deltaPE-OCT4-GFP knock-in reporter in the indicated serum-free and feeder free N2B27 conditions for 3 passages. Dashed line indicate threshold for specific positive fluorescence signal.

Wi= WNTi = TNKi (XAV939 3 μM)

Si= SRCi= CGP77675 1.2 μM

Pi=PKCi= Go6983 2 μM

Ni= NOTCHi= DBZ 0.3 μM

FIG. 35 illustrates the XIST methylation status in primed (FGF) and 8 different naive conditions indicated above after 10 passages of H9 female human ESCs. Black dots represent methylation, white dots non-methylated.

1) Condition 1- PKCi (Go6983 2 μM), TNKi/WNTi (XAV939 2 μM) and RBPJi/NOTCHi (RIN1 0.6 μM)
2) Condition 2- PKCi (Go6983 2 μM), TNKi/WNTi (XAV939 2μM), RBPJi/NOTCHi (RIN1 0.6 μM) and SRCi (CGP77675 1 μM)
3) Condition 3- PKCi (Go6983 2μM), TNKi/WNTi (XAV939 2μM), RBPJi/NOTCHi (RIN1 0.6 μM) and MEK/ERKi (PD0325901 1μM)
4) Condition 4- PKCi (Go6983 2μM), TNKi/WNTi (XAV939 2μM), RBPJi/NOTCHi (RIN1 0.6 μM), SRCi (CGP77675 1μM), MEK/ERKi (PD0325901 1μM)
5) Condition 5- PKCi (Go6983 2μM), TNKi/WNTi (XAV939 2μM) and RBPJi/NOTCHi (RIN1 0.6 μM) + LIF (20ng/ml)
6) Condition 6- PKCi (Go6983 2μM), TNKi/WNTi (XAV939 2μM), RBPJi/NOTCHi (RIN1 0.6 μM) and SRCi (CGP77675 1μM) + LIF (20ng/ml)
7) Condition 7- PKCi (Go6983 2μM), TNKi/WNTi (XAV939 2μM), RBPJi/NOTCHi (RIN1 0.6 μM) and MEK/ERKi (PD0325901 1μM) + LIF (20ng/ml)
8) Condition 8- PKCi (Go6983 2μM), TNKi/WNTi (XAV939 2μM), RBPJi/NOTCHi (RIN1 0.6 μM), SRCi (CGP77675 1μM), MEK/ERKi (PD0325901 1μM) + LIF (20ng/ml)

## DESCRIPTION OF SPECIFIC ASPECTS OF THE DISCLOSURE

The present disclosure, in some aspects thereof, relates to culture media for culturing pluripotent stem cells more particularly, but not exclusively, to naive pluripotent stem cells.

Before explaining at least one aspect of the disclosure in detail, it is to be understood that the disclosure is not necessarily limited in its application to the details set forth in the following description or exemplified by the Examples. The disclosure is capable of other aspects or of being practiced or carried out in various ways.

The present inventors have uncovered novel conditions, which are required for isolating and generating primate (e.g., human) pluripotent stem cells (PSCs), and maintaining them in their pluripotent state.

As shown in the Examples section which follows, the present inventors have uncovered through laborious experimentation, particular combinations of factors that are required for maintaining PSCs in a pluripotent state in general and more specifically, in a "naive state". Unlike combinations of factors previously disclosed, (see for example WO2014/174470), the present combinations were shown to maintain the pluripotent stem cell in a hypomethylated state.

Thus, according to a first aspect of the present disclosure, there is provided a culture medium comprising a Wingless/Integrated (WNT) inhibitor, a SRC Proto-Oncogene, Non-Receptor Tyrosine Kinase (SRC) inhibitor and a protein kinase C (PKC) inhibitor, the medium being devoid of an amount of GSK3β inhibitor that increases β-catenin translocation to the nucleus of a pluripotent stem cell being cultured in the culture medium.

According to another aspect of the present disclosure, there is provided a culture medium comprising a WNT inhibitor, a Notch inhibitor and a protein kinase C (PKC) inhibitor, the medium being devoid of an amount of GSK3β inhibitor that increases β-catenin translocation to the nucleus of a pluripotent stem cell being cultured in said culture medium.

As used herein the phrase "culture medium" refers to a solid or a liquid substance used to support the growth of stem cells and maintain them in an undifferentiated state. Preferably, the phrase "culture medium" as used herein refers to a liquid substance capable of maintaining the stem cells in an undifferentiated state. The culture medium used by the present disclosure can be a water-based medium which includes a combination of substances such as salts, nutrients, minerals, vitamins, amino acids, nucleic acids, proteins such as cytokines, growth factors and hormones, all of which are needed for cell proliferation and are capable of maintaining the stem cells in an undifferentiated state. For example, a culture medium can be a synthetic tissue culture medium such as KO-DMEM (Gibco-Invitrogen Corporation products, Grand Island, NY, USA), DMEM/F12 (Gibco-Invitrogen Corporation products, Grand Island, NY, USA), Neurobasal medium (Invitrogen Corporation products, Grand Island, NY, USA 21103-049) or DMEM/F12 (without HEPES; Biological Industries, Biet Haemek, Israel), supplemented with the necessary additives as is further described hereinunder.

According to a particular aspect, the medium is a 1:1 mix of Neurobasal medium and DMEM F/12.

Preferably, all ingredients included in the culture medium of the present disclosure are substantially pure, with a tissue culture grade.

According to some aspects of the disclosure, the culture medium is devoid of serum, e.g., devoid of any animal serum.

According to some aspects of the disclosure, the culture medium is devoid of any animal contaminants, *i.e.,* animal cells, fluid or pathogens (e.g., viruses infecting animal cells), e.g., being xeno-free.

According to some aspects of the disclosure, the culture medium is devoid of human derived serum.

According to some aspects of the disclosure, the culture medium further comprises a serum replacement (*i.e.,* a substitute of serum) such as KNOCKOUT™ Serum Replacement (Gibco-Invitrogen Corporation, Grand Island, NY USA), ALBUMAX®II (Gibco®; Life Technologies - Invitrogen, Catalogue No. 11021-029;
Lipid-rich bovine serum albumin for cell culture) or a chemically defined lipid concentrate (Gibco®; Invitrogen, Life Technologies - Invitrogen, Catalogue No. 11905-031).

According to some aspects of the disclosure, the culture medium further comprises N2 supplement (Gibco®; Life Technologies - Invitrogen, Catalogue No. 17502-048) a chemically defined, serum-free supplement. For a 500 ml of culture medium 5 ml of the N2 mix (Invitrogen) can be added.

Alternatively, the following materials (substitute the N2 supplement) can be added to a 500 ml culture medium: Recombinant Insulin (Sigma I-1882) at a 12.5 microg/ml (μg/ml) final concentration; Apo-Transferrin (Sigma T-1147) at a 500 μg/ml final concentration; Progesterone (Sigma- P8783) at a 0.02 μg/ml final concentration; Putrescine (Sigma-P5780) at a 16 μg/ml final concentration; and 5 microL (μl) of 3 mM stock of Sodium Selenite (Sigma - S5261) are added per 500 ml culture medium (e.g., the WIS-NHSM).

According to some aspects of the disclosure, the KNOCKOUT™ Serum Replacement is provided at a concentration of at least 0.5%, e.g., in the range of about 0.5%-25%, e.g., about 5%, about 10%, about 15%, about 20% or about 25%.

According to some aspects of the disclosure, the ALBUMAX™ is provided at a concentration of at least 0.01%, e.g., in the range of about 0.01%-10%, e.g., about 0.1%, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9% or about 10%, e.g., 1%.

According to some aspects of the disclosure, the defined lipid concentrate is provided at a concentration of at least about 0.1%, e.g., in the range of 0.1-5%, e.g., about 0.2%, about 0.3%, about 0.4%, about 0.5%, about 0.6%, about 0.7%, about 0.8%, about 0.9%, about 1%, about 2%, about 3%, about 4%, about 5%, e.g., 1%.

According to some aspects of the disclosure, the culture medium comprises the N2 supplement (e.g., 5 ml N2 per 500 ml of culture medium) and the defined lipid concentrate (5 ml defined lipid concentrate per 500 ml medium).

According to some aspects of the disclosure, the culture medium can further include antibiotics (e.g., PEN-STREP), sodium pyruvate, B27, NEAA (non-essential amino acids).

The culture medium may comprise glutamine or be devoid of glutamine (e.g. only comprise trace amounts (e.g. less than $1/10^{th}$ of the amount that is typically present in base media such that it does not bring about a biological effect). In one aspect, the medium is completely devoid of exogenously added glutamine.

The present inventors contemplate addition of a combination of specific inhibitors to the medium disclosed. Such inhibitors are preferably specific towards their target. In one aspect, they are capable of binding the named target with a higher affinity (at least 10 %, 20 %, 30 %, 40 % 50 %, 60 %, 70 %, 80 %, 90 % or even 100 % higher affinity) than another protein which is expressed in the cell.

As mentioned, the media of the present disclosure comprise a Wnt inhibitor.

The term "Wnt inhibitor" as used refers to any agent, including any compound and/or protein that inhibits Wnt signaling, including but not limited to Wnt antagonists that bind either to the Wnt ligand itself, or to Wnt receptors, such as Dickkopf (Dkk) proteins, Wnt Inhibitory Factor-1 (WIF-1), and secreted Frizzled-Related Proteins (sFRPs), as well as Wnt inverse agonists (e.g. an agent that binds to the same receptor as an agonist but induces a pharmacological response opposite to that of an agonist).

According to a particular aspect, the Wnt inhibitor is a small molecule.

In one aspect, the Wnt inhibitor brings about its effect by stabilizing the AXIN/APC complex which in turn degrades β-catenin, thereby inhibiting Wnt signaling.

Exemplary Wnt inhibitors include, but are not limited to ICG-001, IWR-1, IWP2, XAV939, Wnt-C59 (C59), IWP-L6, iCRT3, LF3, PNU-74654, KYA1797K, PRI-724 and WIKI 4, all of which are commercially available from Selleckchem and/or Tocris.

According to a particular aspect, the Wnt inhibitor is a Tankyrase inhibitor (e.g. IWR-1 - Sigma Aldrich 10161; and XAV939 - TOCRIS Cat. No. 3748). In one aspect, the Tankyrase inhibitor is one which blocks the PARP domain of Tankyrase (which ultimately leads to an increase in the stability of AXIN1 and AXIN2 and therefore inhibition of canonical Wnt signaling).

Another exemplary WNT inhibitor is a small molecule inhibitor for Porcupine enzyme which is responsible for processing and secretion of all Wnt signaling ligands (e.g. IWP2).

The Wnt inhibitor is typically present in the medium in an amount such that the overall net effect thereof is a reduction in the amount of β-catenin in the nucleus of a pluripotent stem cell which is cultured within. It will be appreciated that the medium is typically devoid of agents which promote β-catenin translocation to the nucleus. Thus, according to this aspect of the present disclosure, the medium is devoid of an amount of GSK3β inhibitor that increases β-catenin translocation to the nucleus of a pluripotent stem cell being cultured in the culture medium. For example, the medium of the present disclosure should not contain more than 0.5 μM of a GSK3β inhibitor and preferably not more than 0.1 μM of a GSK3β inhibitor. It will be appreciated that the phrase "being devoid of a GSK3β inhibitor" refers to a medium in which no GSK3β inhibitor has been positively added to a medium and does not mean to exclude that a trace amount of GSK3 inhibitor is contained in the base medium.

Exemplary amounts of Wnt inhibitor (e.g. XAV939) are between 0.1 μM - 100 μM, more preferably between 1 μM - 100 μM, 0.1 μM - 10 μM, and more preferably between 1 μM - 10 μM - about 3 μM.

As mentioned, a NOTCH signaling inhibitor is contemplated to be included in the media of the present disclosure. Preferably, the NOTCH signaling inhibitor is added when the medium comprises less than 0.5 μM, for example about 0.4 μM, 0.3 μM, 0.2 μM or 0.1 μM ERK1/2 inhibitor. In a particular aspect, the NOTCH signaling inhibitor is added to a medium which is devoid of an ERK1/2 inhibitor.

NOTCH signaling inhibitors include, but are not limited to the following gamma secretase inhibitors: DAPT (Axon Medchem 1484 - 0.05-50 μM final concentration), LY2886721 hydrochloride (Axon Medchem 1964 - 0.05-50 μM final concentration)], DBZ (Axon Medchem - Axon 1488- 0.05-50 μM final concentration).

According to a particular aspect, the NOTCH signaling inhibitor is one which inhibits the transcription factor RBPJ - Recombination Signal Binding Protein For Immunoglobulin Kappa J Region. An example of such an inhibitor is RIN1 (see for example Hurtado et al., Scientific Reports, Volume 9, Article number: 10811 (2019). An exemplary concentration of RIN1 is 0.1-10 μM and more preferably between 0.1 and 1 μM.

The medium of this aspect of the present disclosure may further comprise a SRC inhibitor, also referred to herein as a src family kinase inhibitor.

The phrase "src family kinase inhibitor" refers to any agent which impedes or inhibits the function of a member of the src kinase family. Such agents include, without limitation, small molecules, chemical compounds and nucleic acid molecules which function to down regulate expression of target genes and inhibit the function of direct and indirect c-Src substrates, such as the focal adhesion kinase, signal transducer and activator of transcription 3 (STAT3), vascular endothelial growth factor (VEGF), paxillin, Cas, p190RhoGAP, RRas, E-cadherin, c-Jun amino-terminal kinase, NEDD9, and others. Exemplary agents include dasatinib, SU6656, and AZD05530. Src inhibitors are also available from Wyeth and include for example, 4-[(2,4-Dichloro-5-methoxyphenyl)amino]-7-[3-(4-ethyl-1-piperazinyl)propo- xy]-6-methoxy-3-quinolinecarbonitrile; 4-[(2,4-Dichloro-5-methoxyphenyl)amino]-6-methoxy-7-[2-(4-methyl-1-pipera- zinyl)ethoxy]-3-quinolinecarbonitrile; 4-[(2,4-Dichloro-5-methoxyphenyl)amino]-7-[2-(4-ethyl-1-piperazinyl)ethox- y]-6-methoxy-3-quinolinecarbonitrile; 4-[(2,4-Dichloro-5-methoxyphenyl)amino]-6-methoxy-7-[(1-methylpiperidin-4- -yl)methoxy]-3-quinolinecarbonitrile; 4-[(2,4-Dichloro-5-methoxyphenyl)amino]-6-methoxy-7-[2-(1-methylpiperidin- -4-yl)ethoxy]-3-quinolinecarbonitrile; 4-[(2,4-Dichloro-5-methoxyphenyl)amino]-6-methoxy-7-[3-(1-methylpiperidin- -4-yl)propoxy]quinoline-3-carbonitrile; 4-[(2,4-Dichloro-5-methoxyphenyl)amino]-7-[(1-ethylpiperidin-4-yl)methoxy-]-6-methoxyquinoline-3-carbonitrile; 4-[(2,4-Dichloro-5-methoxyphenyl)amino]-6-ethoxy-7-[3-(4-methylpiperazin-- 1-yl)propoxy]quinoline-3-carbonitrile; 4-[(2,4-Dichloro-5-methoxyphenyl)amino]-6-ethoxy-7-[(1-methylpiperidin-4-- yl)methoxy]quinoline-3-carbonitrile; 4-[(2,4-Dichloro-5-methoxyphenyl)amino]-6-ethoxy-7-[3-(4-ethylpiperazin-1- -yl)propoxy]quinoline-3-carbonitrile; 4-[(2,4-Dichloro-5-methoxyphenyl)amino]-6-ethoxy-7-[3-(1-methylpiperidin-- 4-yl)propoxy]quinoline-3-carbonitrile; 4-[(2,4-Dichloro-5-methoxyphenyl)amino]-6-ethoxy-7-[2-(4-methyl-1-piperaz- inyl)ethoxy]quinoline-3-carbonitrile; 4-[(2,4-Dichloro-5-methoxyphenyl)amino]-6-ethoxy-7-[2-(1-methylpiperidin-- 4-yl)ethoxy]quinoline-3-carbonitrile; or 4-[(2,4-Dichloro-5-methoxyphenyl)amino]-6-methoxy-7-[3-(4-propyl-1-pipera- zinyl)propoxy]-3-quinolinecarbonitrile; and pharmaceutically acceptable salts thereof.

According to a particular aspect, the agent which possesses inhibitory activity against the Src family kinase is a small molecule agent.

According to a particular aspect, the agent which possesses inhibitory activity against the Src family kinase is a chemical agent.

Suitable compounds possessing inhibitory activity against the Src family of non-receptor tyrosine kinases include the quinazoline derivatives disclosed in International Patent Applications WO 01/94341, WO 02/16352, WO 02/30924, WO 02/30926, WO 02/34744, WO 02/085895, WO 02/092577 (arising from PCT/GB 02/02117), WO 02/092578 (arising from PCT/GB 02/02124) and WO 02/092579 (arising from PCT/GB 02/02128), the quinoline derivatives described in WO 03/008409 (arising from PCT/GB 02/03177), WO 03/047584 and WO 03/048159 and the quinazoline derivatives described in European Patent Applications 02292736.2 (filed 4 Nov. 2002) and 03290900.4 (filed 10 Apr. 2003).

It is disclosed in Journal Medicinal Chemistry, 2001, 44, 822-833 and 3965-3977 that certain 4-anilino-3-cyanoquinoline derivatives are useful for the inhibition of Src-dependent cell proliferation. The 4-anilino-3-cyanoquinoline Src inhibitor known as SKI 606 is described in Cancer Research, 2003, 63, 375.

Other compounds which possess Src kinase inhibitory properties are described in, for example, International Patent Applications WO 96/10028, WO 97/07131, WO 97/08193, WO 97/16452, WO 97/28161, WO 97/32879 and WO 97/49706.

Other compounds which possess Src kinase inhibitory properties are described in, for example, J Bone Mineral Research, 1999, 14 (Suppl. 1), S487, Molecular Cell, 1999, 3, 639-647, Journal Medicinal Chemistry, 1997, 40, 2296-2303, Journal Medicinal Chemistry, 1998, 41, 3276-3292 and Bioorganic & Medicinal Chemistry Letters, 2002, 12, 1361 and 3153.

Particular Src kinase inhibitors include the following:

(i) 4-amino-5-(3-methoxyphenyl)-7-{(4-[2-(2-methoxyethylamino)ethox- y]phenyl)-}-pyrrolo[2,3-d]pyrimidine and 4-amino-5-(3-methoxyphenyl)-7-(4-{(2-[di-(2-methoxyethyl)amino]ethoxy}phe- nyl)pyrrolo[2,3-d]pyrimidine which are obtainable by methods described in International Patent Application WO 96/10028:

(ii) 4-amino-7-tert-butyl-5-(4-tolyl)pyrazolo[3,4-d]pyrimidine which is also known as PP1 and is described in Molecular Cell, 1999, 3, 639-648;

(iii) 2-(2,6-dichloroanilino)-6,7-dimethyl-1,8-dihydroimidazo[4,5-h]isoquinolin- -9-one and 2-(2,6-dichloroanilino)-7-[(E)-3-diethylaminoprop-1-enyl]-6-met--hyl-1,8-dihydroimidazo[4,5-h]isoquinolin-9-one which are obtainable by methods described in Journal Medicinal Chemistry, 2002, 45, 3394;

(iv) 1-[6-(2,6-dichlorophenyl)-2-(4-diethylaminobutyl)pyrido[2,3-d]pyrimidin-7- -yl]-3-ethylurea which is obtainable by methods described in Journal Medicinal Chemistry, 1997, 40, 2296-2303 and Journal Medicinal Chemistry, 2001, 44, 1915;

(v) 6-(2,6-dichlorophenyl)-2-[4-(2-diethylaminoethoxy)anilino]-8-me- thyl-8H-pyrido[2,3-d]pyrimidin-7-one which is also known as PD166285 and is described in J. Pharmacol. Exp. Ther., 1997, 283, 1433-1444;

(vi) the compound known as PD 162531 which is described in Mol. Biol. Cell, 2000, 11, 51-64;

(vii) the compound known as PD166326 which is described in Biochem Pharmacol., 2000, 60, 885-898; and

(viii) the compound known as PD173955 which is described in Cancer Research, 1999, 59, 6145-6152.

Other compounds which may possess Src kinase inhibitory properties are described in, for example, International Patent Applications WO 02/079192, WO 03/000188, WO 03/000266, WO 03/000705, WO 02/083668, WO 02/092573, WO 03/004492, WO 00/49018, WO 03/013541, WO 01/00207, WO 01/00213 and WO 01/00214.

Particular Src inhibitors include those provided in International Patent Application WO 01/94341.

Further particular Src inhibitors include the following compounds from International Patent Application WO 02/16352, WO 02/30924, WO 02/30926 and WO 02/34744.

Exemplary agents include, without limitation, dasatinib, and AZD0530.

Other exemplary agents include CGP77675 (AXON MEDCHEM 2097), SU 6656, AZD0530, Dasatinib, Bosutinib and WH-4-023.

According to some aspects of the disclosure, the Src family kinase inhibitor (e.g. CGP77675) is provided at a concentration range of between about 0.1-70 μM, e.g., from about 0.2 μM to about 70 μM, e.g., between about 0.2-60 μM, e.g., between about 0.2-55 μM, e.g., between about 0.2-50 μM, e.g., between about 0.2-45 μM, e.g., between about 0.2-40 μM, e.g., between about 0.2-35 μM, e.g., between about 0.2-30 μM, e.g., between about 0.2-25 μM, e.g., between about 0.2-20 μM, e.g., between about 0.2-15 μM, e.g., between about 0.2-10 μM, e.g., between about 0.3-10 μM, e.g., between about 0.4-10 μM, e.g., between about 0.5-10 μM, e.g., between about 0.6-10 μM, e.g., between about 0.7-10 μM, e.g., between 0.8-10 μM, e.g., between 0.9-10 μM, e.g., between 0.9-9 μM, e.g., between 1-8 μM, e.g., between 1-7 μM, e.g., between 1-6 μM, e.g., between 1-5 μM, e.g., about 1-3 μM, e.g., about 1.5 μM.

Since SRC inhibition leads to NPKβ signaling inhibition, the present inventors contemplate use of NFKβ pathway inhibitors instead of the SRC inhibitors.

Examples of small molecule NFKβ inhibitors include, but are not limited to Rolipram, JSH-23 and LY 294002. Exemplary concentrations the NFKβ inhibitors may be used is between 0.1-10 μM.

As mentioned, the media described herein also comprise a protein kinase C inhibitor.

As used herein the term "protein kinase C inhibitor" refers to any molecule capable of inhibiting the activity of protein kinase C as determined by reducing the levels of phosphorylated versus non phosphorylated PKC isoforms. According to a particular aspect, the PKC inhibitor is a small molecule inhibitor.

A non-limiting example of a protein kinase C inhibitor is Go6983 (CAS 133053-19-7), a potent, cell-permeable, reversible, and ATP-competitive inhibitor of protein kinase C (PKC) with a broad spectrum protein kinase C (PKC) inhibitor (IC50 values are 7, 7, 6, 10, 60 and 20000 nM for PKCα, PKCβ, PKCγ, PKCδ, PKCζ and PKCμ respectively). Go6983 is available from various suppliers such as Calbiochem (Catalogue number 365251-500UG), and TOCRIS (Catalogue number 2285).

According to some aspects of the disclosure, Go6983 is provided at a concentration range of between about

0.1-100 μM, e.g., from about 0.5 μM to about 100 μM, e.g., between about 0.5-50 μM, 0.5-25 μM, e.g., between about 1-20 μM, e.g., between about 1-10 μM, e.g., between about 1-5 μM, e.g., about 2 μM.

Additional agents that may be added to the medium include a STAT3 activator, an ERK inhibitor, a p38 inhibitor and a ROCK inhibitor each of which will be described herein below.

As used herein the term "STATS" refers to the signal transducer and activator of transcription 3 gene product (acute-phase response factor) (Gene ID 6774). In response to cytokines and growth factors, STAT family members are phosphorylated by the receptor associated kinases, and then form homo - or heterodimers that translocate to the cell nucleus where they act as transcription activators. Known STAT3 activators include, but are not limited to, interferon (IFN), epidermal growth factor (EGF), interleukin 5 (IL5), interleukin 6 (II,6), hepatocyte growth factor (HGF), leukemia inhibitory factor (LIF) and bone morphogenetic protein 2 (BMP2).

According to some aspects of the disclosure, the STAT3 activator, which is used in the medium of some aspects of the disclosure is selected from the group consisting of LIF, IL6 and EGF.

According to some aspects of the disclosure, the STAT3 activator, which is used in the medium of some aspects of the disclosure is selected from the group consisting of LIF and IL6.

According to some aspects of the disclosure, the STAT3 activator, which is used in the medium of some aspects of the disclosure is LIF.

As used herein the term "leukemia inhibitor factor (LIF)" refers to a polypeptide which comprises the amino acid sequence as set forth by GenBank Accession No. NP_001244064.1 (SEQ ID NO: 11), encoded by the nucleotide sequence set forth in GenBank Accession No. NM_001257135 (SEQ ID NO: 12). Preferably, the LIF used by the method according to some aspects of the disclosure is capable of supporting, along with other factors which are described herein, the undifferentiated growth of naive primate (e.g., human) PSCs, while maintaining their pluripotent capacity. LIF can be obtained from various manufacturers such as Millipore, Peprotech, and R&D systems.

According to some aspects of the disclosure, LIF is provided at a concentration range from about 0.5 nanogram per milliliter (ng/ml) to about 1000 ng/ml, e.g., about 1-1000 ng/ml, e.g., about 1-900 ng/ml, e.g., about 1-800 ng/ml, e.g., about 1-700 ng/ml, e.g., about 1-600 ng/ml, e.g., about 1-500 ng/ml, e.g., about 1-400 ng/ml, e.g., about 1-300 ng/ml, e.g., about 1-200 ng/ml, e.g., about 1-100 ng/ml, e.g., about 1-50 ng/ml, e.g., about 2-50 ng/ml, e.g., about 4-50 ng/ml, e.g., about 5-50 ng/ml, e.g., about 10-50 ng/ml, e.g., about 10-40 ng/ml, e.g., about 10-30 ng/ml, e.g., about 20 ng/ml.

As used herein the term "interleukin 6 (II,6)" refers to a polypeptide which comprises the amino acid sequence set forth by GenBank Accession No. NP_000591.1 (SEQ ID NO: 13), which is encoded by the nucleic acid set forth by GenBank Accession No. NM_000600.3 (SEQ ID NO: 14). Preferably, the IL6 used by the method according to some aspects of the disclosure is capable of supporting, along with other factors which are described herein, the undifferentiated growth of naive primate (e.g., human) PSCs, while maintaining their pluripotent capacity. II,6 can be obtained from various manufacturers such as Speed BioSystems, Millipore, Peprotech, and R&D systems.

According to some aspects of the disclosure, IL6 is provided at a concentration range from about 0.1 ng/ml to about 100 ng/ml, e.g., about 0.1-90 ng/ml, e.g., about 0.1-80 ng/ml, e.g., about 0.1-70 ng/ml, e.g., about 0.1-50 ng/ml, e.g., about 0.1-40 ng/ml, e.g., about 0.1-30 ng/ml, e.g., about 0.1-20 ng/ml, e.g., about 0.1-10 ng/ml, e.g., about 0.1-8 ng/ml, e.g., about 0.1-7 ng/ml, e.g., about 0.1-6 ng/ml, e.g., about 0.1-5 ng/ml, e.g., about 0.1-4 ng/ml, e.g., about 0.1-3 ng/ml, e.g., about 0.1-4 ng/ml, e.g., about 0.5-4 ng/ml, e.g., about 0.5-4 ng/ml, e.g., about 3 ng/ml.

As used herein the term "p38" refers to the "p38α (alpha)" mitogen-activated protein kinase 14 (MAPK14), which includes MAPK14 isoform 1 set forth by GenBank Accession No. NP_001306.1 (SEQ ID NO: 15), MAPK14 isoform 2 set forth by GenBank Accession No. NP_620581.1 (SEQ ID NO: 16), MAPK14 isoform 3 set forth by GenBank Accession No. NP_620582.1 (SEQ ID NO: 17) and MAPK14 isoform 4 set forth by GenBank Accession No. NP_620583.1 (SEQ ID NO: 18); "ρ38β (beta)" (MAPK11), which is set forth by GenBank Accession No. NP_002742.3 (SEQ ID NO: 19); "ρ38γ (gamma)" (MAPK12) which is set forth by GenBank Accession No. NP_002960.2 (SEQ ID NO: 20); and/or "p38δ (delta)" (MAPK13) which is set forth in GenBank Accession No. NP_002745.1 (SEQ ID NO: 21), all of them having kinase activity and involved in signal transduction.

As used herein the term "p38 inhibitor" refers to any molecule (e.g., small molecules or proteins) capable of inhibiting the activity of p38 family members as determined by Western blot quantification of phosphorylated p38 levels.

Non-limiting examples of p38 inhibitors include SB203580 (AXONMEDCHEM - Axon 1363), and SB 202190 (AXONMEDCHEM - Axon 1364), LY 2228820 (AXONMEDCHEM-Axon 1895), BIRB0796 (Axon Medchem 1358) and PD169316 (AXONMEDCHEM - Axon 1365).

As BMP signaling is an activator for p38 signaling, examples of p38 inhibitors also include BMP inhibitors like Dorsomorphin (AXONMEDCHEM - Axon 2150) and LDN193189 (AXON MEDCHEM AXON 1509) or other inhibitors of the BMP pathway such as recombinant NOGGIN protein [GenBank Accession No. NP_005441.1 (SEQ ID NO: 22] can be used to replace small molecule inhibitors of BMP signaling.

According to some aspects of the disclosure, SB203580 is provided at a concentration range of between about 0.5-70 μM, e.g., from about 1 μM to about 70 μM, e.g., between about 1-60 μM, e.g., between about 1-55 μM, e.g., between about 1-50 μM, e.g., between about 1-45 μM, e.g., between about 1-40 μM, e.g., between about 1-35 μM,

e.g., between about 1-30 μM, e.g., between about 1-25 μM, e.g., between about 1-20 μM, e.g., between about 1-15 μM, e.g., between about 1-10 μM, e.g., between about 2-10 μM, e.g., between about 3-10 μM, e.g., between about 4-10 μM, e.g., between about 4-6 μM, e.g., about 5 μM, e.g., about 10 μM.

According to some aspects of the disclosure, SB 202190 is provided at a concentration range of between about 0.1 μM to about 50 μM, e.g., from about 0.5 μM to about 50 μM, e.g., from about 1 μM to about 50 μM, e.g., between about 1-45 μM, e.g., between about 1-40 μM, e.g., between about 1-35 μM, e.g., between about 1-30 μM, e.g., between about 1-25 μM, e.g., between about 1-20 μM, e.g., between about 1-15 μM, e.g., between about 1-10 μM, e.g., between about 1-9 μM, e.g., between about 1-8 μM, e.g., between about 1-7 μM, e.g., between about 2-7 μM, e.g., between about 3-7 μM, e.g., between about 4-7 μM, e.g., between about 4-6 μM, e.g., about 5 μM.

According to some aspects of the disclosure, BIRB0796 is provided at a concentration range of between about 0.05 to about 30 μM, e.g., from about 0.1 to about 30 μM, e.g., between about 0.2-30 μM, e.g., between about 0.2-25 μM, e.g., between about 0.2-20 μM, e.g., between about 0.2-15 μM, e.g., between about 0.2-10 μM, e.g., between about 0.2-8 μM, e.g., between about 0.2-6 μM, e.g., between about 0.5-6 μM, e.g., between about 0.5-5 μM, e.g., between about 0.5-4 μM, e.g., between about 0.5-3 μM, e.g., between about 0.5-2 μM, e.g., between about 1-3 μM, e.g., between about 1-2.5 μM, e.g., about 2 μM.

As used herein the term "ROCK" refers to the protein set forth by GenBank Accession No. NP_005397.1 (P160ROCK; SEQ ID NO: 23); and NP_004841.2 (ROCK2; SEQ ID NO: 24) having the serine/threonine kinase activity, and regulates cytokinesis, smooth muscle contraction, the formation of actin stress fibers and focal adhesions, and the activation of the c-fos serum response element.

As used herein the term "ROCK inhibitor" refers to any molecule capable of inhibiting the activity of ROCK as determined by inhibition of ROCK phosphorylation levels (detected by western blot analysis).

According to a particular aspect, the ROCK inhibitor is a small molecule agent.

Non-limiting examples of ROCK inhibitors include Y27632 (TOCRIS, Catalogue number 1254).

According to some aspects of the disclosure, Y27632 is provided at a concentration range of between about 0.1-100 μM, e.g., from about 0.1 μM to about 90 μM, e.g., between about 0.1-85 μM, e.g., between about 0.1-80 μM, e.g., between about 0.1-70 μM, e.g., between about 0.1-60 μM, e.g., between about 0.1-55 μM, e.g., between about 0.1-50 μM, e.g., between about 0.1-45 μM, e.g., between about 0.1-40 μM, e.g., between about 0.1-35 μM, e.g., between about 0.1-30 μM, e.g., between about 0.1-25 μM, e.g., between about 0.1-10 μM, e.g., between about 0.1-5 μM, e.g., between about 0.5-5 μM, e.g., between about 0.5-2 μM, e.g. between about 1-5 μM, e.g., about 1 μM.

It will be appreciated that instead of a ROCK inhibitor, the present inventors contemplate using an inhibitor of JNK.

As used herein the term "JNK" refers to the mitogen-activated protein kinase 8 (MAPK8) protein set forth by GenBank Accession Nos. NP_620637.1 (isoform alpha2) (SEQ ID NO: 25), NP_620635.1 (isoform beta2) (SEQ ID NO: 26), NP_620634.1 (isoform beta1) (SEQ ID NO: 27), NP_002741.1 (isoform alpha1) (SEQ ID NO: 28) which are involved in a wide variety of cellular processes such as proliferation, differentiation, transcription regulation and development.

As used herein the term "JNK inhibitor" refers to any molecule (e.g. small molecule) capable of inhibiting the activity of JNK as determined by phosphorylation of JNK family member protein by western blot analysis.

Non-limiting examples of JNK inhibitors include SP600125 (TOCRIS - Cat no. 1496), AEG3482 (AXONMEDCHEM- AXON 1291), BIX02189, BRAFi (SB590885) and BIRB796 (AXONMEDCHEM - Axon 1358).

According to some aspects of the disclosure, SP600125 is provided at a concentration range of between about 0.5-100 μM, e.g., from about 1 μM to about 100 μM, e.g., between about 1-90 μM, e.g., between about 1-80 μM, e.g., between about 1-70 μM, e.g., between about 1-60 μM, e.g., between about 1-55 μM, e.g., between about 1-50 μM, e.g., between about 1-45 μM, e.g., between about 1-40 μM, e.g., between about 1-35 μM, e.g., between about 1-30 μM, e.g., between about 1-25 μM, e.g., between about 1-20 μM, e.g., between about 1-15 μM, e.g., between about 1-10 μM, e.g., between about 2-10 μM, e.g., between about 3-10 μM, e.g., between about 4-10 μM, e.g., between about 4-6 μM, e.g., about 5 μM.

According to some aspects of the disclosure, BIX02189 is provided at a concentration range of between about 0.5-100 μM, e.g., from about 1 μM to about 100 μM, e.g., between about 1-90 μM, e.g., between about 1-80 μM, e.g., between about 1-70 μM, e.g., between about 1-60 μM, e.g., between about 1-55 μM, e.g., between about 1-50 μM, e.g., between about 1-45 μM, e.g., between about 1-40 μM, e.g., between about 1-35 μM, e.g., between about 1-30 μM, e.g., between about 1-25 μM, e.g., between about 1-20 μM, e.g., between about 1-15 μM, e.g., between about 1-10 μM, e.g., between about 2-10 μM, e.g., between about 3-10 μM, e.g., between about 4-10 μM, e.g., between about 4-6 μM, e.g., about 5 μM.

According to some aspects of the disclosure, BRAFi (SB590885) is provided at a concentration range of between about 0.1-100 μM, e.g., between about 0.1-90 μM, e.g., between about 0.1-80 μM, e.g., between about 0.1-70 μM, e.g., between about 0.1-60 μM, e.g., between about 0.1-50 μM, e.g., between about 0.1-40 μM, e.g., between about 0.1-30 μM, e.g., between about 0.1-20 μM, e.g., between about 0.1-10 μM, e.g., between about 0.1-5 μM, e.g., between about 0.1-2 μM, e.g., between about 0.1-1 μM, e.g., about 0.5 μM.

As used herein the term "ERK1" refers to the mitogen-activated protein kinase 3 (MAPK3) isoform 1 set forth by GenBank Accession No. NP_002737.2 (SEQ ID NO: 29), the MAPK3 isoform 2 set forth by GenBank Accession No. NP_001035145.1 (SEQ ID NO: 30), the MAPK3 isoform 3 set forth by GenBank Accession No. NP_001103361.1 (SEQ ID NO: 31) and/or ERK1 set forth in GenBank Accession No. M84490 (SEQ ID NO: 32) having the MAPK signaling activity.

As used herein the term "ERK2" refers to the mitogen-activated protein kinase 1 (MAPK1) set forth by GenBank Accession No. NP_002736.3 (SEQ ID NO: 33) and/or GenBank Accession No. NP_620407.1 (SEQ ID NO: 34) having the MAPK signaling activity.

As used herein the term "ERK1/2 inhibitor" refers to any molecule capable of inhibiting the activity of ERK1/2 as determined by Western blot protein detection of phosphorylated ERK1/2 proteins.
According to a particular aspect, the ERK1/2 inhibitor is a small molecule agent.

Non-limiting examples of ERK1/2 inhibitors (also known as MEK1/2 inhibitors) include PD0325901 (AXONMEDCHEM - AXON 1408), PD98059 (AXONMEDCHEM - Axon 1223), and PD184352 (AXONMEDCHEM - AXON 1368); and/or even inhibitors of RAF (which is upstream of MEK/ERK pathway) such as Sorafenib tosylate (also known as BAY 43-9006 AXONMEDCHEM -AXON 1397) or SB 590885 (TOCRIS #2650).

According to some aspects of the disclosure, PD0325901 is provided at a concentration range from about 0.01 microM (μM) to about 50 μM, e.g., between about 0.05-45 μM, e.g., between about 0.1-50 μM, e.g., between about 0.1-45 μM, e.g., between about 0.1-40 μM, e.g., between about 0.1-35 μM, e.g., between about 0.1-30 μM, e.g., between about 0.1-25 μM, e.g., between about 0.1-20 μM, e.g., between about 0.1-15 μM, e.g., between about 0.1-10 μM, e.g., between about 0.2-10 μM, e.g., between about 0.3-10 μM, e.g., between about 0.4-10 μM, e.g., between about 0.5-10 μM, e.g., between about 0.6-10 μM, e.g., between about 0.7-10 μM, e.g., between 0.8-10 μM, e.g., between 0.9-10 μM, e.g., between 0.9-9 μM, e.g., between 0.9-8 μM, e.g., between 0.9-7 μM, e.g., between 0.9-6 μM, e.g., between 0.8-5 μM, e.g., between 0.8-4 μM, e.g., between 0.8-3 μM, e.g., between 0.8-2 μM, e.g., between 0.8-1.5 μM, e.g., between 0.9-1.2 μM, e.g., about 1 μM.

According to some aspects of the disclosure, PD98059 is provided at a concentration range from about 0.1 microM (μM) to about 70 μM, e.g., between about 0.1-65 μM, e.g., between about 0.1-55 μM, e.g., between about 0.1-50 μM, e.g., between about 0.1-45 μM, e.g., between about 0.1-40 μM, e.g., between about 0.1-35 μM, e.g., between about 0.1-30 μM, e.g., between about 0.1-25 μM, e.g., between about 0.1-20 μM, e.g., between about 0.1-15 μM, e.g., between about 2-20 μM, e.g., between about 5-15 μM, e.g., about 10 μM, e.g., between about 0.1-10 μM, e.g., between about 0.2-10 μM, e.g., between about 0.3-10 μM, e.g., between about 0.4-10 μM, e.g., between about 0.5-10 μM, e.g., between about 0.6-10 μM, e.g., between about 0.7-10 μM, e.g., between 0.8-10 μM, e.g., between 0.9-10 μM, e.g., between 0.9-9 μM, e.g., between 0.9-8 μM, e.g., between 0.9-7 μM, e.g., between 0.9-6 μM, e.g., between 0.8-5 μM, e.g., between 0.8-4 μM, e.g., between 0.8-3 μM, e.g., between 0.8-2 μM, e.g., between 0.8-1.5 μM, e.g., between 0.9-1.2 μM.

According to some aspects of the disclosure, PD184352 is provided at a concentration range from about 0.1 microM (μM) to about 70 μM, e.g., between about 0.1-60 μM, e.g., between about 0.1-50 μM, e.g., between about 0.5-50 μM, e.g., between about 0.5-45 μM, e.g., between about 0.5-40 μM, e.g., between about 0.1-35 μM, e.g., between about 0.5-30 μM, e.g., between about 0.5-25 μM, e.g., between about 0.5-20 μM, e.g., between about 0.5-15 μM, e.g., between about 0.5-10 μM, e.g., between 0.5-9 μM, e.g., between 0.5-8 μM, e.g., between 0.5-7 μM, e.g., between 0.9-6 μM, e.g., between 0.8-5 μM, e.g., between 0.8-4 μM, e.g., between 0.8-3 μM, e.g., about 3 μM. e.g., between 0.8-2 μM, e.g., between 0.8-1.5 μM, e.g., between 0.9-1.2 μM.

According to some aspects of the disclosure, Sorafenib is provided at a concentration range from about 0.1 microM (μM) to about 70 μM, e.g., between about 0.1-60 μM, e.g., between about 0.1-50 μM, e.g., between about 0.5-50 μM, e.g., between about 0.5-45 μM, e.g., between about 0.5-40 μM, e.g., between about 0.1-35 μM, e.g., between about 0.5-30 μM, e.g., between about 0.5-25 μM, e.g., between about 0.5-20 μM, e.g., between about 0.5-15 μM, e.g., between about 0.5-10 μM, e.g., between 0.5-9 μM, e.g., between 0.5-8 μM, e.g., between 0.5-7 μM, e.g., between 0.9-6 μM, e.g., between 0.8-5 μM, e.g., about 5 μM, e.g., between 0.8-4 μM, e.g., between 0.8-3 μM, e.g., between 0.8-2 μM, e.g., between 0.8-1.5 μM, e.g., between 0.9-1.2 μM.

A particular contemplated media is one which comprises each of the following components: LIF, WNT inhibitor, ERK inhibitor, P38 inhibitor, PKC inhibitor SRC inhibitor and Rock inhibitor.

In some cases the amount of ERK1/2 inhibitor present in the medium is less than 0.5 μM, for example about 0.4 μM, 0.3 μM, 0.2 μM or 0.1 μM. In some cases, the medium is devoid of ERK1/2 inhibitor. It will be appreciated that the phrase "being devoid of ERK1/2 inhibitors" refers to a medium in which no ERK1/2 inhibitors have been positively added to a medium and does not mean to exclude trace amounts of ERK1/2 inhibitors contained in the base medium.

The present inventors contemplate addition of an activator of the TGF-Activin pathway to the medium when the ERK1/2 in the medium is less than 0.5 μM.

According to some aspects of the disclosure, activators of TGF/ACTIVIN pathway including ACTIVIN A (also known as Inhibin beta A, INHBA, Gene ID: 3624; GenBank Accession No. NM_002192.2 (SEQ ID NO: 35), which encodes GenBank Accession No. NP_002183.1; SEQ ID NO: 36).

Preferably the amount of ACTIVIN A added is between 1-100 ng/ml and more preferably between 1-10 ng/ml (for example about 4 ng/ml).

NOTCH signaling inhibitors may also be included in the media of the present disclosure. Preferably, the NOTCH signaling inhibitor is added when the medium comprises less than 0.5 μM, for example about 0.4 μM, 0.3 μM, 0.2 μM or 0.1 μM ERK1/2 inhibitor. NOTCH signaling inhibitors include, but are not limited to the following gamma secretase inhibitors: DAPT (Axon Medchem 1484 - 0.05-50 μM final concentration), LY2886721 hydrochloride (Axon Medchem 1964 - 0.05-50 μM final concentration)], DBZ (Axon Medchem - Axon 1488-0.05-50 μM final concentration).

A particular contemplated media is one which comprises each of the following components: LIF, WNT inhibitor, Notch inhibitor, P38 inhibitor, PKC inhibitor SRC inhibitor, Activin A and Rock inhibitor.

In one aspect, the media of the present disclosure are devoid of exogenously added TGF (e.g. TGFβ1, TGFβ2) and FGF (e.g. bFGF). A medium devoid of TGF or FGF refers to a medium which does not comprise TGF or FGF in an amount that has an effect on the mitogenic activity of pluripotent cells cultured within. In one aspect, "being devoid of TGF or FGF" refers to a medium in which no TGF or FGF has been positively added to a medium and does not mean to exclude trace amounts TGF or FGF contained in the base medium.

Additional agents that may be added to the media of the present disclosure include at least one, at least two, at least three, at least four, at least five, at least six or more of the following agents: a ROCK inhibitor, Ascorbic acid, NFKb inhibitor, a YAP/TAZ inhibitor, an SHH inhibitor, a TGFβR inhibitor, a BMP inhibitor, an FGFR inhibitor, a JNK inhibitor, an ERK5 inhibitor, a BRAF inhibitor, an ARAFi, a CRAFi, a p38 inhibitor, an LSD1 inhibitor, a PI3K activator, a SMAD activator and a DOT1L inhibitor, Forskolin, Kenpaullone, BayK8644, an inhibitor of G9a, an inhibitor of Glp, stem cell factor (SCF), insulin-like growth factor 1 (IGF1), insulin-like growth factor II (IGFII), Mbd3/Gatad2a/NuRD complex inhibitor, HDAC inhibitor, Recombinant human Vitronectin, Recombinant human Laminin and Recombinant human Biolaminin.

Additional components that may be added to the media of this aspect of the present are disclosed in WO2014/174470.

The media described herein can be used to culture cells. Thus, according to an aspect of some aspects of the disclosure, there is provided a cell culture comprising cells and the culture medium of some aspects of the disclosure.

The cells may be any cells, e.g., prokaryotic or eukaryotic cells, e.g., primate cells, e.g., mammalian cells, e.g., human cells.

According to some aspects of the disclosure, the cells are somatic cells, pluripotent stem cells (PSCs), primed pluripotent stem cells, non-naive pluripotent stem cell and/or naive pluripotent stem cells.

According to some aspects of the disclosure, the culture medium is capable of maintaining pluripotent stem cells in an undifferentiated state for at least 2 passages, e.g., for at least 5, 10, 20, 30, 40, 50, 60, 70, 80, 90 or 100 passages. The pluripotent stem cells cultured in the presently disclosed media retain their hypomethylated state for the number of passages.

According to some aspects of the disclosure, the pluripotent stem cells are primate pluripotent stem cell (homo sapiens (human), monkey, chimpanzee, Gorillas, Rhesus and/or Baboon). Other pluripotent stem cells contemplated by the present disclosure are swine (porcine) pluripotent stem cells.

Preferably, the pluripotent stem cells are not rodent pluripotent stem cells.

In one aspect, the pluripotent stem cell is a naive pluripotent stem cell.

The phrase "naive pluripotent stem cell (PSC) " refers to a cell capable of forming a PSC, and that exhibits a pre-X-inactivation state, and therefore is considered to be the origin of the PSC.

The pre-X-inactivation state according to some aspects of the disclosure is characterized by presence of two unmethylated alleles of an X-inactive specific transcript (XIST) gene in the female cell, and presence an unmethylated allele of the XIST gene in a male cell.

The XIST gene is located on human Xq13.2 chromosome and has the sequence depicted in clone NC_000023.10 (73040486.73072588, complement, based on GenBank version GRCh37.p10. The XIST gene has a non-coding RNA which is provided in GenBank Accession NO. NR_001564.2 (SEQ ID NO: 37).

According to some aspects of the disclosure, presence of two unmethylated alleles of XIST gene in a female cell refers to having below about 20% of CpG methylated reads sequenced in the XIST promoter, e.g., below about 19%, below about 18%, below about 17%, below about 16%, below about 15%, below about 14%, below about 13%, below about 12%, below about 11%, below about 10%, below about 9%, below about 8%, below about 7%, below about 6%, below about 5%, below about 4%, below about 3%, below about 2%, below about 1%, e.g., 0% (e.g., complete absence) of CpG methylated reads sequenced in the XIST promoter.

According to some aspects of the disclosure, presence of one unmethylated allele of XIST gene in a male cell refers to having below about 20% of CpG methylated reads sequenced in the XIST promoter, e.g., below about 19%, below about 18%, below about 17%, below about 16%, below about 15%, below about 14%, below about 13%, below about 12%, below about 11%, below about 10%, below about 9%, below about 8%, below about 7%, below about 6%, below about 5%, below about 4%, below about 3%, below about 2%, below about 1%, e.g., 0% of CpG methylated reads sequenced in the XIST promoter.

A non-limited example of the XIST promoter which includes CpG islands which can be either methylated or unmethylated is provided in the XIST promoter amplicon set forth by SEQ ID NO: 38.

According to some aspects of the disclosure, the human naive PSC is characterized by a reduced methylation of CpG islands as compared to a level of methylation of the CpG islands in a human primed PSC.

Some human naive ESCs are characterized by significantly low levels of total methylated cytosine out of the total guanine nucleotides in each cell (e.g., 1-2%,) as determined by Liquid Chromatography - Mass Spectrometry (LC-MS) quantitative analysis.

According to some aspects of the disclosure, the human naive PSC is characterized by 0-3% of total methylated cytosine out of the total Guanine nucleotides in the naive PSC cell. For comparison, the primed PSC or a somatic cell has between 3.5%-5% of total methylated cytosine out of the total Guanine nucleotides in the primed PSC cell.

Thus, the naive pluripotent stem cell of some aspects of the disclosure is in a naive, hypomethylated state (relating to global levels of DNA methylation). For example in one aspect, less than 70 % of the cytosines of a CG sequence of the DNA of the naive pluripotent stem cell are methylated, less than 60 % of the cytosines of a CG sequence of the DNA of the naive pluripotent stem cell are methylated, less than 50 % of the cytosines of a CG sequence of the DNA of the naive pluripotent stem cell are methylated.

As used herein the phrase "naive state" refers to being in an undifferentiated state wherein both alleles of the X-inactive specific transcript (XIST) gene of the female cell are unmethylated, or wherein the XIST allele of the male cell is unmethylated.

It should be noted that the naive PSCs of some aspects of the disclosure (which are in a pre-X inactivation and a naive state) can upon differentiation inactivate one of the X chromosome alleles and methylate one of the XIST genes.

As used herein the term *"isolated"* refers to at least partially separated from the natural environment e.g., from the primate (e.g., mammalian) embryo or the primate (e.g., mammalian) body.

According to some aspects of the disclosure, the non-naive PSC is selected from the group consisting of a primed PSC, an embryonic stem cell, a non-human blastocyst, an induced pluripotent stem cell (a primed iPSC) and a somatic cell.

The phrase "embryonic stem cells" refers to embryonic cells which are capable of differentiating into cells of all three embryonic germ layers *(i.e.,* endoderm, ectoderm and mesoderm), or remaining in an undifferentiated state. The phrase "embryonic stem cells" may comprise cells which are obtained from the non-human embryonic tissue formed after gestation (e.g., blastocyst) before implantation of the non-human embryo (*i.e.,* a pre-implantation blastocyst), extended non-human blastocyst cells (EBCs) which are obtained from a post-implantation/pre-gastrulation stage blastocyst (see WO2006/040763) and embryonic germ (EG) cells which are obtained from the genital tissue of a fetus any time during gestation, preferably before 10 weeks of gestation.

Induced pluripotent stem cells (iPS; embryonic-like stem cells), are cells obtained by de-differentiation of adult somatic cells which are endowed with pluripotency (*i.e.,* being capable of differentiating into the three embryonic germ cell layers, *i.e.,* endoderm, ectoderm and mesoderm). According to some aspects of the disclosure, such cells are obtained from a differentiated tissue (e.g., a somatic tissue such as skin) and undergo de-differentiation by genetic manipulation which re-program the cell to acquire embryonic stem cells characteristics. According to some aspects of the disclosure, the induced pluripotent stem cells are formed by inducing the expression of Oct-4, Sox2, Kfl4 and c-Myc in a somatic stem cell.

The embryonic stem cells of some aspects of the disclosure can be obtained using well-known cell-culture methods. For reference only, human embryonic stem cells can be isolated from human blastocysts. Human blastocysts are typically obtained from human *in vivo* preimplantation embryos or from *in vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 4-7 days. For further details on methods of preparation human ES cells see Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; and Gardner et al., [Fertil. Steril. 69: 84, 1998].

Another method for preparing ES cells is described in Chung et al., Cell Stem Cell, Volume 2, Issue 2, 113-117, 7 February 2008. This method comprises removing a single cell from an embryo during an in vitro fertilization process. The embryo is not destroyed in this process.

It will be appreciated that commercially available stem cells can also be used according to some aspects of the disclosure. Human ES cells can be purchased from the NIH human embryonic stem cells registry [Hypertext Transfer Protocol://grants (dot) nih (dot) gov/stem_cells/registry/current (dot) htm]. For reference, non-limiting examples of commercially available embryonic stem cell lines are BG01, BG02, BG03, BG04, CY12, CY30, CY92, CY10, TE03, TE32, CHB-4, CHB-5, CHB-6, CHB-8, CHB-9, CHB-10, CHB-11, CHB-12, HUES 1, HUES 2, HUES 3, HUES 4, HUES 5, HUES 6, HUES 7, HUES 8, HUES 9, HUES 10, HUES 11, HUES 12, HUES 13, HUES 14, HUES 15, HUES 16, HUES 17, HUES 18, HUES 19, HUES 20, HUES 21, HUES 22, HUES 23, HUES 24, HUES 25, HUES 26, HUES 27, HUES 28, CyT49, RUES3, WA01, UCSF4, NYUES1, NYUES2, NYUES3, NYUES4, NYUES5, NYUES6, NYUES7, UCLA 1, UCLA 2, UCLA 3, WA077 (H7), WA09 (H9), WA13 (H13), WA14 (H14), HUES 62, HUES 63, HUES 64, CT1, CT2, CT3, CT4, MA135, Eneavour-2, WIBR1, WIBR2, WIBR3, WIBR4, WIBR5, WIBR6, HUES 45, Shef 3, Shef 6, BJNhem 19, BJNhem20, SA001, SA001.

In addition, ES cells can be obtained from other species as well, including mouse (Mills and Bradley, 2001), golden hamster [Doetschman et al., 1988, Dev Biol. 127: 224-7], rat [Iannaccone et al., 1994, Dev Biol. 163: 288-92] rabbit [Giles et al. 1993, Mol Reprod Dev. 36: 130-8; Graves & Moreadith, 1993, Mol Reprod Dev. 1993, 36: 424-33], several domestic animal species [Notarianni et al., 1991, J Reprod Fertil Suppl. 43: 255-60; Wheeler 1994, Reprod Fertil Dev. 6: 563-8; Mitalipova et al., 2001, Cloning. 3: 59-67] and non-human primate species (Rhesus monkey and marmoset) [Thomson et al., 1995, Proc Natl Acad Sci U S A. 92: 7844-8; Thomson et al., 1996, Biol Reprod. 55: 254-9].

Extended non-human blastocyst cells (EBCs) can be obtained from a non-human blastocyst of at least nine days post fertilization at a stage prior to gastrulation. Prior to culturing the blastocyst, the zona pellucida is digested [for example by Tyrode's acidic solution (Sigma Aldrich, St Louis, MO, USA)] so as to expose the inner cell mass. The blastocysts are then cultured as whole embryos for at least nine and no more than fourteen days post fertilization (i.e., prior to the gastrulation event) *in vitro* using standard embryonic stem cell culturing methods.

EG cells are prepared from the primordial germ cells obtained from fetuses of about 8-11 weeks of gestation (in the case of a human fetus) using laboratory techniques known to anyone skilled in the arts. The genital ridges are dissociated and cut into small chunks which are thereafter disaggregated into cells by mechanical dissociation. The EG cells are then grown in tissue culture flasks with the appropriate medium. The cells are cultured with daily replacement of medium until a cell morphology consistent with EG cells is observed, typically after 7-30 days or 1-4 passages. For additional details on methods of preparation human EG cells see Shamblott et al., [Proc. Natl. Acad. Sci. USA 95: 13726, 1998] and U.S. Pat. No. 6,090,622.

Induced pluripotent stem cells (iPS) (embryonic-like stem cells) can be generated from somatic cells by genetic manipulation of somatic cells, e.g., by retroviral transduction of somatic cells such as fibroblasts, hepatocytes, gastric epithelial cells with transcription factors such as Oct-3/4, Sox2, c-Myc, and KLF4 [Yamanaka S, Cell Stem Cell. 2007, 1(1):39-49; Aoi T, et al., Generation of Pluripotent Stem Cells from Adult Mouse Liver and Stomach Cells. Science. 2008 Feb 14. (Epub ahead of print); IH Park, Zhao R, West JA, et al. Reprogramming of human somatic cells to pluripotency with defined factors. Nature 2008;451:141-146; K Takahashi, Tanabe K, Ohnuki M, et al. Induction of pluripotent stem cells from adult human fibroblasts by defined factors. Cell 2007;131:861-872]. Other embryonic-like stem cells can be generated by non-human nuclear transfer to oocytes, fusion with embryonic stem cells or non-human nuclear transfer into zygotes if the recipient cells are arrested in mitosis.

Culturing the cells in the media described herein may be effected in any vesicle, e.g. plate, chamber, bioreactor etc.

The number of cells that may be selected and/or cultured according to the present disclosure may be any number including small batches - e.g. $100 \times 10^4$ cells to larger batches - e.g. $100 \times 10^6$ or $100 \times 10^7$ cells.

The cells may be cultured in a bioreactor (or in multi-level industrial flasks), the size of which is selected according to the number of cells being cultured.

As used herein, the term "bioreactor" refers to any device in which biological and/or biochemical processes develop under monitored and controlled environmental and operating conditions, for example, pH, temperature, pressure, nutrient supply and waste removal. According to one aspect of the disclosure, the basic classes of bioreactors suitable for use with the present disclosure include static bioreactors, stirred flask bioreactors, rotating wall bioreactors, hollow fiber bioreactors and direct perfusion bioreactors.

According to a particular aspect, the cells are cultured (i.e. expanded) on an adherent surface.

Examples of such surfaces are provided herein under.

1. Laminin/Fibronectin coated plates. Sources for Fibronectin: (Sigma Aldrich Bovine Fibronectin F1141, or human Fibronectin Millipore FC010). Sources for Laminin (Sigma Aldrich Ewing Sarcoma derived Laminin L2020).
2. Cells can be expanded on gelatin and vitronectin coated plates (e.g. 0.2% gelatin and 1 μg/ml Vitronectin coated plates).
3. Cells can be expanded on plates coated with 0.2% gelatin/irradiated mouse or human fibroblast feeder cells.
4. Human naive cells can be expanded on plates coated with only 0.2% gelatin coated plates.
5. Human naive cells can be expanded on plates coated with only Matrigel or Geltrex (BD Biosciences).
6. Human naïve and primed cells can be expanded in suspension in plates, flasks or plastic bags with rocking or rotation movements.

The culture media described in the present application may be used for a myriad of purposes.

According to a particular aspect, the culture media are used for expanding (i.e. increasing the number of) cells - e,g, expanding PSCs. The present inventors have noted that expansion of pluripotent stem cells in the presently disclosed media maintains the pluripotent state of the cells and further ensures that less than 80 % of the Cs of a CG sequence in the DNA are not methylated. In some aspects less than 70 % of the Cs of a CG sequence in the DNA are not methylated.

It should be noted that culturing PSC involves replacing the culture medium with a "fresh" medium (of identical composition) every 24-48 hours, and passaging each culture dish (e.g., a plate) to 2 or 3 culture dishes (e.g., plates) every 3-5 days. Thus, when cells in the culture reach about 60-90% confluence the supernatant is discarded, the culture dishes are washed [e.g., with phosphate buffered saline (PBS)] and the cells are subjected to enzymatic dissociation from the culture dish, e.g., using trypsinization (0.25% or 0.05% Try sin + EDTA), e.g., until single cells or cell clumps are separated from each other.

The culture media described herein can be used in the generation of iPSCs from somatic cells. Methods of generating iPSCs are known in the art and include for example genetically modifying the somatic cells to express at least one dedifferentiating factor selected from the group consisting of KLF4, c-MYC, OCT4, SOX2, Nanog, and LIN28. Alternatively, the somatic cells can be provided directly with the RNA that encodes the transcription factors.

According to a particular aspect, the generation of iPSCs comprises expressing in the somatic cells at least two dedifferentiating factors - the first factor selected from the group consisting of Nanog, ESRRB, KLF2, KLF17, TBX3, TFAP2C, ERAS and the second factor selected from the group consisting of Nanog, ESRRB, KLF2, KLF17, TFAP2C, TBX3, ERAS, Oct4, Sox2, Klf4c-Myc.

Methods of DNA transfections into mammalian cells are known in the art and include those described in Reference (Mansour et al. 2012). Further description of preparation of expression vectors and modes of administering them into cells are provided hereinunder.

According to some aspects of the disclosure, expressing the factors is performed using RNA transfection of the growth factors.

Methods of RNA transfections into mammalian cells are known in the art and include those described for example in (Warren et al. 2010) .

Examples of somatic cell types retinal pigment epithelial cells, cardiomyocytes, epithelial cells such as keratin-containing cells, hepatocytes, pancreatic cells (e.g. pancreatic beta cells), muscle cells, blood cells, fat cells, bone cells, chondrocytes, neurons, astrocytes and oligodendrocytes.

The culture media described herein can be used in the generation of naive pluripotent stem cells from non-naïve pluripotent stem cells. Preferably the media used for generation or maintenance of naive pluripotent stem cells comprises: LIF, WNT inhibitor, Notch inhibitor, P38 inhibitor, PKC inhibitor SRC inhibitor, Activin A and Rock inhibitor.

Thus, according to another aspect, the culture media described herein are used to generate naive pluripotent stem cells from non-naïve cells.

More specifically, according to another aspect of the present disclosure there is provided a method of generating a naive pluripotent stem cell (PSC), comprising:

incubating a non-naive PSC cell in the culture medium described herein, the culture medium allowing generation of the naive PSC from the non-naive PSC, wherein:

(i) when the naive PSC is a female PSC, then the naive female PSC has two unmethylated alleles of an X-inactive specific transcript (XIST) gene; and
(ii) when the naive PSC is a male PSC, then the naive male PSC has an unmethylated allele of the XIST gene; and/or

an expression level of transcription factor E3 (TFE3) in the naive PSC is characterized by a nucleus to cytoplasm expression ratio which is equal to or higher than 1 as determined by an immunostaining assay, thereby generating the naive PSC.

It is expected that during the life of a patent maturing from this application many relevant WNT inhibitors, SRC inhibitors and protein kinase C (PKC) inhibitors will be developed and the scope of these terms is intended to include all such new technologies *a priori*.

As used herein the term "about" refers to ± 10 %
The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various aspects of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the disclosure, which are, for clarity, described in the context of separate aspects, may also be provided in combination in a single aspect. Conversely, various features of the disclosure, which are, for brevity, described in the context of a single aspect, may also be provided separately or in any suitable subcombination or as suitable in any other described aspect of the disclosure. Certain features described in the context of various aspects are not to be considered essential features of those aspects, unless the aspect is inoperative without those elements.

Various aspects and aspects of the present disclosure as delineated hereinabove and as claimed in the claims section below find experimental support in the following examples.

## REFERENCE EXAMPLES

Reference is now made to the following examples, which together with the above descriptions illustrate some aspects of the disclosure in a non limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

## MATERIALS AND METHODS

***PolyA-RNA-seq library preparation:*** Total RNA was isolated from indicated cell lines and extracted from Trizol pellets by chloroform-phenol extraction protocol, then utilized for RNA-Seq by ScriptSeq Preparation Kit v2 (Illumina) according to manufacturer's instruction.

***ATAC-seq library preparation:*** Cells were trypsinized and counted, 50,000 cells were centrifuged at 500g for 3 min, followed by a wash using 50 μl of cold PBS and centrifugation at 500g for 3 min. Cells were lysed using cold lysis buffer (10 mM Tris-HCl, pH 7.4, 10 mM NaCl, 3 mM $MgCl_2$ and 0.1% IGEPAL CA-630). Immediately after lysis, nuclei were spun at 500g for 10 min using a refrigerated centrifuge. Next, the pellet was resuspended in the transposase reaction mix (25 μl 2× TD buffer, 2.5 μl transposase (Illumina) and 22.5 μl nuclease-free water). The transposition reaction was carried out for 30 min at 37 °C and immediately put on ice. Directly afterwards, the sample was purified using a Qiagen MinElute kit. Following purification, the library fragments were amplified using custom Nextera PCR primers 1 and 2 for a total of 12 cycles. Following PCR amplification, the libraries were purified using a QiagenMinElute Kit and sequenced.

**Whole-Genome Bisulfite Sequencing (WGBS) Library preparation:** DNA was isolated from cells using the Quick-gDNA miniprep kit (Zymo). DNA (50ng) was then converted by bisulfite using the EZ DNA Methylation-Gold kit (Zymo). Libraries were prepared using the TruSeq kit (Illumina) and length distribution of each library was measured using the Bioanalyzer and product concentration was measured using Qubit Fluorometric Quantitation. For sequencing, the libraries, NextSeq 500/550 High Output v2 kit (150 cycles) was used.

**ChIP-seq library preparation:** Cells were crosslinked in formaldehyde (1% final concentration, 10 min at room temperature), and then quenched with glycine (5 min at room temperature). Antibodies detailed in Table 1 were then lysed in 50 mM HEPES KOH pH 7.5, 140 mM NaCl, 1 mM EDTA, 10% glycerol, 0.5% NP-40 alternative, 0.25% Triton supplemented with protease inhibitor at 4 °C (Roche, 04693159001) for 10 min, and later centrifuged at 950g for 10 min.

***Table 1***

| | antibody name | amount antibody | amount of cells | Incubation time |
|---|---|---|---|---|
| KLF17 | HPA024629 | 6ug | 30 million | Over Night |
| KLF4 | AF3158 | 10ug | 30 million | Over Night |
| NANOG | AF1997 | 6ug | 30 million | Over Night |
| OCT4 | SC8628 | 10ug | 30 million | Over Night |
| TFAP2c | sc-8977 | 5ug | 30 million | Over Night |
| SOX2 | AF2018 | 10ug | 30 million | Over Night |
| H3K27ac | ab4729 | 5ug | 5 million | 6 hours |

Supernatant was discarded and pellet was resuspended in RIPA-1 (0.2% SDS, 1 mM EDTA, 0.1% DOC, 140 mM NaCl and 10 mM Tris-HCl) with protease inhibitor. Cells were then fragmented with a Branson Sonifier (model S-450D) at - 4 °C to size ranges between 200 and 800 bp and centrifugation at max speed for 10 min. Supp lysate was extracted and diluted with RIPA 2-3-fold (0.1% SDS, 1 mM EDTA, 0.1% DOC, Triton 1%, 140 mM NaCl and 10 mM Tris-HCl). Small amount of lysate were saved for whole cell extract at this point. Antibody was pre-bound by incubating with Protein-G Dynabeads (Invitrogen 10004D) in blocking buffer (PBS supplemented with 0.5% TWEEN and 0.5% BSA) for 1 h at room temperature. Washed beads were added to the lysate for incubation. Samples were washed five times with RIPA buffer, twice with RIPA buffer supplemented with 500 mM NaCl, twice with LiCl buffer (10 mM TE, 250mM LiCl, 0.5% NP-40, 0.5% DOC), once with TE (10Mm Tris-HCl pH 8.0, 1mM EDTA), and then eluted in 0.5% SDS, 300 mM NaCl, 5 mM EDTA, 10 mM Tris HCl pH 8.0. Eluate was incubated treated sequentially with RNaseA (Roche, 11119915001) for 30 min in 37°C and proteinase K (NEB, P8102S) for 2 h in 37°C and de-crosslinked in 65 °C for 8 h. DNA was purified with The Agencourt AMPure XP system (Beckman Coulter Genomics, A63881). Libraries of cross-reversed ChIP DNA samples were prepared according to a modified version of the Illumina Genomic DNA protocol, as described previously (Rais et al., 2013).

**PolyA-RNA analysis:** hESCs grown in naive and primed conditions, from different cell lines (LIS41, LIS49, WIBR2) were used for RNA-seq analysis. STAR software version 2.5.2b was used to align reads to human GRCh38 reference genome (2013), using the following flags: - -outFilterMultimapNmax 1 --outReadsUnmapped Fastx --twopass-Mode Basic-outSAMstrandField intronMotif. FPKM values were estimated with HTSeq software over all genes in GRCh38 assembly using the following flags: -a 10 -s no -t exon -i gene_id. Genes with accumulated expression of FPKM>10 over all samples, were selected for analysis. The filtering was done independently in each analysis, therefore the number of genes included may change, as it is dependent on the samples that were included for that analysis.

FPKM values were further normalized using R DESeq software, and corrected for batch effects using R limma package. Hierarchical clustering was carried out using R pheatmap command. PCA analysis was carried out using R prcomp command.

Differentially expressed genes between naive and primed samples were selected from HTSeq output in the following parameters: FC>2 of FC<0.5, and adjusted p-value<0.1.

***Whole-Genome Bisulfite Sequencing (WGBS) analysis:*** The sequencing reads were aligned to the human hg19 reference genome (UCSC, 2009), using a proprietary script based on Bowtie2. In cases where the two reads were not aligned in a concordant manner, the reads were discarded. Methylation levels of CpGs calculated by WGBS were unified. Mean methylation was calculated for each CpG that was covered by at least 5 distinct reads (X5). Average methylation level was calculating by taking the average over all covered X5 covered CpG sites in that genome.

***ChIP-seq analysis:*** Chip-seq data of the following DNA-binding proteins was analyzed: NANOG, SOX2, OCT4, KLF4, KLF17, TFAP2C, H3K27AC. For alignment and peak detection, bowtie2 software was used to align reads to human hg19 reference genome (UCSC, 2009), with default parameters. Enriched intervals of all measured proteins were analyzed using MACS version 1.4.2-1. Sequencing of whole-cell extract was used as control to define a background model. Duplicate reads aligned to the exact same location were excluded by MACS default configuration. Peaks were assigned to genes using Homer software.

**ATAC-seq analysis:** Reads were aligned to hg19 human genome using Bowtie2 with the parameter -X2000 (allowing fragments up to 2 kb to align). Duplicated aligned reads were removed using Picard MarkDuplicates tool with the command REMOVE_DUPLICATES=true. To identify chromatin accessibility signal we considered only short reads (≤ 120bp) that correspond to nucleosome free region. To detect and separate accessible loci in each sample, we used MACS version 1.4.2-1 with --call-subpeaks flag (PeakSplitter version 1.0).

***Enhancer Identification:*** H3K27ac peaks were detected using MACS version 1.4.2-1 and merged for each condition (naive and primed) using bedtools merge command. All ATAC peaks were filtered to include only peaks which co-localized with the merged H3K27ac peaks in at least one condition. Finally, peaks that co-localized with promoter or exon regions based on hg19 assembly (UCSC, 2009) were filtered out. Finally, the data was confined to defined genomic intervals which was annotated as enhancers.

**Motif analysis:** Enriched binding motifs were searched in chromatin accessible loci using findMotifsGenome function from homer software package version 4.7, using the software default parameters.

***Culture medium:***
***Enhanced NHSM composition***
WIS-NHSM media (B27, vitamin C and N2 based) (No Activin/TGFIFGF)
**Primary Cytokines + inhibitors:**

- 1:1 mix of Neurobasal (Invitrogen 21103-049) and DMEM/F12 (Invitrogen 21331) - 470ml
- Pen-strep 5 ml (Biological Industries 03-033-1B)
- Sodium Pyruvate 5 ml (Biological Industries 03-042-01B, 100mM stock solution)
- Glutamax - 5 ml (Invitrogen 35050061)
- NEAA- 5 ml (Biological Industries 01-340-1B)
- 10 ml B27 supplement: Invitrogen 17504-044 or Xenofree A1486701 or in-house made
- N2 comp.% - Insulin (Sigma I-1882) - 5mg insulin per bottle (10microg/ml final concentration)
- N2 comp.% -Apo-transferrin (Sigma T-1147), 50 μg/ml final concentration
- N2 comp.% -Progesterone (Sigma P8783), 0.02 μg/ml final concentration;
- N2 comp.% -Putrescine (Sigma P5780), 16 μg/ml final concentration
- N2 comp.% - Sodium selenite (Sigma S5261), add 5 μL of 3 mM stock solution per 500ml.
- L-ascorbic acid 2-phosphate (Sigma - A8960) (50 μg/ml final concentration) (1 vial)
- Geltrex (Invitrogen A1413202/A1413302) - add 1 ml rapidly in media (0.2% final conc.)
- Alpha-KG (Dimet2-oxoglutarate; Sigma 349631; add 60 μL) - 0.8 mM final

**Primary Cytokines + inhibitors:**

- 1) LIF (in house produced or *Peprotech 300-05*) - 20ng/ml final (1 vial=50μL)
- 2) WNTi: *TNKi = XAV939 (Axon 1527)* - 3μM final (0.75 vial = 75μL)
- 3) MEKi/ERKi PD0325901 *(Axon 1408)* - 1μM final (1 vial=50μL)
- 4) P38i/JNKi BIRB0796 *(Axon 1358)* - 0.9μM final (0.45 vial = 22.5μL)
- 5) PKCi Go6983 *(Axon 2466)* - 2μM final (1 vial=50μL)
- 6) SRCi CGP77675 *(Axon 2097)* - 1.2μM (1.2 vial=60μL)

- 7) ROCKi Y27632 *(Axon 1683)* - 1.2μM (60μL include upon assembling media)

***Enhanced NHSM composition with low or no ERKi (tENHSM or OENHSM)***

WIS-NHSM media (B27, vitamin C and N2 based) (No Activin/TGFIFGF)

**Primary Cytokines + inhibitors:**

- 1:1 mix of Neurobasal (Invitrogen 21103-049) and DMEM/F12 (Invitrogen 21331) - 470ml
- Pen-strep 5 ml (Biological Industries 03-033-1B)
- Sodium Pyruvate 5 ml (Biological Industries 03-042-01B, 100mM stock solution)
- Glutamax - 5 ml (Invitrogen 35050061)
- NEAA- 5 ml (Biological Industries 01-340-1B)
- 10 ml B27 supplement: Invitrogen 17504-044 or Xenofree A1486701 or in-house made
- N2 comp.% - Insulin (Sigma I-1882) - 5mg insulin per bottle (10microg/ml final concentration)
- N2 comp.% -Apo-transferrin (Sigma T-1147), 50 μg/ml final concentration
- N2 comp.% -Progesterone (Sigma P8783), 0.02 μg/ml final concentration;
- N2 comp.% -Putrescine (Sigma P5780), 16 μg/ml final concentration
- N2 comp.% - Sodium selenite (Sigma S5261), add 5 μL of 3 mM stock solution per 500ml.
- L-ascorbic acid 2-phosphate (Sigma - A8960) (50 μg/ml final concentration) (1 vial)
- Geltrex (Invitrogen A1413202/A1413302) - add 1 ml rapidly in media (0.2% final conc.)
- Alpha-KG (Dimet2-oxoglutarate; Sigma 349631; add 60 μL) - 0.8 mM final

**Primary Cytokines + inhibitors:**

- 1) LIF (in house produced or *Peprotech 300-05)* - 20ng/ml final (1 vial=50μL)
- 2) WNTi: *TNKi = XAV939 (Axon 1527)* - 3μM final (0.75 vial = 75μL)
- 3) NOTCHi DBZ *(Axon 1488)* - 1μM final ( 0.25 μM, 7.5μL)
- 4) P38i/JNKi BIRB0796 *(Axon 1358)* - 0.9μM final (0.45 vial = 22.5μL)
- 5) PKCi Go6983 *(Axon 2466)* - 2μM final (1 vial=50μL)
- 6) SRCi CGP77675 *(Axon 2097)* - 1.2μM (1.2 vial=60μL)
- 7) ROCKi Y27632 *(Axon 1683)* - 1.2μM (60μL include upon assembling media)
- MEKi/ERKi PD0325901 *(Axon 1408)* - 0 μM (for OENHSM) or 0.33 μM final (for tENHSM)
- ***Additional media:***

Human PSCs (H9 female 46XX human ESC line) were expanded for passages in N2B27 defined base media supplemented with:

9) Condition 1- PKCi (Go6983 2μM), TNKi/WNTi (XAV939 2μM) and RBPJi/NOTCHi (RIN1 0.6 μM)
10) Condition 2- PKCi (Go6983 2μM), TNKi/WNTi (XAV939 2μM), RBPJi/NOTCHi (RIN1 0.6 μM) and SRCi (CGP77675 1μM)
11) Condition 3- PKCi (Go6983 2μM), TNKi/WNTi (XAV939 2μM), RBPJi/NOTCHi (RIN1 0.6 μM) and MEK/ERKi (PD0325901 1μM)
12) Condition 4- PKCi (Go6983 2μM), TNKi/WNTi (XAV939 2μM), RBPJi/NOTCHi (RIN1 0.6 μM), SRCi (CGP77675 1μM), MEK/ERKi (PD0325901 1μM)
13) Condition 5- PKCi (Go6983 2μM), TNKi/WNTi (XAV939 2μM) and RBPJi/NOTCHi (RIN1 0.6 μM) + LIF (20ng/ml)
14) Condition 6- PKCi (Go6983 2μM), TNKi/WNTi (XAV939 2μM), RBPJi/NOTCHi (RIN1 0.6 μM) and SRCi (CGP77675 1μM) + LIF (20ng/ml)
15) Condition 7- PKCi (Go6983 2μM), TNKi/WNTi (XAV939 2μM), RBPJi/NOTCHi (RIN1 0.6 μM) and MEK/ERKi (PD0325901 1μM) + LIF (20ng/ml)
16) Condition 8- PKCi (Go6983 2μM), TNKi/WNTi (XAV939 2μM), RBPJi/NOTCHi (RIN1 0.6 μM), SRCi (CGP77675 1μM), MEK/ERKi (PD0325901 1μM) + LIF (20ng/ml).

**RESULTS**

In order to identify culture conditions which capture human naive PSC, the present inventors looked for agents which are capable of maintaining stem cells in a pluripotent state in the absence of defined epigenetic repressors. Human knock-in WIBR3 hESC lines with conditional inducible ablation expression of METTL3 were engineered **(Figure 1A).** This was carried out by introducing an exogenous METTL3 transgene under the regulation of Tet-OFF promoter (Liao et al., 2015), followed by CRISPR/Cas9 mediated ablation of both endogenous human METTL3 alleles. Two resultant clones were validated for METTL3 expression only from the exogenous allele, which can be shut off by addition of DOX to the media (called Tet-OFF-METTL3 lines) **(Figure 1B).**

Primed Tet-OFF-METTL3 hESCs expanded in TeSR or KSR/FGF2 primed conditions could not be sustained in the presence of DOX for more than four passages (both on MEF or on Geltrex coated dishes) and resulted in massive cell death and differentiation **(Figure 1C).** In the presence of MEFs, Tet-OFF-METTL3 could be stably maintained in NHSM conditions, but not in 4iLA-MEF, 5iLAF-MEF, 5iLA-MEF, 6iLA-MEF, TESR/3iL-MEF conditions **(Figure 2A).** The latter further support rewiring toward naïve pluripotency in NHSM conditions. However, in the absence of MEFs, NHSM conditions could not support maintenance of pluripotency when METTL3 was ablated, suggesting that NHSM conditions can be enhanced to endow the cells with such ability **(Figure 1D).** Candidate molecules were tested for the ability to enrich NHSM conditions that allow Tet-OFF-METTL3 to be maintained on Geltrex coated plates in the presence of DOX.

NHSM conditions were supplemented with individual small molecules **(Figure 1D).** Remarkably, supplementing NHSM conditions with the Tankyrase inhibitor named IWR1, but not any of the other 15 compounds tested, enabled the expansion of Tet-OFF-METTL3 on DOX with great homogeneity **(Figure 1E).** IWR1 is a WNT inhibitor (WNTi) small molecule that stabilizes AXIN protein in the cytoplasm by inhibiting Tankyrase enzyme (abbreviated herein as TNK inhibitor - TNKi). An additional TNKi, XAV939, yielded a similar effect, while using exo-IWR1 an inactive modified version of IWR1 failed to do so, supporting specific inhibition of Tankyrase as the target yielding stability of these pluripotent cells **(Figure 1F).**

Two additional cell lines based on WIBR3 hESC line carrying knock in ΔPE-OCT4-GFP reporter (Theunissen et al., 2014a) were used in parallel to optimize and enhance NHSM conditions **(FIG. 2B).** Supplementation of TNKi to NHSM conditions yielded a dramatic increase in GFP signal when compared to primed, NHSM or 4i-LA conditions **(Figure 1G, Figure 2B).** Consistent with studies conducted in mice (Kim et al., 2013), including TNKi rendered exogenous supplementation of FGF2 dispensable even in feeder free conditions **(FIG. 1G).** Further, as XAV939 inhibits WNT signaling, the present inventors validated that including GSK3 inhibitor is dispensable and, in fact, compromises the intensity of ΔPE-OCT4-GFP signal **(Figure 2C).** Pluripotent cells could be maintained in the absence of JNK and P38 inhibitors, they boosted naive pluripotency marker expression and therefore were maintained in the media used herein **(Figure 2E).** Importantly, after optimizing NHSM conditions, the present inventors attempted to substitute TNKi with other components included in the screen, to exclude the possibility that the latter optimizations may facilitate a different screening result. However, none of them allowed expanding METTL3 depleted cells *in vitro* as seen with supplementing TNKi (including VPA, BRAFi, Forskolin, Kenpaullone, SHHi, DOT1Li, LSD1i, TGFRi, ERK5i) **(Figure 2F).**

**Defining human naive pluripotency conditions independent of TGF/ACTIVIN/NODAL signaling**

Under the above described conditions, human ESCs maintained uniformly high ΔPE-OCT4-GFP levels only in the presence of exogenous ACTIVIN A, and consistently differentiated when TGFR inhibitor was provided **(Figures 1H-I**, **2G**). Thus, the present inventors for the identification of a small molecule whose supplementation will render human PSCs that are independent of exogenous ACTIVIN/TGF supplementation. It should be noted that none of the previously described human naive conditions have been able to maintain teratoma competent pluripotent cells that can be maintained long term and validated for their naive identity after prolonged specific inhibition of ACTIVIN/NODAL signaling. To do this, the latter TNKi supplemented and modified conditions were used in the absence of ACTIVIN A, and candidate molecules were added to allow expanding OCT4+ PSCS independent of METTL3 expression (on DOX) **(Figure 3A).** While under most conditions, Oct4+ cell fraction rapidly deteriorated, it was noted that a validated SRCi (CGP77675) dramatically maintained the stability of dome like cells that were uniformly OCT4+ **(Figure 3B).** This led to the assembly of a defined FGF/TGF/ACTIVINV/MEF free and independent growth conditions which is referred to herein as Enhanced NHSM - "ENHSM" **(Figures 3C-D**). Of note, supplementation of SRCi in ACTIVIN A containing conditions, although not essential to maintain ΔPE-OCT4-GFP+ when ACTIVIN was provided, it did support consistency and domed like morphology among naive cells **(Figure 2D)** (conditions referred to as ENHSM-ACT).

Following METTL3 depletion in ENHSM conditions, WIBR3 cells maintained their typical domed lie morphology and uniformly expressed pluripotency markers including KLF17 that is specific to the naive state both with and without METTL3 depletion **(Figure 3E).** Measurement of $m^6A$ on mRNA showed over 90% depletion of total levels after DOX addition **(Figure 3F),** comparable to those seen upon knockout of the Mettl3/14 complex in mouse naive ESCs. ESCs maintained in the absence of METTL3 for over 30 passages remained pluripotent and were capable of generating mature teratomas *in vivo* without the need to passage them first for a period of time under primed conditions **(Figure 3G).** The latter validates maintenance of naive pluripotency in human PSCs expanded in ENHSM without METTL3 protein and ablated $m^6A$ levels deposited on mRNA in the cells.

To extend the previous findings to another repressor machinery, OCT4-GFP-WIBR3 reporter ESCs were targeted by TALENs to generate DGCR8 null cells **(Figure 3H).** While conducting such targeting on primed cells did not yield any null cells **(Figure 2H),** DGCR8 null cells could be obtained when the targeted cells were expanded in ENHSM and ENHSM-ACT conditions **(Figure 3H-I**, **Figure 2I).** To test which of the naive conditions enable expanding human naive PSCs in the absence of DNMT1 cells, a similar approach to that applied for making Tet-OFF-METTL3 herein, has been recently used to generate TET-OFF DNMT1 in HUES64 ESC line **(Figures 4A-B**). Cells expanded in previously described naïve conditions including NHSM-MEF, 4i/LA-MEF, 5i/LA-MEF, 6i/LA-MEF, 5i/LAF-MEF, TESR/3iL-MEF and NHSM could not be maintained in the presence of DOX for more than 3 passages **(Figures 4C-D**). ENHSM and ENHSM-ACT conditions allowed stable and unlimited expansion of DNMT1 depleted human naive ESCs both in feeder and feeder free conditions **(Figures 4C-D**). Whole Genome Bisulfite Sequencing (WGBS) confirmed global loss of methylation in naive DNMT1 depleted cells expanded in ENHSM conditions and analyzed at passage 14 **(Figure 4E)** and maintained expression of pluripotency markers **(Figure 5A).** Collectively these results demonstrate that ENHSM conditions mimic mouse naive ESC and for the first time enable generation of human PSCs ablated for epigenetic repressors (both in feeder and feeder free conditions) and that are independent from ACTIVIN/TGF signaling.

**Tolerance for absence of exogenous L-Glutamine in ENHSM conditions**

Murine naive ESCs retain bivalent metabolic capability utilizing both Oxidative phosphorylation (OXPHOS) and Glycolytic metabolism, while upon priming then become dependent only on glycolytic metabolism. As shown previously, NHSM, 5i-LA and transgene containing reset cells increase OXPHOS activity leading to retaining bivalent metabolic profile. ENHSM condition were similarly tested herein and by measuring basal oxygen consumption rate (OCR) it was substantially higher in ENHSM conditions than in conventional PSC **(Figure 4F).** Higher electron transport chain activity in ENHSM was evidenced by a greater OCR increase in response to the mitochondrial uncoupler FCCP **(Figure 4F).** Cells expanded in ENHSM condition displayed more intense staining with tetramethylrhodamine methyl ester (TMRE) **(Figure 5B),** indicative of mitochondrial membrane depolarization. The latter supports the conclusion that this is a naive feature is relatively less stringent and can be obtained in a variety of human naive protocols devised so far.

However, a newly identified stringent metabolic feature recently identified in naive ESCs in 2i or 2i/LIF is that they can endogenously synthesize glutamine at sufficient levels to maintain adequate alpha-ketoglutarate ($\alpha$KG) levels. While they benefit form exogenous L-Glutamine supplementation, it is not essential for their stability or pluripotency as they can metabolically synthesize it internally as part of their altered metabolic configuration. FBS/LIF naive murine ESCs or primed EpiSCs cannot be maintained in the absence of exogenous L-Glutamine. To compare the latter observation and apply them on distinct human pluripotent states, WIBR3-OCT4-GFP knock-in ESC line, $\Delta$PE-WIBR3-OCT4-GFP knock-in ESC line, H9-NANOG-GFP ESC lines were then tested for their ability to maintain pluripotency in the presence and absence of L-Glutamine **(Figure 4G).** Importantly, the present inventors failed to maintain primed PSCs or other naive PSCs in the absence of L-Glutamine (in NHSM, 4i/LA-MEF, Si/LAF-MEF, 6iLA-MEF, TESR/3iL-MEF) even when MEFs were used **(Figure 4G).** However, $\Delta$PE-WIBR3-OCT4-GFP expanded in ENHSM was not compromised when L-glutamine was not included in ENHSM conditions **(Figure 5C)** and GFP signal was positive for H9-NANOG-GFP ESC both in the presence and absence of L-Glutamine (both on feeder and feeder free conditions) **(Figure 5C).** Cells expressed general and naive specific pluripotency markers in ENHSM with and without exogenous L-Glutamine and generated differentiated teratomas without the need for passaging in vitro in primed conditions **(Figures 5D-E**). Collectively, these results validate that ENHSM conditions can maintain naive pluripotency characteristics and endow the cells with ability to be expanded in the absence of exogenous L-Glutamine, as with murine 2i/LIF naïve PSCs.

**Transcriptional characterization of human PSCs in ENHSM conditions**

The present inventors next aimed to convert previously established primed PSCs lines and to derive new lines directly in ENHSM-ACT and ENHSM conditions from the ICM of human blastocysts. Human blastocysts were plated on mouse embryonic fibroblast (MEF) coated plates and medium successfully generated domed cell outgrowths following 6-8 days of plating. ICM derived outgrowths were then trypsinized and passaged. Subsequently, 3 new stem cell lines termed LIS36, LIS42 and LIS46 were derived in ENHSM-ACT; LIS41 and LIS49 ESCs in ENHSM conditions **(Figure 6A).** Multiple conventional (hereafter will be named "primed") hESC lines (WIBR1, WIBR2, WIBR3, HUES64, H9) were plated on Geltrex coated dishes in ENHSM or ENHSM-ACT medium **(Figure 6B).** Within 4-8 days of applying this protocol, dome-shaped colonies with packed round cell morphology, typical of mESCs, could be readily isolated and further expanded **(Figure 6B).** Adult human dermal fibroblast cells or peripheral blood cells were reprogrammed to iPSCs in ENHSM conditions following either lentiviral transduction with DOX inducible OKSM factors (BF1 hiPSC) or by non-integrating sendai viruses (JH1, BC1 and MECP5 hiPSC) **(Figure 6C).** All polyclonal and subcloned hESC and iPSC lines expanded in ENHSM conditions were uniformly positive for pluripotent markers AP, OCT4, NANOG, SSEA4, TRA1-60 and TRA1-81 (representative images in **Figure 7)** and robustly formed mature teratomas *in vivo* without the need for short- or long-term exposure to primed growth conditions, and as typically observed with rodent ground state naive PSCs **(Figure 8).** Naive lines were passaged with TryplE every 3-5 days and had single cell cloning efficient up to 40-60%, while primed cell single cell cloning increased only up to 10-20% when Y27632 was used. Human naive pluripotent lines maintained normal karyotype after extended passaging in ENHSM-ACT or ENHSM in most lines of tested **(Figure 10).** In some cultures, minor aneuploidy cells were observed; however no recurrent abnormality was observed between any of these lines as determined by G-banding of metaphase chromosomes **(Figure 10).** The results indicate that epigenetic resetting in ENHSM does not cause obligatory chromosomal abnormalities nor select for pre-existing variants, as had been observed for other naive conditions like 5iLA-MEF conditions (96-100% chromosomal abnormality by passage 10 only) (Liu et al., 2017).

Global gene expression patterns were compared between naive and primed hESCs and hiPSCs, many of which were genetically matched. Unbiased clustering of genome-wide expression profiles demonstrated that naive hESC and hiPSCs possess a distinct gene expression pattern and clustered separately from conventional/primed hESCs and hiPSCs **(Figures 9A-B**). Transcripts associated with naive pluripotency were significantly upregulated in naive cells. The later included NANOG, TFCP2L1, KLF17, KLF4, STELLA (DPPA3), DPPA5 **(Figure 11A).** RT-PCR analysis validated the dramatic upregulation in naive PSCs expanded both in ENHSM and ENHSM-ACT conditions **(Figure 9C).** When including naive datasets generated in 5iLA-MEF, 4i-LAF and t2i-LIF-GO-NK2, it was noted that cells generated in ENHSM and ENHSM-ACT conditions clustered with all the latter naïve conditions and not with primed samples **(Figures 9A-B**, **Figures 11B-C**). FACS analysis confirmed upregulation of previously identified human naïve pluripotency markers CD77 and CD130 in ENHSM conditions, and primed pluripotency marker CD24 was downregulated in ENHSM conditions (Figure 11D) (Collier et al., 2017; Shakiba et al., 2015). Importantly, naive pluripotent cells had profoundly down regulated transcripts associated with lineage commitment genes including T, ZIC2 and VIM1 that are expressed at low, but appreciable, levels in primed hESCs **(Figure 11A**, **12A-B**). STELLA-CFP knock-in allele was introduced via CRISPR/Cas9 **(Figures 13A-B**), to monitor pluripotency maintenance in the different tested conditions, and STELLA-CFP was induced in both ENHSM and previously described 5iLA conditions **(Figure 13C).** Similar to its upregulation and importance in maintaining human naive pluripotency in 5iLA conditions (Pastor et al., 2018), TFAP2C KO lines showed that it is essential for deriving and maintain human naïve PSCs in both ENHSM and ENHSM ACT conditions **(Figures 13D-E**, **14A**). The latter results confirm that ENHSM conditions attain consensus transcriptional feature observed in other previously published naive hPSCs studies or in vivo human embryo data.

Transposable Element (TE)-derived transcripts were profiled and compared in conventional and naive human PSCS expanded in ENHSM conditions (Theunissen et al., 2016). The top 5,000 TEs with largest SD separated naive and primed samples both in hierarchical clustering **(Figure 15A)** and in PCA based analysis **(Figure 15B).** Members of the SINE-VTR-Alu (SVA) family of TEs and HERVK- associated LTR were transcribed almost exclusively in ENHSM conditions similar to previously obtained in 5i/LA and transgene dependent t2iLGo conditions **(Figure 16)** (Theunissen et al., 2016). TE profiling was used to measure the degree to which ENHSM and primed conditions resemble pluripotent cells in early human embryos in vivo. Naive, but not primed cells, demonstrated the most significant overlap with the human morula and epiblast stages when looking TEs **(Figure 9D),** as was similarly shows for coding genes. These results support the endowment of late pre-implantation like transposon expression profile in PSCs expanded in ENHSM conditions in vitro.

**Epigenetic characterization of human PSCs in ENHSM conditions**

ENHSM conditions were tested to see whether they endow human naive PSCs with a pre-X chromosome configuration. Primed human WIBR2 hESC carrying knock-in MECP2-dTomato and MECP2-mCherry alleles were used (Theunissen et al., 2016). Correctly targeted clone #9 expresses only the red allele, however upon transferring the cells into ENHSM conditions >99% of cells expressed bother fluorescent markers consistent with reactivation of both x chromosome alleles. Transferring the cells into primed media allowed inactivation of X chromosome in a non-random manner as evident by obtaining GFP-/tdTomato+ pattern >95% of the reprised cells **(Figure 17A).** FISH analysis was carried out for ATRX in Female cells as this locus is expressed from one copy even in human primed WIBR3 hESCs that have undergone erosion of X chromosome (Xe). Indeed, two ATRX foci could be uniformly found in naive, but not primed, human female PSCs supporting reactivation of X chromosome **(Figure 17B).** Primed human iPSCs were obtained from donor fibroblast carrying a null mutation in only one of the MECP2 allele, and this primed clone was validated to inactivate the wild-type MECP2 allele and thus lacks MECP2 protein expression (Sahakyan et al., 2016). Expanding the cells in ENHSM or ENHSM-ACT for 3 passages was sufficient to yield >99% of iPSC cultures as positive for MECP2+. Subsequently naive cells were re-primed for 4 passages and stained negative for Mecp2 upon repriming, thus indicating ability to inactive X chromosome upon reprogramming. SNP based analysis of X chromosome allele expression as detected in RNA-seq datasets showed biallelic expression of X chromosome encoded genes in ENHSM but not primed conditions, consistent with functional reactivation of X chromosome in female naive PSCS induced in ENHSM conditions. Collectively, the above findings indicate that ENHSM and ENHSM-ACT conditions consolidate human naive pluripotency identity and endows them with nearly all known naive pluripotency features that have been attributed to human ICM *in vivo,* previously derived human naive cells and murine ground state naive cells.

Human naive and primed pluripotent cell's DNA methylation states were sampled by Whole genome Bisulfite Sequencing (WGBS). Lines tested displayed profound downregulation of global methylation levels from 82% in primed hPSCs to 65% in ENHSM expanded human hPSCs and down to 53% when Activin was supplemented (ENHSM-ACT conditions) (Figure 17D). DNMT1 methyltransferase is maintained in ENHSM, while UHRF1 protein is partially (%30-50%) depleted which may underlie the global downregulation in DNA methylation in ENHSM conditions **(Figure 17C).** It is important to note that supplementing ENHSM conditions with BRAF inhibitor used in 5i/LAF conditions leads to dramatic downregulation in both DNMT1 and UHRF1 levels. This pattern is also observed in 5i/LAF condition which might explain the immediate and global loss of imprinting. As DNMT1 levels are maintained in ENHSM conditions and UHRF1 protein downregulation was partial, immediate global loss of imprints in ENHSM or ENHSM-ACT conditions was not observed **(Figure 17B),** but they appeared sporadically and after at least 10 passages in ENHSM conditions.

**WNT/ß-CATENIN and SRC/NFkB signaling are major priming pathways compromising human naive pluripotency**

The results above indicate that functional naive pluripotency in ENHSM composition not only relies on inhibition of ERKi and PKCi, but also on inhibition of TNK and SRC. Depletion of any of these 4 components compromised naïve pluripotency hallmarks like X chromosome inactivation in female cell lines **(Figure 18).** Combined depletion of TNKi and SRCi pushed human PSCs toward complete loss of pluripotency within a number of passaging, underlining the conclusions that ERKi together with tripartite inhibition of PKCi-SRCi-TNKi are essential for functional defined conditions for human naïve pluripotency.

The present inventors next aimed to define the signaling pathway downstream of Tankyrase inhibition facilitating human naive PSCs stabilization. Bcat-KO ESCs had higher levels of Oct4-GFP in ENHSM condition, and upon removal of XAV939 GFP level was not decreased in KO, but in WT ESCs. A similar trend was shown in RT-PCR analysis. Supplementing naive cells with WNT stimulator compromised delta-PE-OCT4-GFP levels, compromised their domed shape like morphology and their transcriptional profile. Using a tamoxifen induced Beta-Catenin-ERT transgene, the present inventors noted that delta-PE-OCT4-GFP signal and domed morphology were compromised upon tamoxifen stimulation. This is in striking contrast to mouse delta-PE-Oct4-GFP ESCs expanded in N2B27 LIF conditions that upon tamoxifen treatment induced deltaPe-Oct4-GFP reporter and nave characteristic domed like morphology. Similarly, while KO of TCF3 boosts mouse naive pluripotency and alleviates the need for WNT stimulation, TCF3 KO ESCs still required WNTi and/or SRCi to maintain their naïve identity in humans. Finally, Supplementing ENHSM conditions with CHIR compromised their ability to maintain pluripotency upon inhibition of TGFB inhibitor, depletion of DNA and RNA methylation or omitting L-Glutamine from the culture conditions. Collectively, these findings clearly establish WNT as a priming agent for human, but not mouse, naive pluripotency and establish that KO of beta-catenin can substitute for Tankyrase inhibition.

SRC inhibition has been shown previously to deplete activation of downstream effectors including ERK, PKC and NFKB signaling. Given that SRCi was needed in ENHSM conditions despite independent direct blocking of ERK and PKC pathways, this led the present inventors to focus on NFKB as a potential effector mediating the beneficial effect of the use of SRCi. Indeed, it was noted that the active subunit of NFKB, P65, was found predominantly in the nucleus of human and mouse primed PSCs, and was excluded to the cytoplasm upon transfer to naïve conditions. Transfection of NFKb signaling luciferase reporter showed high levels of activation in primed but not naive ENHSM conditions. Depletion of SRCi in ENHSM conditions induced nuclear P65 localization and a boost in luciferase reporter signal. Finally, the transfection of dominant negative NFKB subunit in Bcat-KO deltaPE-OCT4-GFP hESCs allowed maintenance of deltaPE-OCT4- GFP not only in ENHSM without TNKi but also without SRCi. These results establish that WNT/BCAT and SRC-NFKB pathways compromises human naive pluripotency.

In mouse ground state naive conditions, LIF/Stat3 has been shown to be a booster for naive marker expression however they can be omitted without entire collapse of the naive PSC circuit (Ying et al., 2008). By omitting LIF from ENHSM conditions and by generating STAT3 KO human naive PSCs, we show that LIF can slightly boost the purity of undifferentiated cells in culture and naïve marker expression by RT-PCR, however it is dispensable and human naive PSCs can maintain their naive identity even in the absence of LIF/STAT3 signaling **(Figure 18)** as has been previously shown for rodent ground state naïve PSCs.

**Inhibition of NOTCH pathway facilitates maintenance of human naive pluripotency without use of MEK/ERK inhibition**

As has been previously shown in mice, the use of ERK inhibition is the major mediator for inducing global hypomethylation which in turns leads to sporadic erosion of imprinting that gets more severe with extended passaging (Choi et al., 2017). In mice, using alternative naive conditions that do not employ ERK inhibitor or titrating ERKi allows isolating murine PSCs with all features of naivety except for global hypomethylation (Choi et al., 2017). The latter murine cells are fully naive and are capable of generate all-iPS mice with contribution to the germline, and thus provide a safer route for exploiting defined mouse naive PSCs (Choi et al., 2017).

Although ENHSM conditions had modest levels of hypomethylation, and erosion of imprinting was slow and sporadic on few loci and only after extended passaging **(Figure 17D),** this may complicate the use of naive cells in future clinical applications if they are expanded in these conditions more than 10 passages. The present inventors thus aimed at defining conditions that allow human naive cell isolation but without global hypomethylation.

Withdrawal of ERK inhibitor form ENHSM conditions compromised the naivety of human ESC as evident be a decrease in deltaPE-Oct4-GFP levels and loss of x-reactivation state in most of the cells within the expanded population **(Figure 19A).** Mere addition of ACTIVIN A upon omission of ERKi form ENHSM conditions did not block loss X reactivation in female cell lines upon depleting ERKi **(Figure 19C).** Thus, the present inventors set out to screen for added compounds that would enable maintenance of pre-x inactivation upon omitting or depleting ERKi **(Figure 19B).** Remarkably, it was noted that addition of gamma secretase inhibitor DBZ, which blocks NOTCH pathway allowed robust and feeder free maintenance of human naive cells when ERKi was omitted (0ENHSM conditions) or titrated down to 0.33microM termed (tENHSM conditions) **(Figure 19C).** The use dominant negative Notch allowed maintenance of naive PSCs without adding DBZ, proving that Notch targeting is the effector mediator of maintaining robust naive pluripotency in human cells when ERK inhibition is depleted.

Human PSCs expanded in ENHSM conditions maintain deltaPE-OCT4-GFP signal equivalent to ENHSM conditions, and maintained pre-X inactivation state in female cell lines **(Figure 19C).** RT-PCR analysis showed that the lines expressed naive pluripotency markers, although levels were less induced to concentration of ERKi used **(Figure 19H).** Global gene expression analysis showed that the cells clustered with ENHSM naive PSCs rather than primed PSCs **(Figures 20A-B**). At the functional levels, the cells were competent in making teratomas without any need for priming in vitro before injections **(Figure 8).** Cells could be maintained upon depletion of DNMT1, METTL3, DGCR8 or exogenous L-glutamine and these qualities depended on the presence of DBZ **(Figures 19E-G**). WGBS analysis showed that these alternative 0ENHSM and tENHSM conditions did not show trends of global hypomethylation that were seen in ENHSM or ENHSM-ACT even after extended passaging and consistently do not show loss of imprinting **(Figure 17D).** These results show that, similar to what was obtained in rodent in vitro PSCs with t2iL and a2iL conditions that endow naïve feature in murine PSCs without compromising global DNA methylation and imprinting regulation (Choi et al., 2017), this can be obtained also in human naive PSCs as well.

**ENHSM-derived hiPSCs give rise to interspecies chimaeras**

Naive hiPSCs can contribute to interspecies chimaerism with highly variable and limited efficiency - Gafni et al. Nature 2013. The present inventors therefore examined whether the refined ENHSM conditions can endow hiPSCs to integrate and contribute to cross-species chimaerism more successfully and with higher propensity.

HiPSCs were labeled with GFP and maintained for at least 3 passages in ENHSM before being micro-injected into E2.5 mouse morulas. Following transplantation into pseudo-pregnant foster mothers the day after, their survival and integration was assayed throughout 14 days using various imaging techniques. ENHSM-derived hiPSCs are able to colonize mouse embryos up to E17.5 at various anatomic regions of different embryonic germ layers as shown in **(Figure 21).** Specific marker staining for human nuclei excluded any contamination **(Figure 21).** The present inventors next investigated whether this integration is based on random differentiation or following the respective tissue identity in-vivo. Immunofluorescence staining of frozen sections confirmed distinct expression of various lung antigens (CC10, Prosurfactant Protein C and Aquaporin 5) substantiating proper lineage commitment and functionality of hiPSC-derived descendants **(Figure 22).**

In order to enhance and boost survival and integration of interspecies chimaera P53 was depleted. AAVS1-GFP labelled hiPSCs were CRISPR/Cas9 targeted for P53 and knock-out were generated extremely efficiently **(Figure 16).** These cells were injected analogously to WT cells and their integration behavior was examined in developing mouse embryos. Strikingly, not only was there an increased chimaeric yield per injection, but additionally a higher GFP+ contribution per embryo as illustrated in **Figures 23-32.** Notably high-degree chimaeras were generated with wide-spread GFP signal throughout the mouse embryo **(Figures 23-32).** Furthermore, live imaging of these embryos revealed a very large contribution especially in the heart and brain region **(Figures 23-32).** In order to obtain an estimate for overall contribution, whole-embryo FACS was performed, showing different degree of chimaeric percentages with up to 50% GFP+ hiPSC-derivatives. Random spontaneous differentiation was ruled out by staining chimaeras for human-specific antigens **(Figures 23-32)** and most importantly for major developmental drivers covering all three embryonic germ layers overlapping extensively with human-derived GFP signal **(Figures 23-32).** Taken together, these results substantiate unequivocal generation of advanced human-mouse interspecies chimaera with various engraftment and functional integration in many defined lineages up to E17.5.

Additional culture conditions were analyzed to determine if additional media are capable of capturing human naive PSCs.

The culture conditions are described in the Materials and methods section and labeled conditions 1-8.

As illustrated in Figure 35, all the media that were tested maintained the pluripotent stem cells in a hypomethylated state.

WIBR3 female human ESCs were expanded for 10 passages in the indicated conditions and immunostained for TFE3 protein expression. Nuclear/cytoplasmic ratio was calculated for each of the conditions. The results are provided in Table 2, herein below.

***Table 2***

| ***Condition*** | ***Nuclear cytoplasmic ratio for TFE3*** |
|---|---|
| Primed (FGF) | 0.5 |
| Naïve condition 1 | 42 |
| Naïve condition 2 | 60 |
| Naïve condition 3 | 53 |
| Naïve condition 4 | 38 |
| Naïve condition 5 | 70 |
| Naïve condition 6 | 64 |
| Naïve condition 7 | 48 |
| Naïve condition 8 | 57 |

REFERENCES

Betschinger, J., Nichols, J., Dietmann, S., Corrin, P.D., Paddison, P.J., and Smith, A. (2013). Exit from Pluripotency Is Gated by Intracellular Redistribution of the bHLH Transcription Factor Tfe3. Cell 153, 335-347.

Brons, I., Clarkson, A., Ahrlund-Richter, L., and Pedersen, R.A. (2007). Derivation of pluripotent epiblast stem cells from mammalian embryos : Article : Nature.

Carey, B.W., Finley, L.W.S., Cross, J.R., Allis, C.D., and Thompson, C.B. (2015). Intracellular $\alpha$-ketoglutarate maintains the pluripotency of embryonic stem cells. Nature 518, 413-416.

Chia, N.-Y., Chan, Y.-S., Feng, B., Lu, X., Orlov, Y.L., Moreau, D., Kumar, P., Yang, L., Jiang, J., Lau, M.-S., et al. (2010). A genome-wide RNAi screen reveals determinants of human embryonic stem cell identity. 468, 316-320.

Choi, J., Huebner, A.J., Clement, K., Walsh, R.M., Savol, A., Lin, K., Gu, H., Di Stefano, B., Brumbaugh, J., Kim, S.Y., et al. (2017). Prolonged Mek1/2 suppression impairs the developmental potential of embryonic stem cells. Nature 548, 219-223.

Collier, A.J., Panula, S.P., Schell, J.P., Chovanec, P., Reyes, A.P., Petropoulos, S., Corcoran, A.E., Walker, R., Douagi, I., Lanner, F., et al. (2017). Comprehensive Cell Surface Protein Profiling Identifies Specific Markers of Human Naive and Primed Pluripotent States. Cell Stem Cell 1-41.

Gafni, O., Weinberger, L., Mansour, A.A., Manor, Y.S., Chomsky, E., Ben-Yosef, D., Kalma, Y., Viukov, S., Maza, I., Zviran, A., et al. (2013). Derivation of novel human ground state naive pluripotent stem cells. Nature 504, 282-286.

Geula, S., Moshitch-Moshkovitz, S., Dominissini, D., Mansour, A.A., Kol, N., Hershkovitz, V., Peer, E., Mor, N., Manor, Y.S., Ben-haim, M.S., et al. (2015a). m6 mRNA methylation facilitates resolution of naive pluripotence toward differentiation. Science (80-. ). 3, 1002-1006.

Geula, S., Moshitch-Moshkovitz, S., Dominissini, D., Mansour, A.A.F., Kol, N., Salmon-Divon, M., Hershkovitz, V., Peer, E., Mor, N., Manor, Y.S., et al. (2015b). m6A mRNA methylation facilitates resolution of naive pluripotency toward differentiation. Science (80-. ). 347, 1002-1006.

Guo, G., von Meyenn, F., Santos, F., Chen, Y., Reik, W., Bertone, P., Smith, A., and Nichols, J. (2016). Naive Pluripotent Stem Cells Derived Directly from Isolated Cells of the Human Inner Cell Mass. Stem Cell Reports 1-19.

Guo, G., von Meyenn, F., Rostovskaya, M., Clarke, J., Dietmann, S., Baker, D., Sahakyan, A., Myers, S., Bertone, P., Reik, W., et al. (2017). Epigenetic resetting of human pluripotency. Development 144, 2748-2763.

Hackett, J.A., and Surani, M.A. (2014). Regulatory principles of pluripotency: from the ground state up. Cell Stem Cell 15, 416-430.

Hanna, J., Markoulaki, S., Mitalipova, M., Cheng, A.W., Cassady, J.P., Staerk, J., Carey, B.W., Lengner, C.J., Foreman, R., Love, J., et al. (2009). Metastable Pluripotent States in NOD-Mouse-Derived ESCs. Cell Stem Cell 4, 513-524.

Hanna, J.H., Saha, K., and Jaenisch, R. (2010). Pluripotency and cellular reprogramming: Facts, hypotheses, unresolved issues. Cell 143, 508-525.
Hayashi, K., Ohta, H., Kurimoto, K., Aramaki, S., and Saitou, M. (2011). Reconstitution of the mouse germ cell specification pathway in culture by pluripotent stem cells. Cell 146, 519-532.
Irie, N., Weinberger, L., Tang, W.W.C., Kobayashi, T., Viukov, S., Manor, Y.S., Dietmann, S., Hanna, J.H., and Surani, M.A. (2015). SOX17 is a critical specifier of human primordial germ cell fate. Cell 160, 253-268.
Kim, H., Wu, J., Ye, S., Tai, C.-I., Zhou, X., Yan, H., Li, P., Pera, M., and Ying, Q.-L. (2013). Modulation of $β$-catenin function maintains mouse epiblast stem cell and human embryonic stem cell self-renewal. Nat. Commun. 4, 2403.
Kojima, Y., Kaufman-Francis, K., Studdert, J.B., Steiner, K.A., Power, M.D., Loebel, D.A.F., Jones, V., Hor, A., de Alencastro, G., Logan, G.J., et al. (2014). The transcriptional and functional properties of mouse epiblast stem cells resemble the anterior primitive streak. Cell Stem Cell 14, 107-120.
Liao, J., Karnik, R., Gu, H., Ziller, M.J., Clement, K., Tsankov, A.M., Akopian, V., Gifford, C.A., Donaghey, J., Galonska, C., et al. (2015). Targeted disruption of DNMT1, DNMT3A and DNMT3B in human embryonic stem cells. Nat. Genet.
Liu, X., Nefzger, C.M., Rossello, F.J., Chen, J., Knaupp, A.S., Firas, J., Ford, E., Pflueger, J., Paynter, J.M., Chy, H.S., et al. (2017). Comprehensive characterization of distinct states of human naive pluripotency generated by reprogramming. Nat. Methods 14, 1-14.
Marks, H., Kalkan, T., Menafra, R., Denissov, S., Jones, K., Hofemeister, H., Nichols, J., Kranz, A., Francis Stewart, A., Smith, A., et al. (2012). The transcriptional and epigenomic foundations of ground state pluripotency. Cell 149, 590-604.
Nichols, J., and Smith, A. (2009). Naive and Primed Pluripotent States. Cell Stem Cell 4, 487-492.
Pastor, W.A., Liu, W., Chen, D., Ho, J., Kim, R., Hunt, T.J., Lukianchikov, A., Liu, X., Polo, J.M., Jacobsen, S.E., et al. (2018). TFAP2C regulates transcription in human naive pluripotency by opening enhancers. Nat. Cell Biol.
Rais, Y., Zviran, A., Geula, S., Gafni, O., Chomsky, E., Viukov, S., Mansour, A.A., Caspi, I., Krupalnik, V., Zerbib, M., et al. (2013). Deterministic direct reprogramming of somatic cells to pluripotency. Nature 502, 65-70.
Sahakyan, A., Kim, R., Chronis, C., Sabri, S., Bonora, G., Theunissen, T.W., Kuoy, E., Langerman, J., Clark, A.T., Jaenisch, R., et al. (2016). Human Naive Pluripotent Stem Cells Model X Chromosome Dampening and X Inactivation. Cell Stem Cell 1-36.
Shakiba, N., White, C.A., Lipsitz, Y.Y., Yachie-Kinoshita, A., Tonge, P.D., Hussein, S.M.I., Puri, M.C., Elbaz, J., Morrissey-Scoot, J., Li, M., et al. (2015). CD24 tracks divergent pluripotent states in mouse and human cells. Nat. Commun. 6.
Takashima, Y., Guo, G., Loos, R., Nichols, J., Ficz, G., Krueger, F., Oxley, D., Santos, F., Clarke, J., Mansfield, W., et al. (2014). Resetting Transcription Factor Control Circuitry toward Ground-State Pluripotency in Human. Cell 158, 1254-1269.
Tesar, P.J., Chenoweth, J.G., Brook, F.A., Davies, T.J., Evans, E.P., Mack, D.L., Gardner, R.L., and McKay, R.D.G. (2007). New cell lines from mouse epiblast share defining features with human embryonic stem cells. 448, 196-199.
Theunissen, T.W. (2016). Molecular Criteria for Defining the Naive Human Pluripotent State. 1-50.
Theunissen, T.W., Powell, B.E., Wang, H., Mitalipova, M., Faddah, D.A., Reddy, J., Fan, Z.P., Maetzel, D., Ganz, K., Shi, L., et al. (2014a). Systematic Identification of Defined Conditions for Induction and Maintenanceof Naive Human Pluripotency. Cell Stem Cell 1-17.
Theunissen, T.W., Powell, B.E., Wang, H., Mitalipova, M., Faddah, D.A., Reddy, J., Fan, Z.P., Maetzel, D., Ganz, K., Shi, L., et al. (2014b). Systematic Identification of Culture Conditions for Induction and Maintenance of Naive Human Pluripotency. Cell Stem Cell 15, 471-487.
Theunissen, T.W., Friedli, M., He, Y., Planet, E., O'Neil, R.C., Markoulaki, S., Pontis, J., Wang, H., Iouranova, A., Imbeault, M., et al. (2016). Molecular Criteria for Defining the Naive Human Pluripotent State. Cell Stem Cell 1-49.
Weinberger, L., Ayyash, M., Novershtern, N., and Hanna, J.H. (2016a). Dynamic stem cell states: Naive to primed pluripotency in rodents and humans. Nat. Rev. Mol. Cell Biol. 17, 155-169.
Weinberger, L., Ayyash, M., Novershtern, N., and Hanna, J.H. (2016b). Dynamic stem cell states: Naive to primed pluripotency in rodents and humans. Nat. Rev. Mol. Cell Biol. 17, 155-169.
Yang, Y., Liu, B., Xu, J., Wang, J., Wu, J., Shi, C., Xu, Y., Dong, J., Wang, C., Lai, W., et al. (2017). Derivation of Pluripotent Stem Cells with In~Vivo Embryonic and Extraembryonic Potency. Cell 169, 243--257.e25.
Ying, Q.-L., Wray, J., Nichols, J., Batlle-Morera, L., Doble, B., Woodgett, J., Cohen, P., and Smith, A. (2008). The ground state of embryonic stem cell self-renewal. 453, 519-523.
Zimmerlin, L., Park, T.S., Huo, J.S., Verma, K., Pather, S.R., Talbot Jr, C.C., Agarwal, J., Steppan, D., Zhang, Y.W., Considine, M., et al. (2016). Tankyrase inhibition promotes a stable human naiive pluripotent state with improved functionality. Development dev.138982--78.

## Claims

1. A culture medium comprising a WNT inhibitor selected from the group consisting of (i) Tankyrase inhibitor and (ii) a small molecule inhibitor for Porcupine enzyme, a SRC inhibitor, a protein kinase C (PKC) inhibitor and an ERK inhibitor, said medium does not contain more than 0.1 μM of a GSK3β inhibitor , and wherein said medium maintains human pluripotent stem cells in a naive, undifferentiated and pre-X-inactivation state for at least 2 passages, wherein said pre-X-inactivation is **characterized by** presence of two unmethylated alleles of an X-inactive specific transcript (XIST) gene in a female cell, and presence an unmethylated allele of the XIST gene in a male cell.

2. A culture medium comprising a WNT inhibitor selected from the group consisting of (i) Tankyrase inhibitor and (ii) a small molecule inhibitor for Porcupine enzyme, a Notch inhibitor selected from the group consisting of gamma secretase inhibitor and RBPj inhibitor and a protein kinase C (PKC) inhibitor, said medium does not contain more than 0.1 μM of a GSK3β inhibitor, and wherein said medium maintains human pluripotent stem cells in a naive, undifferentiated and pre-X-inactivation state for at least 2 passages, wherein said pre-X-inactivation is **characterized by** presence of two unmethylated alleles of an X-inactive specific transcript (XIST) gene in a female cell, and presence an unmethylated allele of the XIST gene in a male cell.

3. The culture medium of claim 2, further comprising at least one agent selected from the group consisting of a STAT3 activator, a SRC inhibitor and an ERK inhibitor.

4. The culture medium of claim 1, further comprising at least one agent selected from the group consisting of a STAT3 activator, a p38 inhibitor, a JNK inhibitor and a ROCK inhibitor.

5. The culture medium of claim 1, further comprises a Notch inhibitor selected from the group consisting of gamma secretase inhibitor and/or RBPj inhibitor.

6. The culture medium of any one of claim 1-5, further comprising Activin A.

7. The culture medium of any one of claims 1-6, being devoid of animal serum.

8. The culture medium of any one of claims 1-6, further comprising serum replacement.

9. A cell culture comprising primate pluripotent stem cells and the culture medium of any one of claims 1-8.

10. A method of expanding primate pluripotent stem cells (PSCs), comprising culturing the primate pluripotent stem cell in the culture medium of any one of claims 1-8, thereby culturing the primate pluripotent stem cells.

11. A method of generating an induced pluripotent stem cell (iPSC) from a primate somatic cell, comprising:

    (a) expressing within the primate somatic cell a first factor selected from the group consisting of Nanog, ESRRB, KLF17, TFAP2C, TBX3, ERAS and a second factor selected from the group consisting of Nanog, ESRRB, KLF17, TBX3, ERAS, Oct4, Sox2, Klf4,c-Myc, wherein the first and second factor are non-identical; and
    (b) culturing said primate somatic cell in the culture medium of any one of claims 1-8 under conditions that promote the generation of an iPSC, thereby generating the iPSC from a somatic cell.

12. A method of generating a naive pluripotent stem cell (PSC), comprising culturing a non-naive primate PSC cell in the culture medium of any one of claims 2, and 5-8, under conditions which allow generation of the naive PSC from said non-naive PSC, thereby generating the naive PSC.

13. The cell culture of claim 9, or the method of any one of claims 10-12, wherein said primate pluripotent stem cells comprise human pluripotent stem cells.

## Patentansprüche

1. Kulturmedium, umfassend einen WNT-Inhibitor, der ausgewählt ist aus einer Gruppe bestehend aus (i) einem Tankyrase-Inhibitor und (ii) einem niedermolekularen Inhibitor für ein PORCN-Enzym, einem SRC-Inhibitor, einem Proteinkinase C (PKC) -Inhibitor und einem ERK-Inhibitor, wobei das Medium nicht mehr als 0,1 μM eines GSK3ß-

Inhibitors enthält, und wobei das Medium humane pluripotente Stammzellen in einem naiven, undifferenzierten und Vor-X-InaktivierungsZustand über mindestens 2 Durchgänge aufrechterhält, wobei die Vor-X-Inaktivierung **gekennzeichnet ist durch** die Anwesenheit von zwei unmethylierten Allelen eines X-inaktiven spezifischen Transkript (XIST) -Gens in einer weiblichen Zelle und die Anwesenheit eines unmethylierten Allels des XIST-Gens in einer männlichen Zelle.

**2.** Kulturmedium, umfassend einen WNT-Inhibitor, der ausgewählt ist aus einer Gruppe bestehend aus (i) einem Tankyrase-Inhibitor und (ii)
einem niedermolekularen Inhibitor für ein PORCN-Enzym, einem Notch-Inhibitor, der ausgewählt ist aus der Gruppe bestehend aus einem Gamma-Sekretase-Inhibitor und einem RBPj-Inhibitor und einem Protein-Kinase-C (PKC) -Inhibitor, wobei das Medium nicht mehr als 0,1 μM eines GSK3ß-Inhibitors enthält, und wobei das Medium humane pluripotente Stammzellen in einem naiven, undifferenzierten und Vor-X-Inaktivierungs-Zustand über mindestens 2 Durchgänge aufrechterhält, wobei die Vor-X-Inaktivierung **gekennzeichnet ist durch** die Anwesenheit von zwei unmethylierten Allelen eines X-inaktiven spezifischen Transkript (XIST) -Gens in einer weiblichen Zelle und die Anwesenheit eines unmethylierten Allels des XIST-Gens in einer männlichen Zelle.

**3.** Kulturmedium nach Anspruch 2, ferner umfassend mindestens ein Mittel, das ausgewählt ist aus der Gruppe bestehend aus einem STAT3-Aktivator, einem SRC-Inhibitor und einem ERK-Inhibitor.

**4.** Kulturmedium nach Anspruch 1, ferner umfassend mindestens ein Mittel, das ausgewählt ist aus der Gruppe bestehend aus einem STAT3-Aktivator, einem p38-Inhibitor, einem JNK-Inhibitor und einem ROCK-Inhibitor.

**5.** Kulturmedium nach Anspruch 1, ferner umfassend einen Notch-Inhibitor, der ausgewählt ist aus der Gruppe bestehend aus einem Gamma-Sekretase-Inhibitor und/oder einem RBPj-Inhibitor.

**6.** Kulturmedium nach einem der Ansprüche 1-5, ferner umfassend Aktivin A.

**7.** Kulturmedium nach einem der Ansprüche 1-6, das frei von Tierserum ist.

**8.** Kulturmedium nach einem der Ansprüche 1-6, ferner umfassend einen Serumersatz.

**9.** Zellkultur, umfassend pluripotente Stammzellen von Primaten und das Kulturmedium nach einem der Ansprüche 1-8.

**10.** Verfahren zum Erweitern von pluripotenten Stammzellen (PSCs) von Primaten, umfassend das Kultivieren der pluripotenten Stammzellen von Primaten in dem Kulturmedium nach einem der Ansprüche 1-8, dadurch Kultivieren der pluripotenten Stammzellen von Primaten.

**11.** Verfahren zum Erzeugen einer induzierten pluripotenten Stammzelle (iPSC) aus einer somatischen Zelle von Primaten, umfassend

(a) Exprimieren innerhalb der somatischen Zelle von Primaten eines ersten Faktors, der ausgewählt ist aus der Gruppe bestehend aus Nanog, ESRRB, KLF17, TFAP2C, TBX3, ERAS und einem zweiten Faktor, der ausgewählt ist aus der Gruppe bestehend aus Nanog, ESRRB, KLF17, TBX3, ERAS, Oct4, Sox2, Klf4,c-Myc, wobei der erste und der zweite Faktor nicht identisch sind; und
(b) Kultivieren der somatischen Zelle von Primaten in dem Kulturmedium nach einem der Ansprüche 1-8 unter Bedingungen, welche die Erzeugung einer iPSC fördern, dadurch Erzeugen der iPSC aus einer somatischen Zelle.

**12.** Verfahren zum Erzeugen einer naiven pluripotenten Stammzelle (PSC), umfassend das Kultivieren einer nicht-naiven PSC-Zelle von Primaten in dem Kulturmedium nach einem der Ansprüche 2 und 5-8, unter Bedingungen, welche die Erzeugung der naiven PSC aus der nicht-naiven PSC erlauben, dadurch Erzeugen der naiven PSC.

**13.** Zellkultur nach Anspruch 9 oder Verfahren nach einem der Ansprüche 10-12, wobei die pluripotenten Stammzellen von Primaten humane pluripotente Stammzellen umfassen.

**Revendications**

1. Milieu de culture comprenant un inhibiteur de WNT sélectionné dans le groupe consistant en (i) inhibiteur de la tankyrase et (ii) un inhibiteur à petites molécules pour l'enzyme de porc-épic, un inhibiteur de SRC, un inhibiteur de protéine kinase C (PKC) et un inhibiteur d'ERK, ledit milieu ne contient pas plus de 0,1 μM) d'un inhibiteur de GSK3ß, et dans lequel ledit milieu maintient les cellules souches pluripotentes humaines dans un état naïf, indifférencié et de pré-inactivation de l'X pendant au moins 2 passages, dans lequel ladite pré-inactivation de l'X est **caractérisée par** la présence de deux allèles non méthylés d'un gène transcrit spécifique de l'inactivation de l'X (XIST) dans une cellule femelle, et par la présence d'un allèle non méthylé du gène XIST dans une cellule mâle.

2. Milieu de culture comprenant un inhibiteur de WNT sélectionné dans le groupe consistant en (i) inhibiteur de la tankyrase et (ii)
un inhibiteur à petites molécules pour l'enzyme de porc-épic, un inhibiteur de Notch sélectionné dans le groupe consistant en inhibiteur de sécrétase gamma et inhibiteur de RBPj et un inhibiteur de protéine kinase C (PKC), ledit milieu ne contient pas plus de 0,1 μM) d'un inhibiteur de GSK3ß, et dans lequel ledit milieu maintient les cellules souches pluripotentes humaines dans un état naïf, indifférencié et de pré-inactivation de l'X pendant au moins 2 passages, dans lequel ladite pré-inactivation de l'X est **caractérisée par** la présence de deux allèles non méthylés d'un gène transcrit spécifique de l'inactivation de l'X (XIST) dans une cellule femelle, et par la présence d'un allèle non méthylé du gène XIST dans une cellule mâle.

3. Milieu de culture selon la revendication 2, comprenant en outre au moins un agent sélectionné dans le groupe consistant en un activateur de STAT3, un inhibiteur de SRC et un inhibiteur de ERK.

4. Milieu de culture selon la revendication 1, comprenant en outre au moins un agent sélectionné dans le groupe consistant en un activateur de STAT3, un inhibiteur de p38, un inhibiteur de JNK et un inhibiteur de ROCK.

5. Milieu de culture selon la revendication 1, comprenant en outre un inhibiteur de Notch sélectionné dans le groupe consistant en inhibiteur de sécrétase gamma et/ou inhibiteur de RBPj.

6. Milieu de culture selon l'une quelconque des revendications 1 à 5, comprenant en outre de l'activine A.

7. Milieu de culture selon l'une quelconque des revendications 1 à 6, étant dépourvu de sérum animal.

8. Milieu de culture selon l'une quelconque des revendications 1 à 6, comprenant en outre un remplacement sérique.

9. Culture cellulaire comprenant des cellules souches pluripotentes de primates et le milieu de culture selon l'une quelconque des revendications 1 à 8.

10. Procédé de développement de cellules souches pluripotentes (PSC) de primates, comprenant la mise en culture de la cellule souche pluripotente de primate dans le milieu de culture selon l'une quelconque des revendications 1 à 8, mettant ainsi en culture les cellules souches pluripotentes de primates.

11. Procédé de génération d'une cellule souche pluripotente induite (iPSC) à partir d'une cellule somatique de primate, comprenant :

    (a) l'expression à l'intérieur de la cellule somatique de primate d'un premier facteur sélectionné dans le groupe consistant en Nanog, ESRRB, KLF17, TFAP2C, TBX3, ERAS et d'un deuxième facteur sélectionné dans le groupe consistant en Nanog, ESRRB, KLF17, TBX3, ERAS, Oct4, Sox2, Klf4, c-Myc, dans lequel le premier et le deuxième facteur ne sont pas identiques ; et
    (b) la mise en culture de ladite cellule somatique de primate dans le milieu de culture selon l'une quelconque des revendications 1 à 8 dans des conditions qui favorisent la génération d'une iPSC, générant ainsi l'iPSC à partir d'une cellule somatique.

12. Procédé de génération d'une cellule souche pluripotente (PSC) naïve, comprenant la mise en culture d'une cellule PSC de primate non naïve dans le milieu de culture selon l'une quelconque des revendications 2 et 5 à 8, dans des conditions qui permettent la génération de la PSC naïve à partir de ladite PSC non naïve, générant ainsi la PSC naïve.

13. Culture cellulaire selon la revendication 9, ou procédé selon l'une quelconque des revendications 10 à 12, dans laquelle/lequel lesdites cellules souches pluripotentes de primates comprennent des cellules souches pluripotentes humaines.

FIG. 1A

**Step 1: Primed** WIBR3 hESC line (FUW-CAGGS-rtTa)

**Step 2: Exogenous** EF1a-EGFP-**TetOff**-FLAG-METTL3$^{mut}$

**Step 3: Knockout endogenous METTL3** with CRISPR/Cas9

FIG. 1B

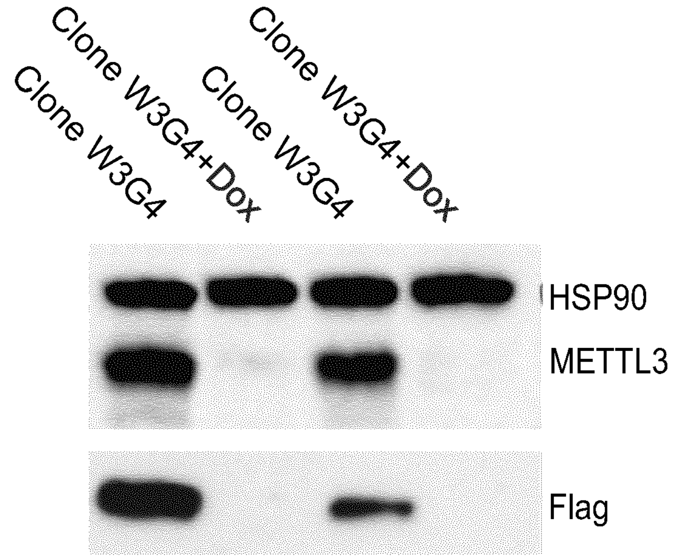


FIG. 1C

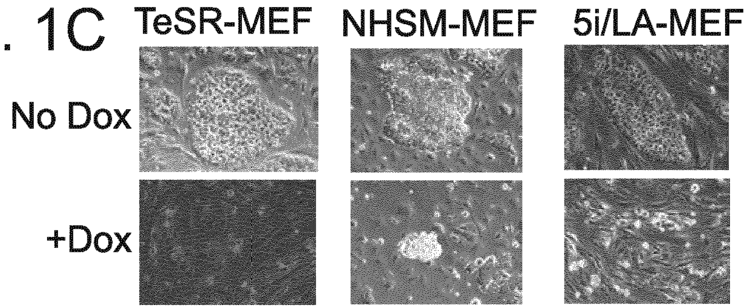


FIG. 1D

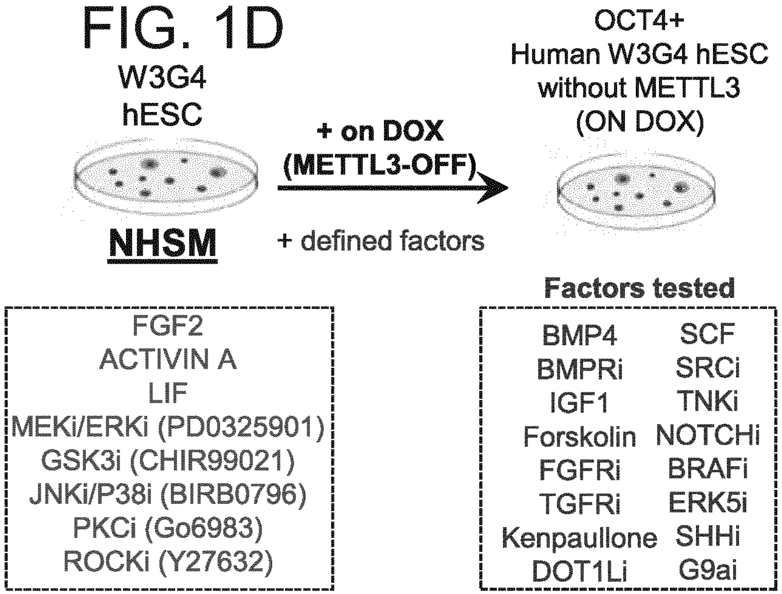


FIG. 1E

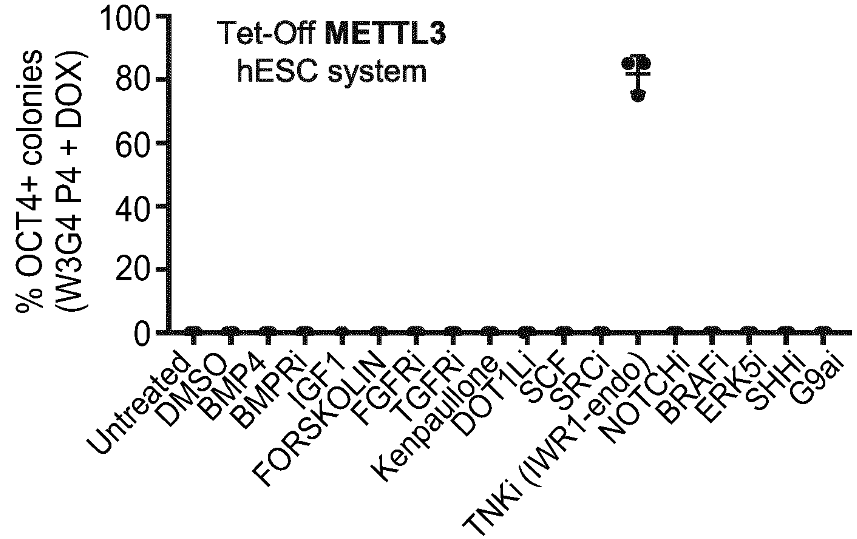

FIG. 1F

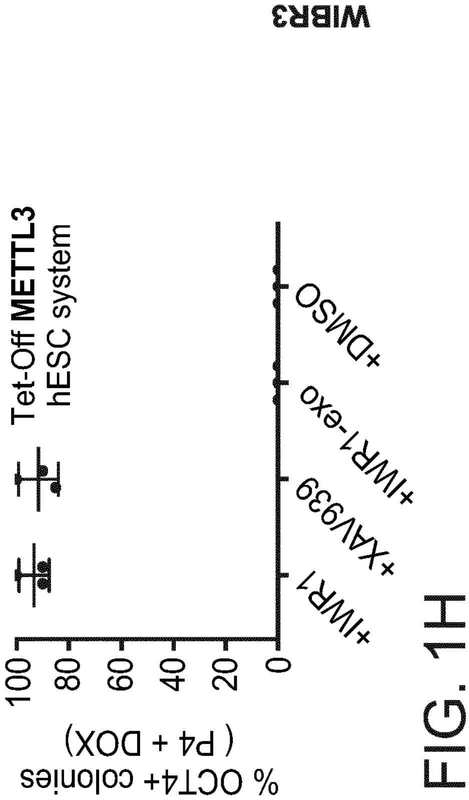


FIG. 1G

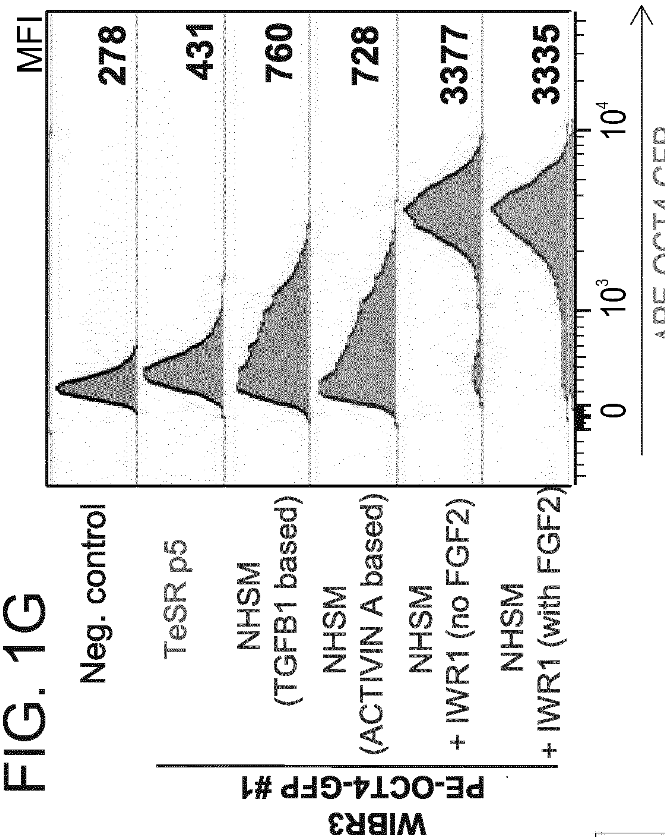


FIG. 1H

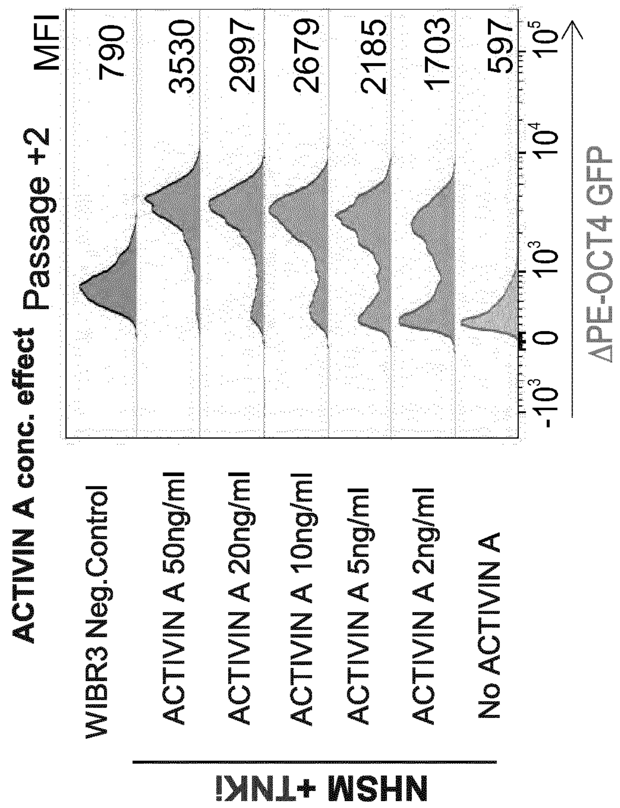


FIG. 1I

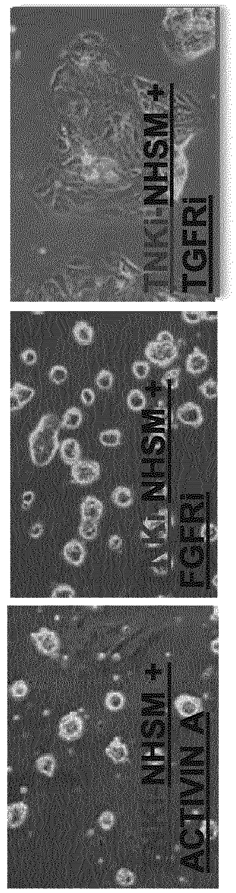

FIG. 2A

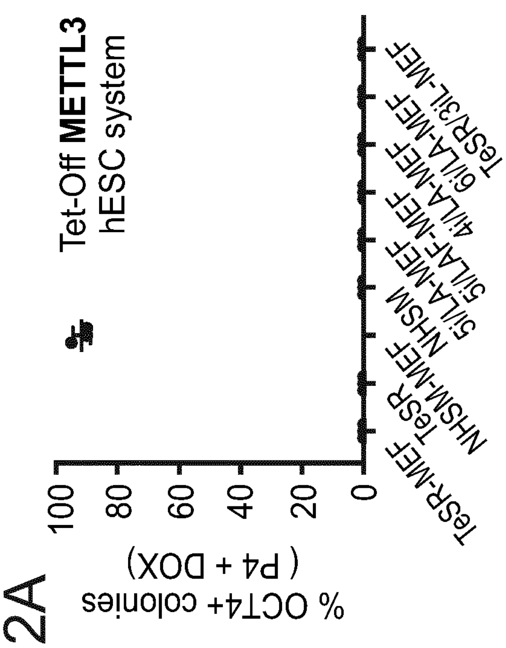


FIG. 2B

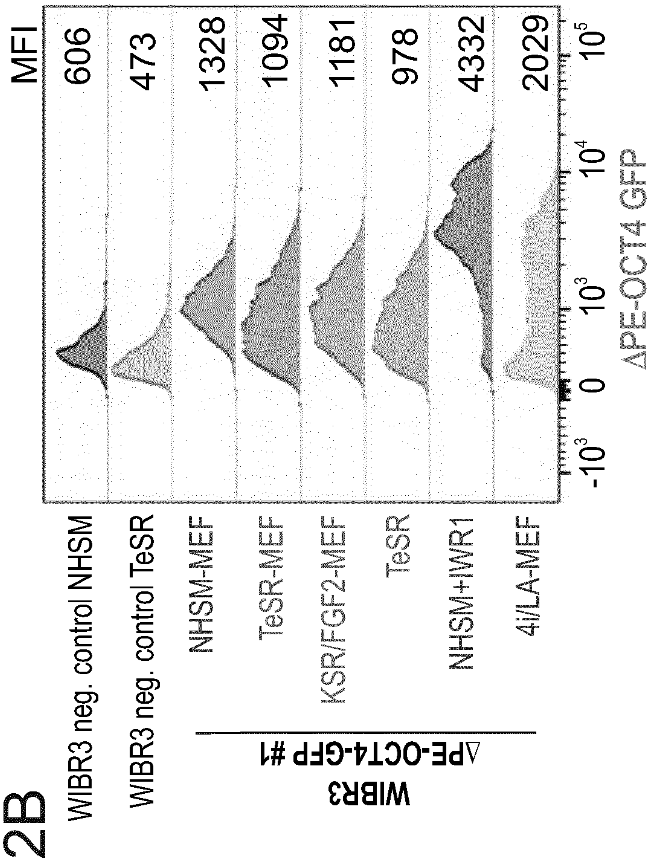


FIG. 2C

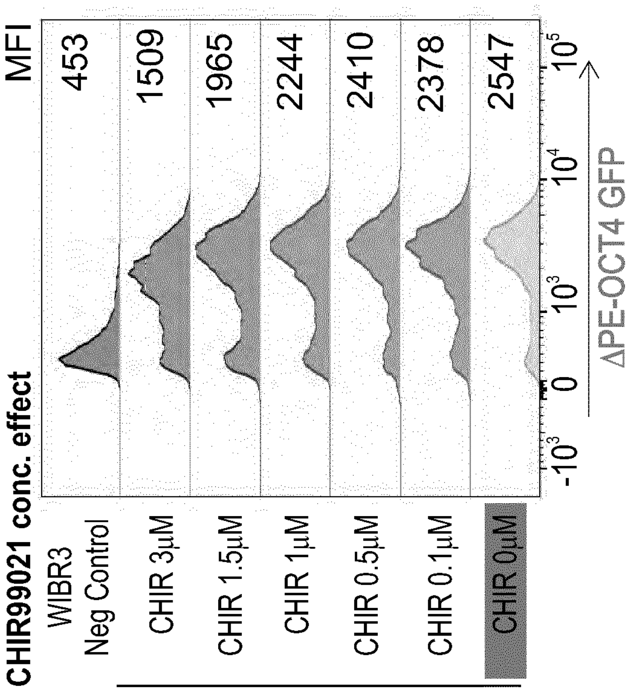


FIG. 2D

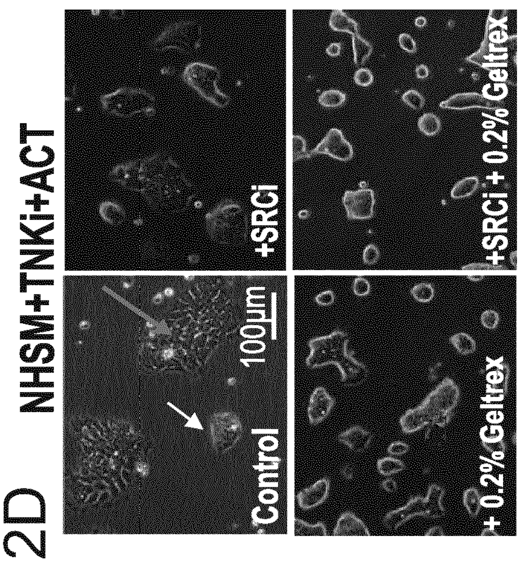

FIG. 2E

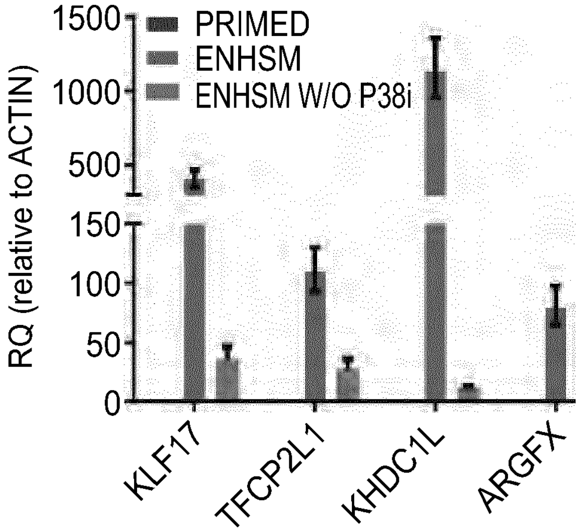


FIG. 2H

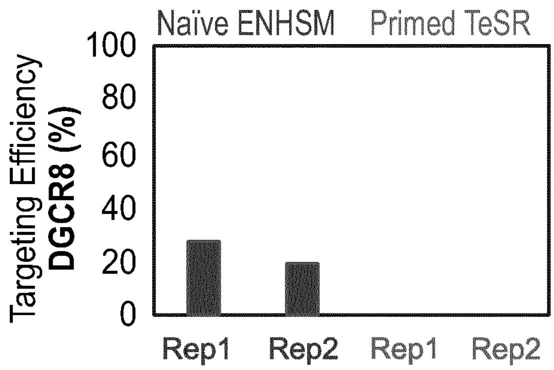


FIG. 2F

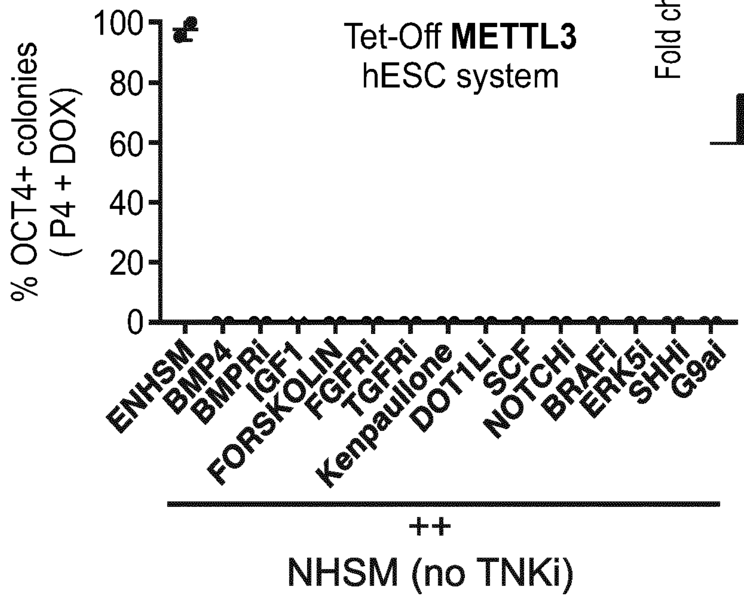


FIG. 2I

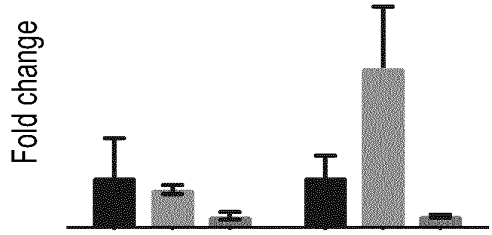


FIG. 2G

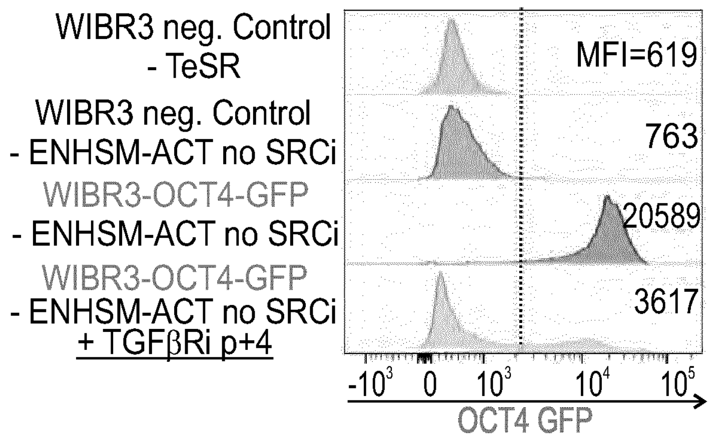

FIG. 3A

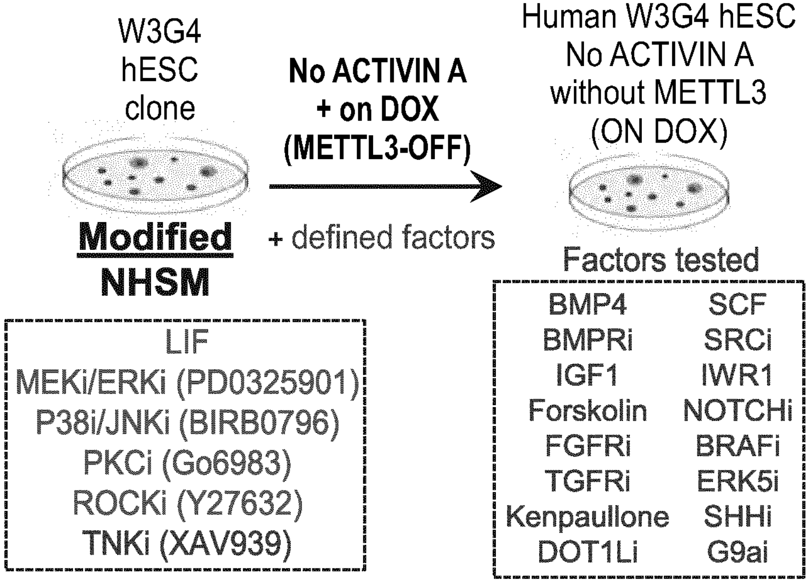


FIG. 3B

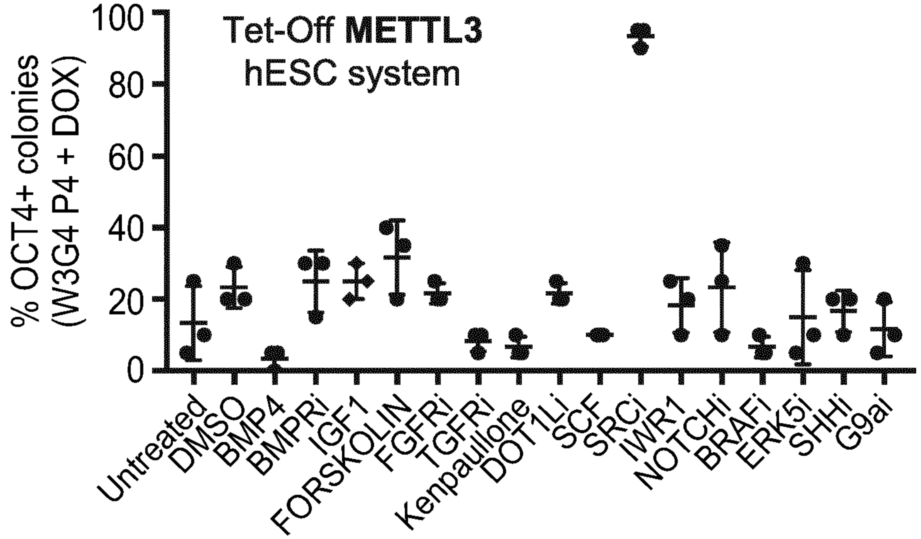


FIG. 3C

**ENHSM (6iL)**
**Conditions**

LIF (20ng/ml)
MEKi/ERKi (PD0325901 1μM)
P38i/JNKi (BIRB0796 0.9μM)
PKCi (Go6983 2μM)
ROCKi (Y27632 1.2μM)
TNKi (XAV939 3μM)
SRCi (CGP77675 1.2μM)

FIG. 3D

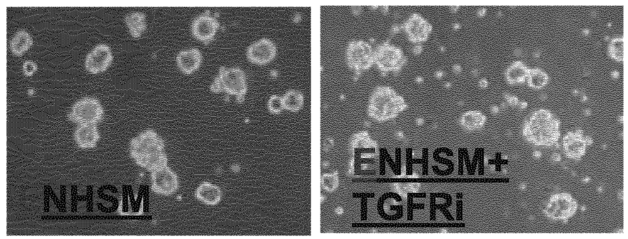

FIG. 3E

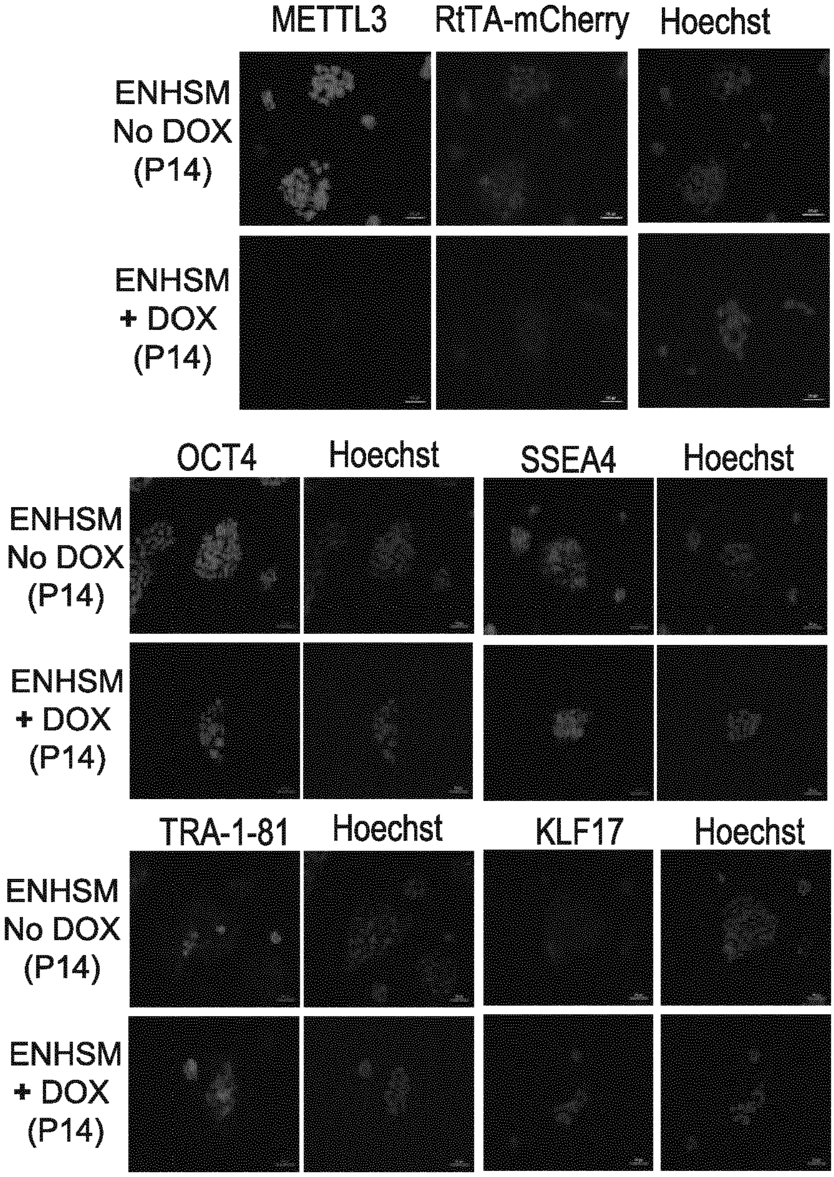


FIG. 3F

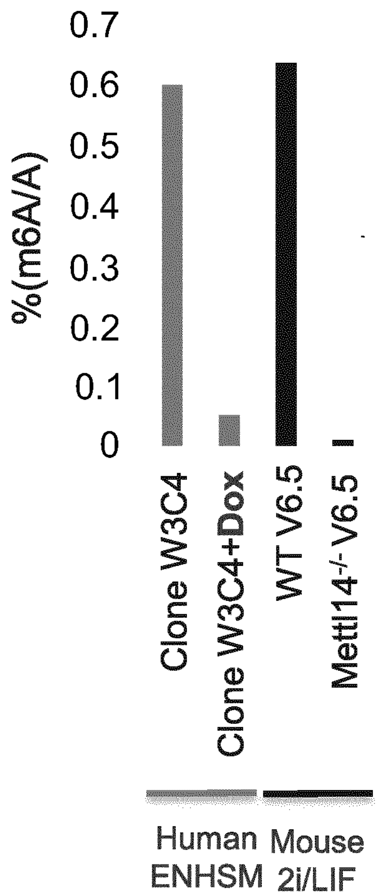


FIG. 3G

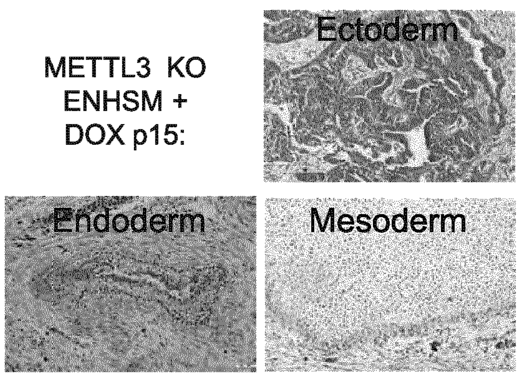

FIG. 3H

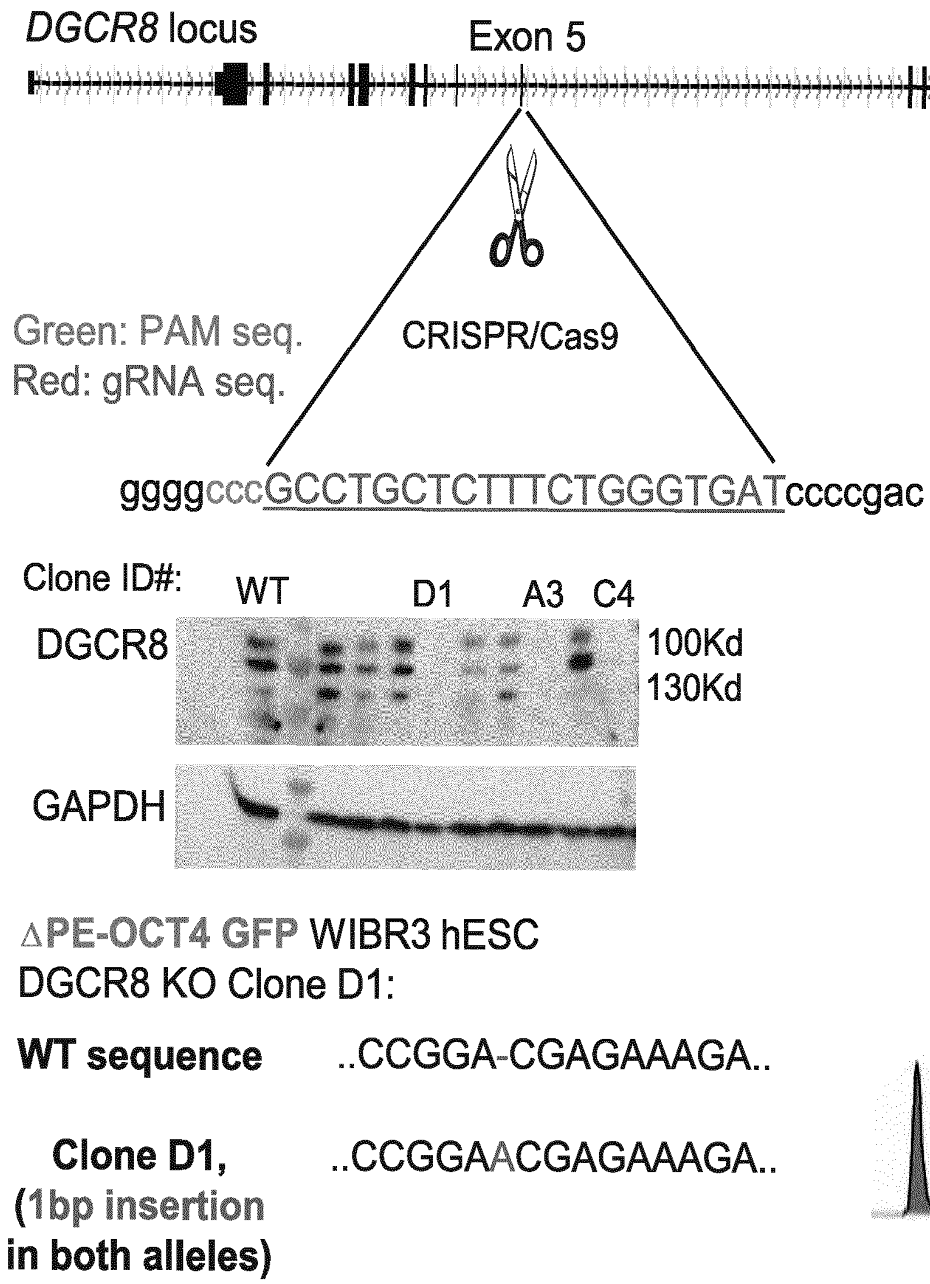
DGCR8 locus
Exon 5
CRISPR/Cas9
Green: PAM seq.
Red: gRNA seq.
ggggcccGCCTGCTCTTTCTGGGTGATccccgac
Clone ID#: WT D1 A3 C4
DGCR8
100Kd
130Kd
GAPDH
ΔPE-OCT4 GFP WIBR3 hESC
DGCR8 KO Clone D1:
WT sequence ..CCGGA-CGAGAAAGA..
Clone D1, ..CCGGAACGAGAAAGA..
(1bp insertion
in both alleles)

FIG. 3I

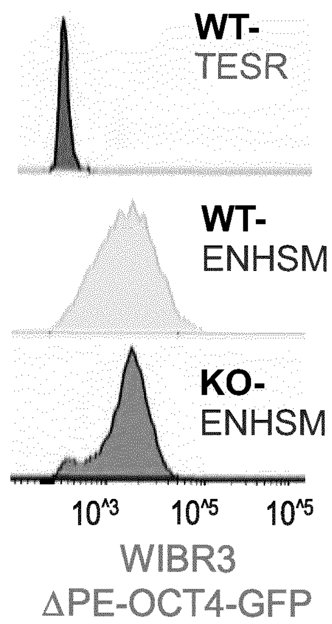
WT-
TESR
WT-
ENHSM
KO-
ENHSM
10^3 10^5 10^5
WIBR3
ΔPE-OCT4-GFP

FIG. 4A

**Step 1: Primed** HUES64 hESC line (FUW-CAGGS-rtTa)

**Step 2: Exogenous** EF1a-EGFP-**TetOff**-DNMT1$^{mut}$

**Step 3: Knockout endogenous DNMT1** with CRISPR/Cas9

FIG. 4B

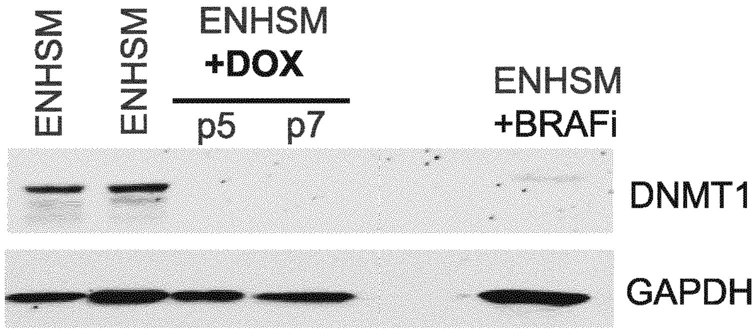


FIG. 4C

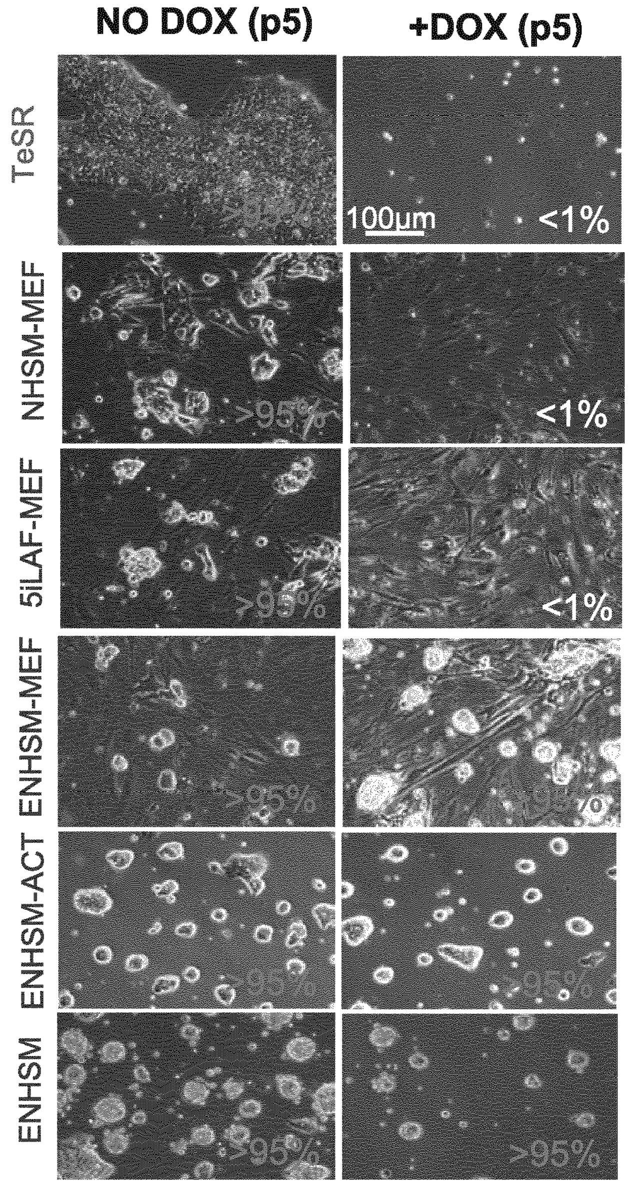


FIG. 4D

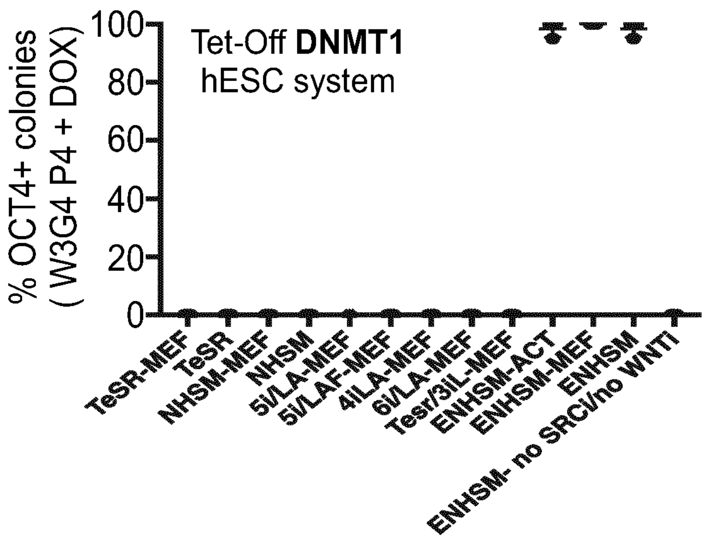


FIG. 4E

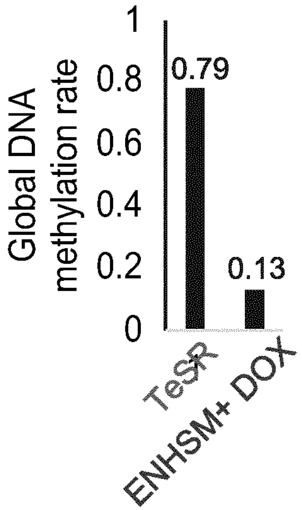

FIG. 4F

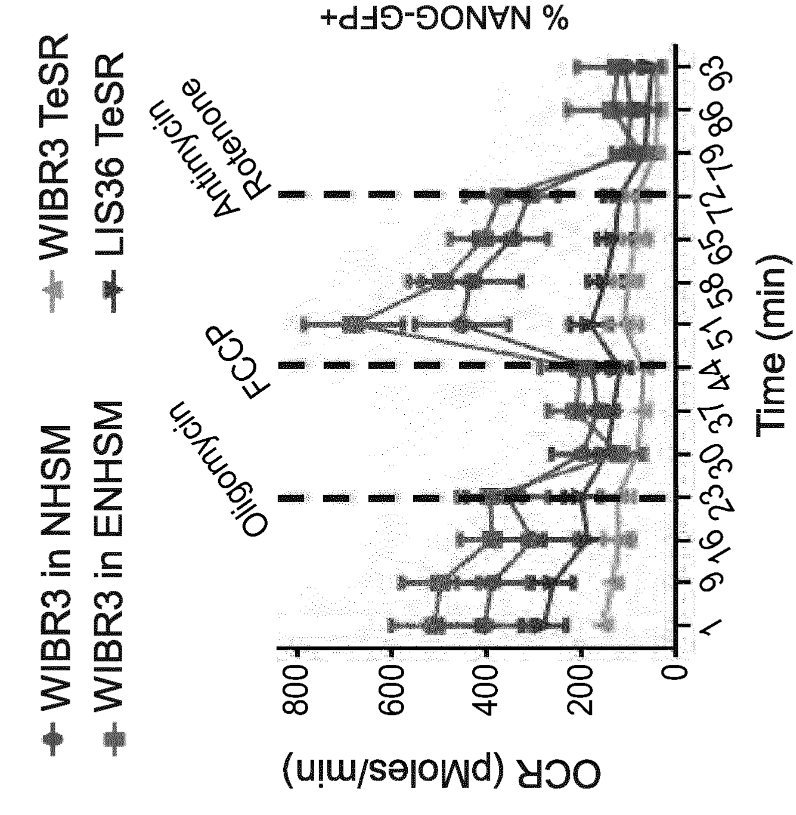
WIBR3 in NHSM
WIBR3 in ENHSM
WIBR3 TeSR
LIS36 TeSR
OCR (pMoles/min)
800
600
400
200
0
Oligomycin
FCCP
Antimycin
Rotenone
Time (min)

FIG. 4G

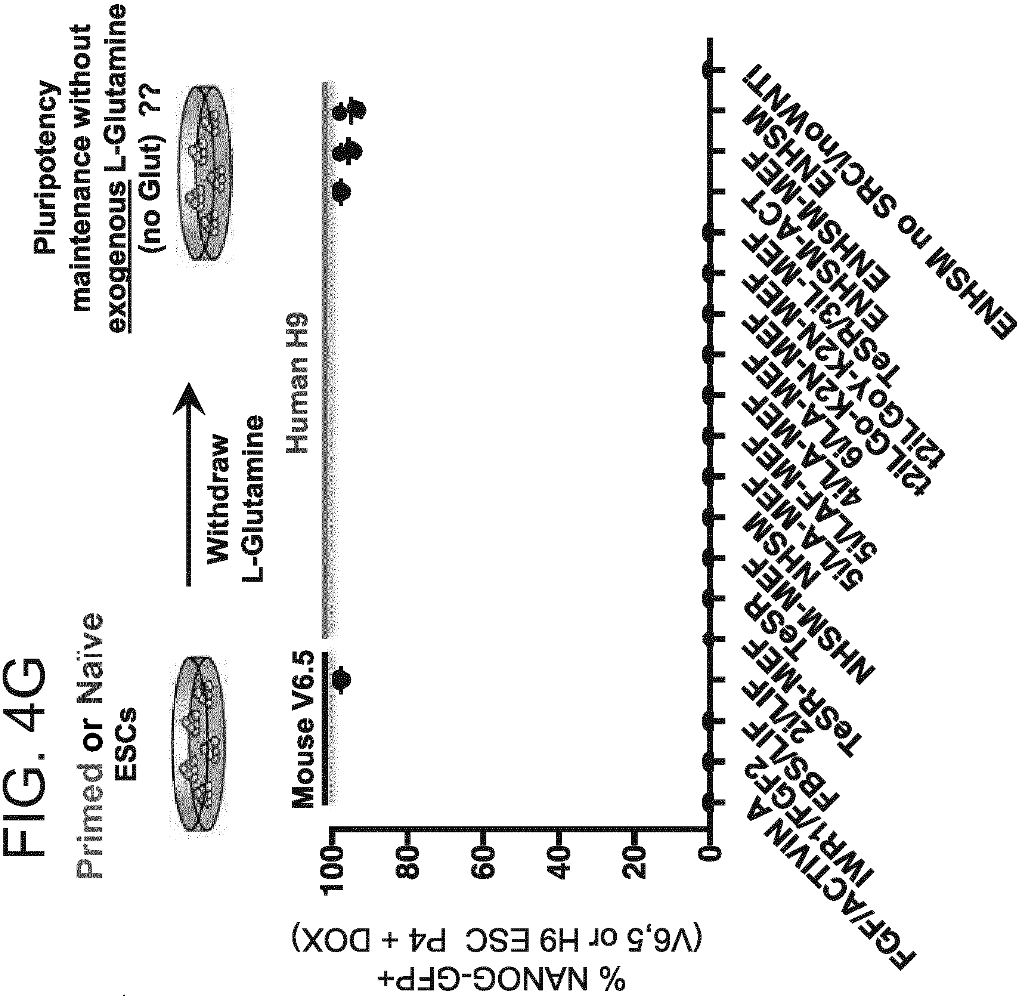
Primed or Naive
ESCs
Withdraw
L-Glutamine
Pluripotency
maintenance without
exogenous L-Glutamine
(no Glut) ??
Mouse V6.5
Human H9
% NANOG-GFP+
(V6,5 or H9 ESC P4 + DOX)
100
80
60
40
20
0
FGF/ACTIVIN A
IWR1/FGF2
FBS/LIF
2i/LIF
TeSR-MEF
TeSR
NHSM-MEF
NHSM
5i/LA-MEF
5i/LAF-MEF
4i/LA-MEF
6i/LA-MEF
t2iLGo-K2N-MEF
t2iLGoY-K2N-MEF
TeSR/3iL-MEF
ENHSM-MEF
ENHSM-ACT
ENHSM-MEF
ENHSM
ENHSM no SRC/noWNT
ENHSM no SRC

FIG. 5A

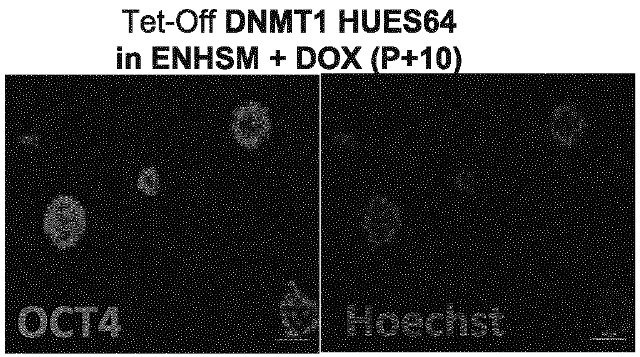


FIG. 5B

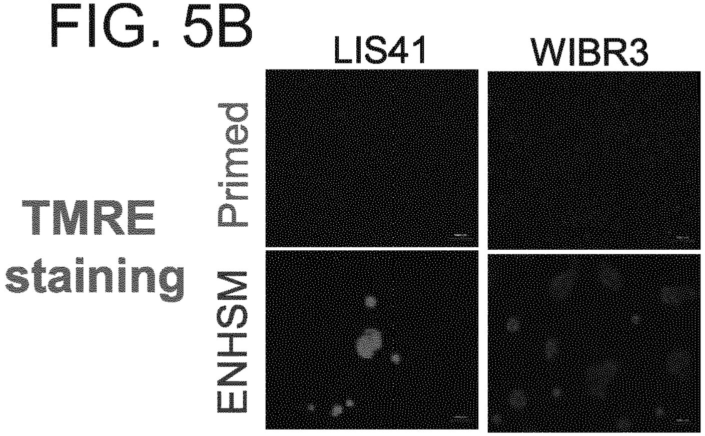


FIG. 5C

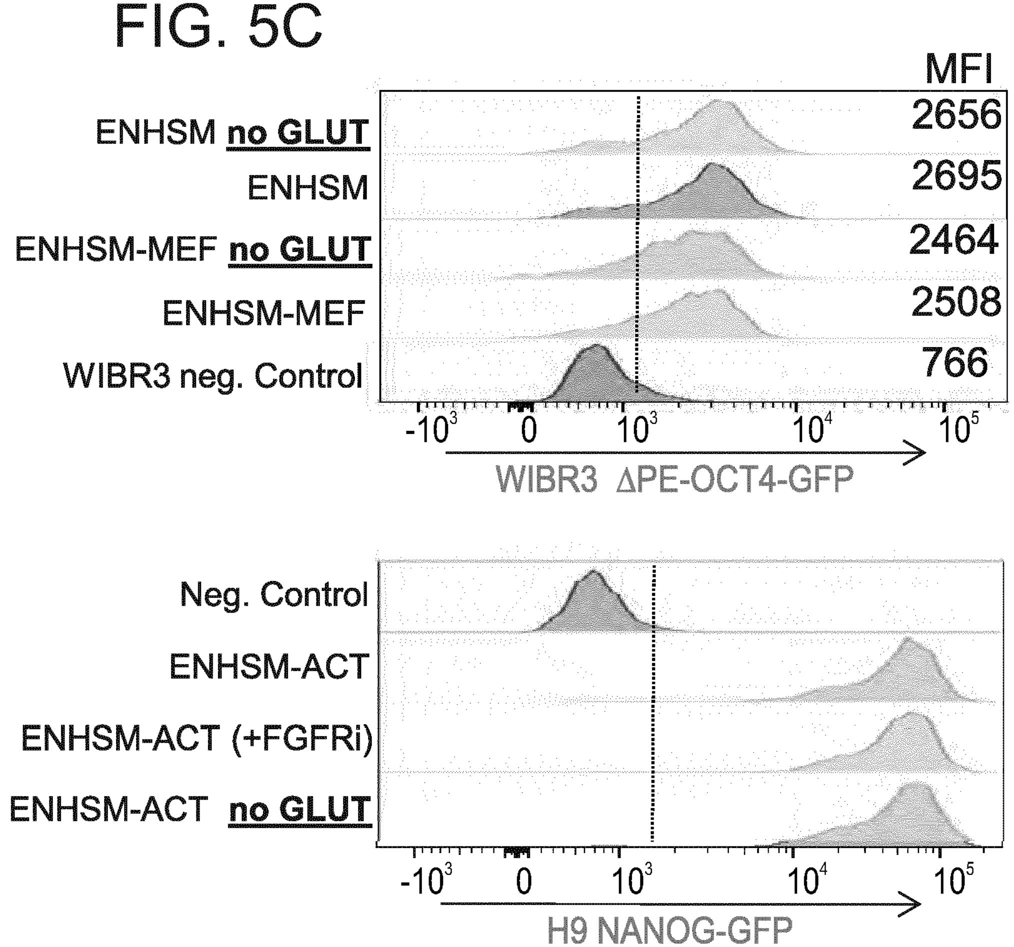

FIG. 5D

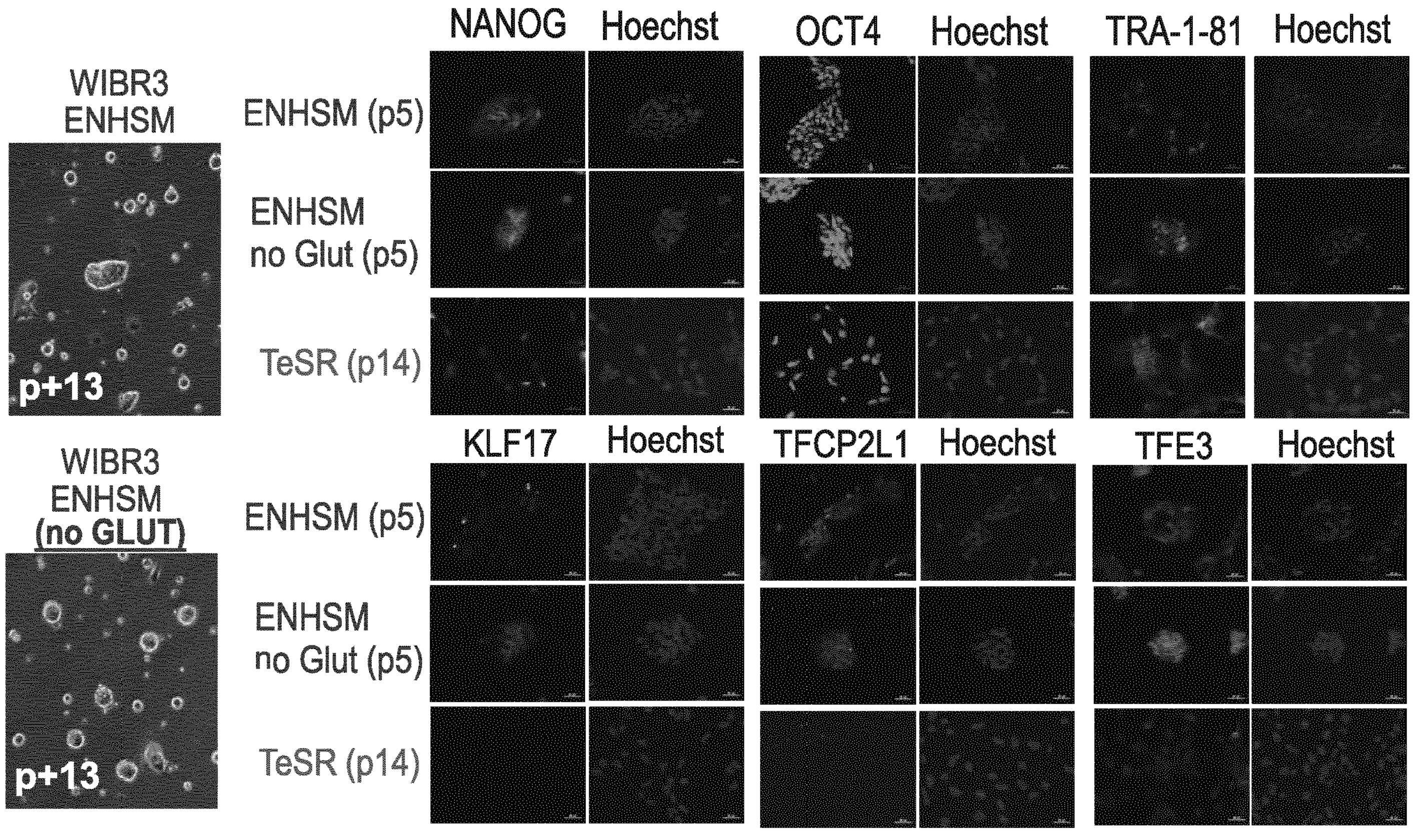
WIBR3
ENHSM
p+13
WIBR3
ENHSM
(no GLUT)
p+13
NANOG
Hoechst
OCT4
Hoechst
TRA-1-81
Hoechst
ENHSM (p5)
ENHSM
no Glut (p5)
TeSR (p14)
KLF17
Hoechst
TFCP2L1
Hoechst
TFE3
Hoechst
ENHSM (p5)
ENHSM
no Glut (p5)
TeSR (p14)

FIG. 5E

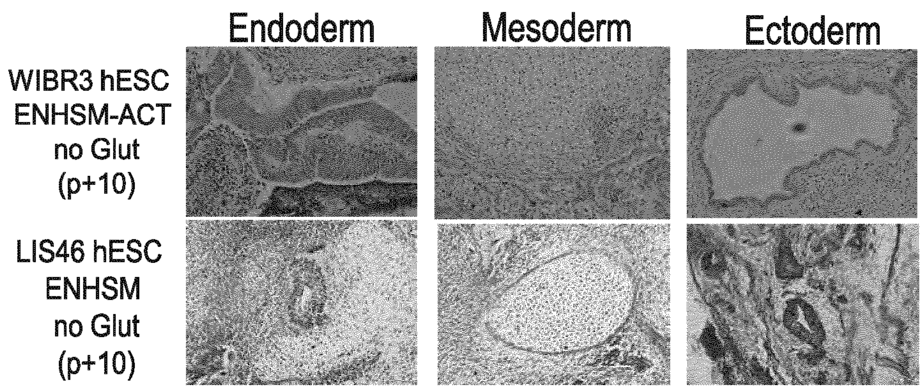
Endoderm
Mesoderm
Ectoderm
WIBR3 hESC
ENHSM-ACT
no Glut
(p+10)
LIS46 hESC
ENHSM
no Glut
(p+10)

FIG. 6A

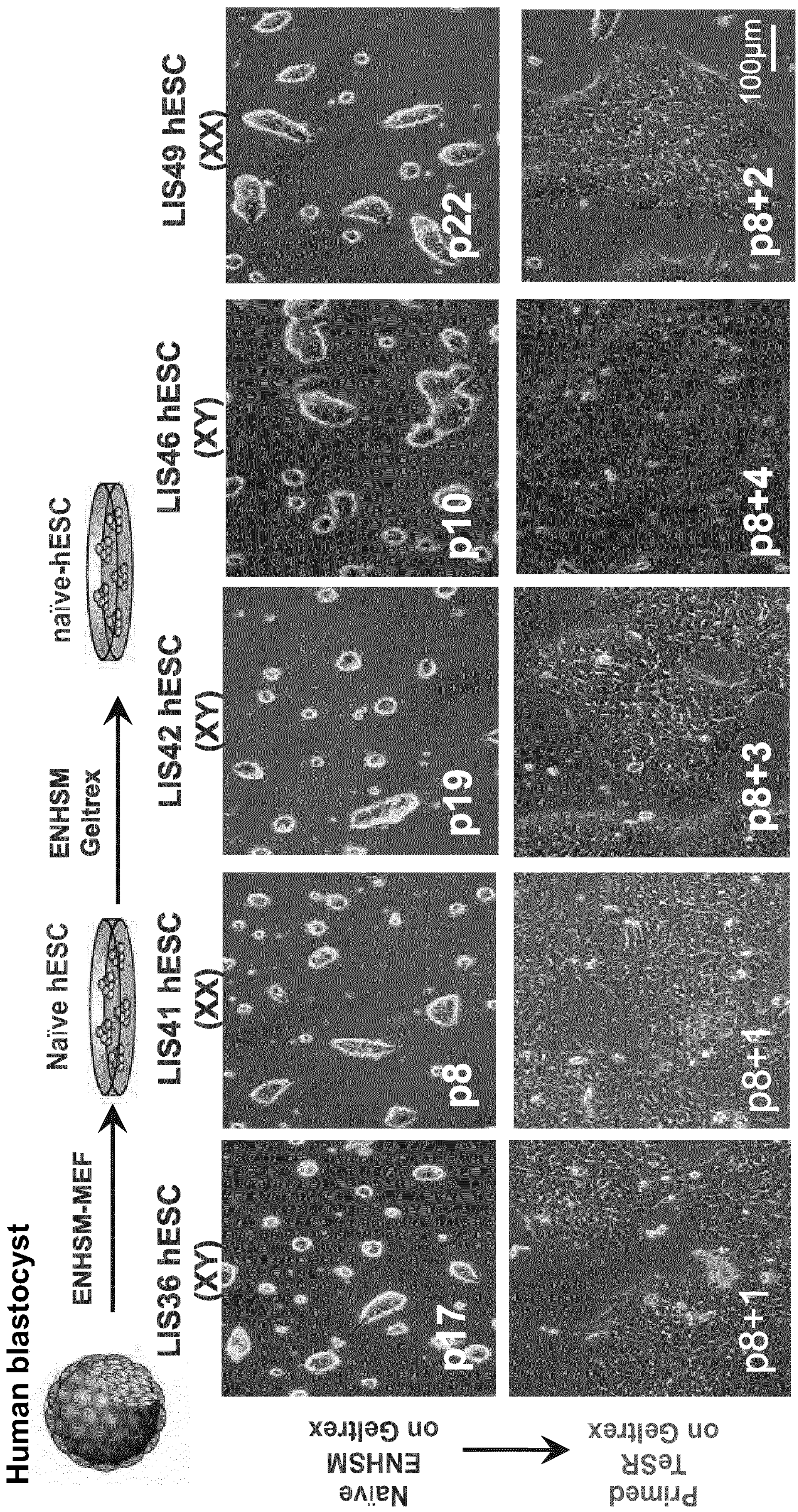

Human blastocyst
ENHSM-MEF
Naïve hESC
ENHSM
Geltrex
naïve-hESC
LIS36 hESC (XY)
LIS41 hESC (XX)
LIS42 hESC (XY)
LIS46 hESC (XY)
LIS49 hESC (XX)
p17
p8
p19
p10
p22
p8+1
p8+1
p8+3
p8+4
p8+2
100µm
Naïve ENHSM on Geltrex
Primed TeSR on Geltrex

FIG. 6B

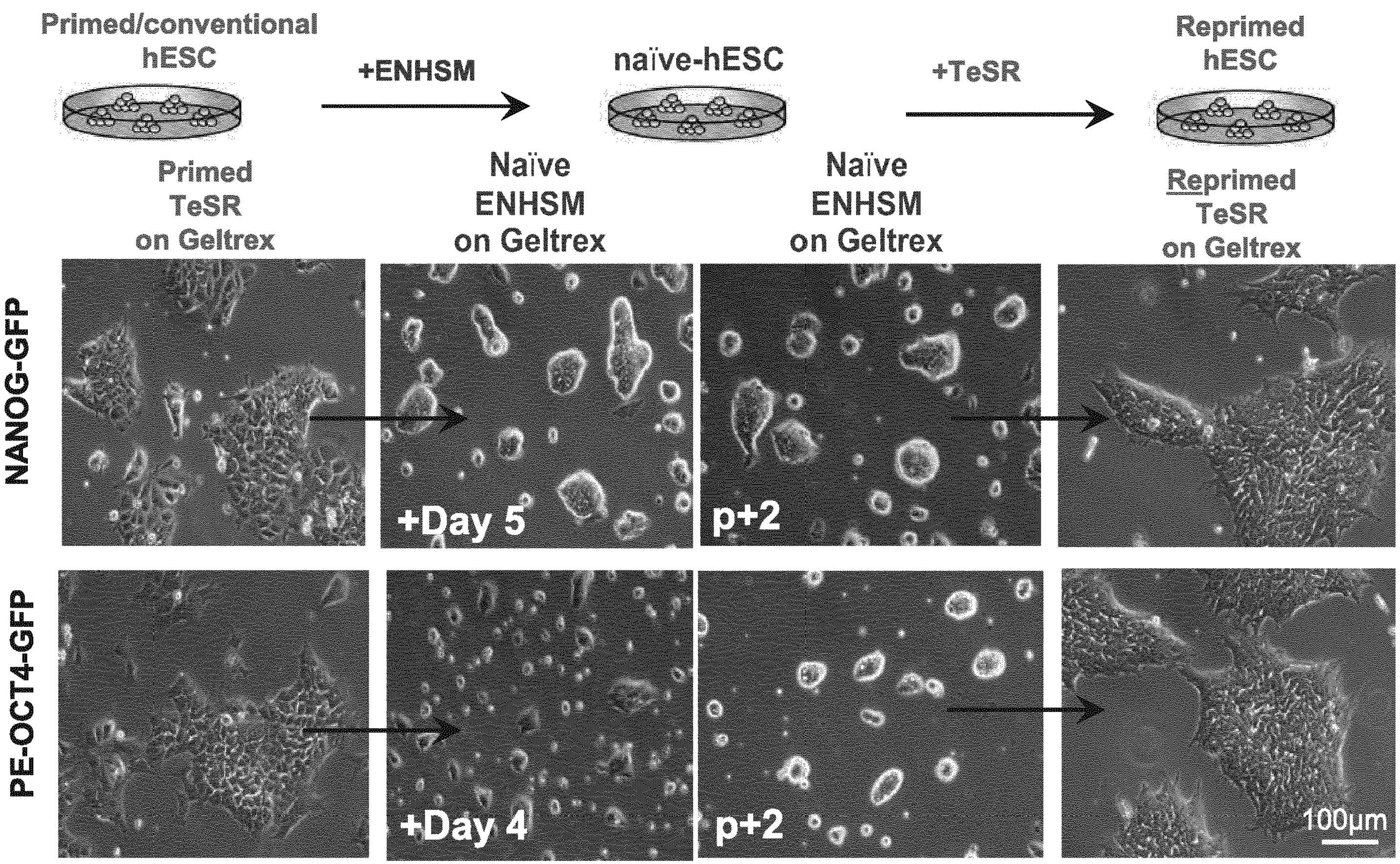
Primed/conventional hESC
+ENHSM
naïve-hESC
+TeSR
Reprimed hESC
Primed TeSR on Geltrex
Naïve ENHSM on Geltrex
Naïve ENHSM on Geltrex
Reprimed TeSR on Geltrex
H9 NANOG-GFP
WIBR3 PE-OCT4-GFP
+Day 5
p+2
+Day 4
p+2
100μm

FIG. 6C

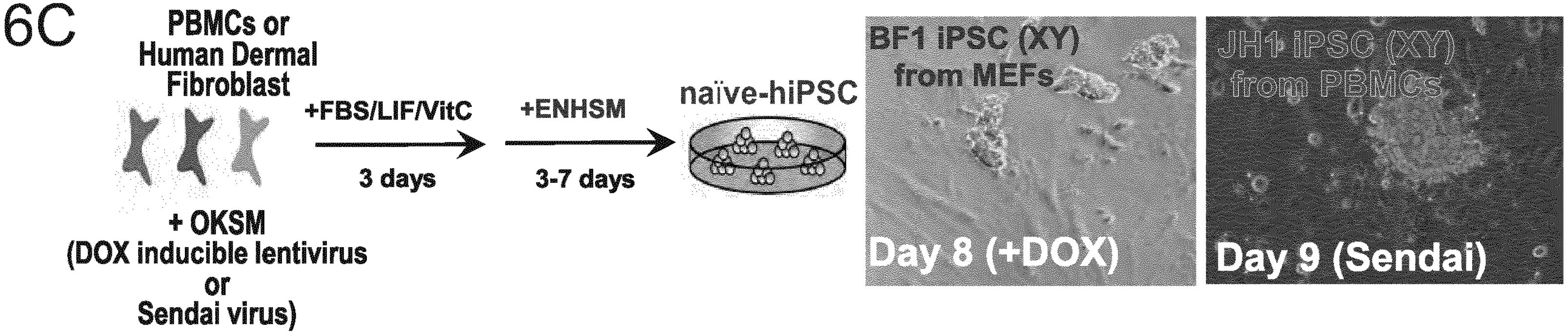
PBMCs or Human Dermal Fibroblast
+ OKSM (DOX inducible lentivirus or Sendai virus)
+FBS/LIF/VitC
3 days
+ENHSM
3-7 days
naïve-hiPSC
BF1 iPSC (XY) from MEFs
Day 8 (+DOX)
JH1 iPSC (XY) from PBMCs
Day 9 (Sendai)

FIG. 7

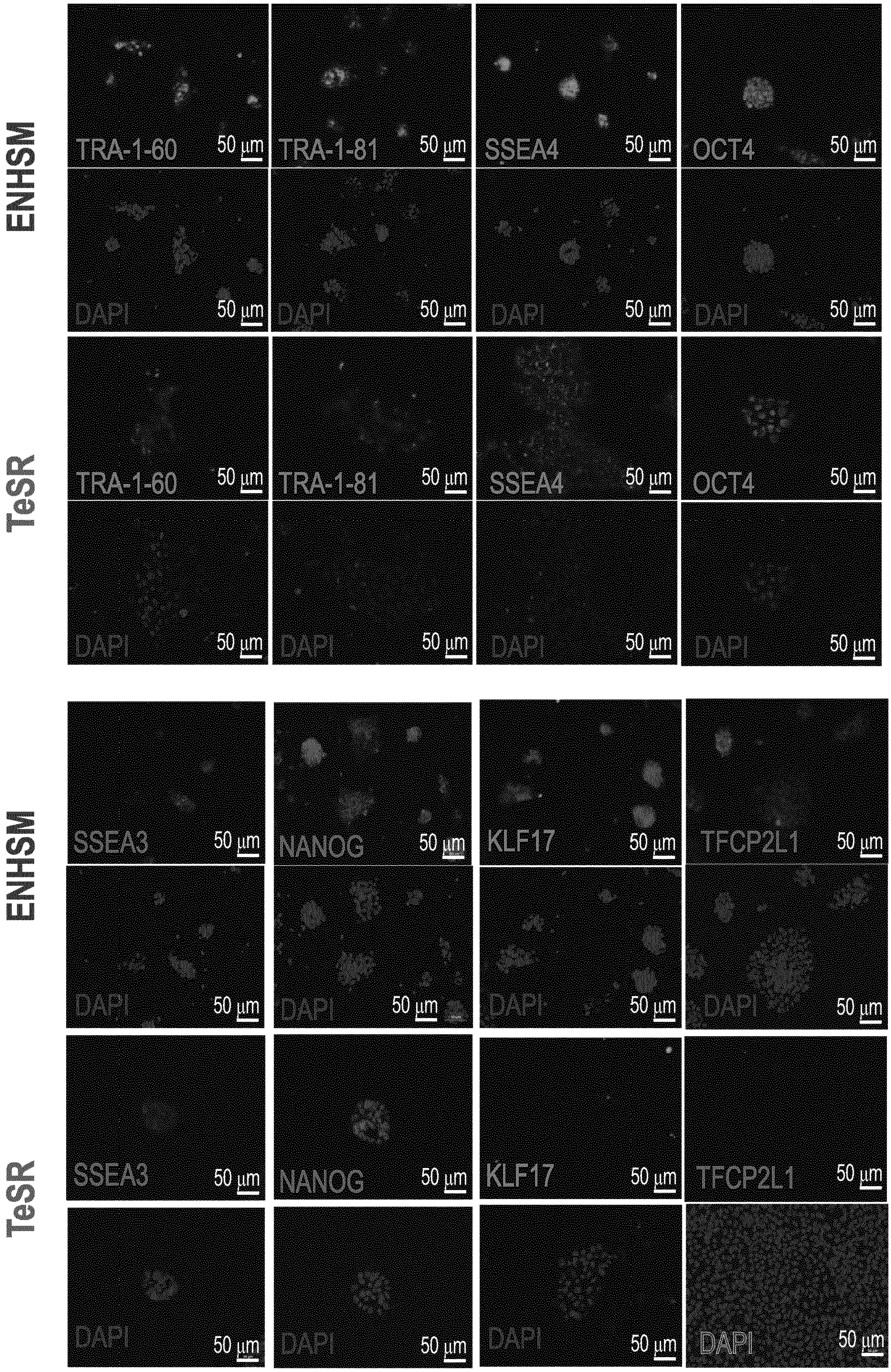
ENHSM
TeSR
TRA-1-60
TRA-1-81
SSEA4
OCT4
DAPI
SSEA3
NANOG
KLF17
TFCP2L1
50 µm

FIG. 8

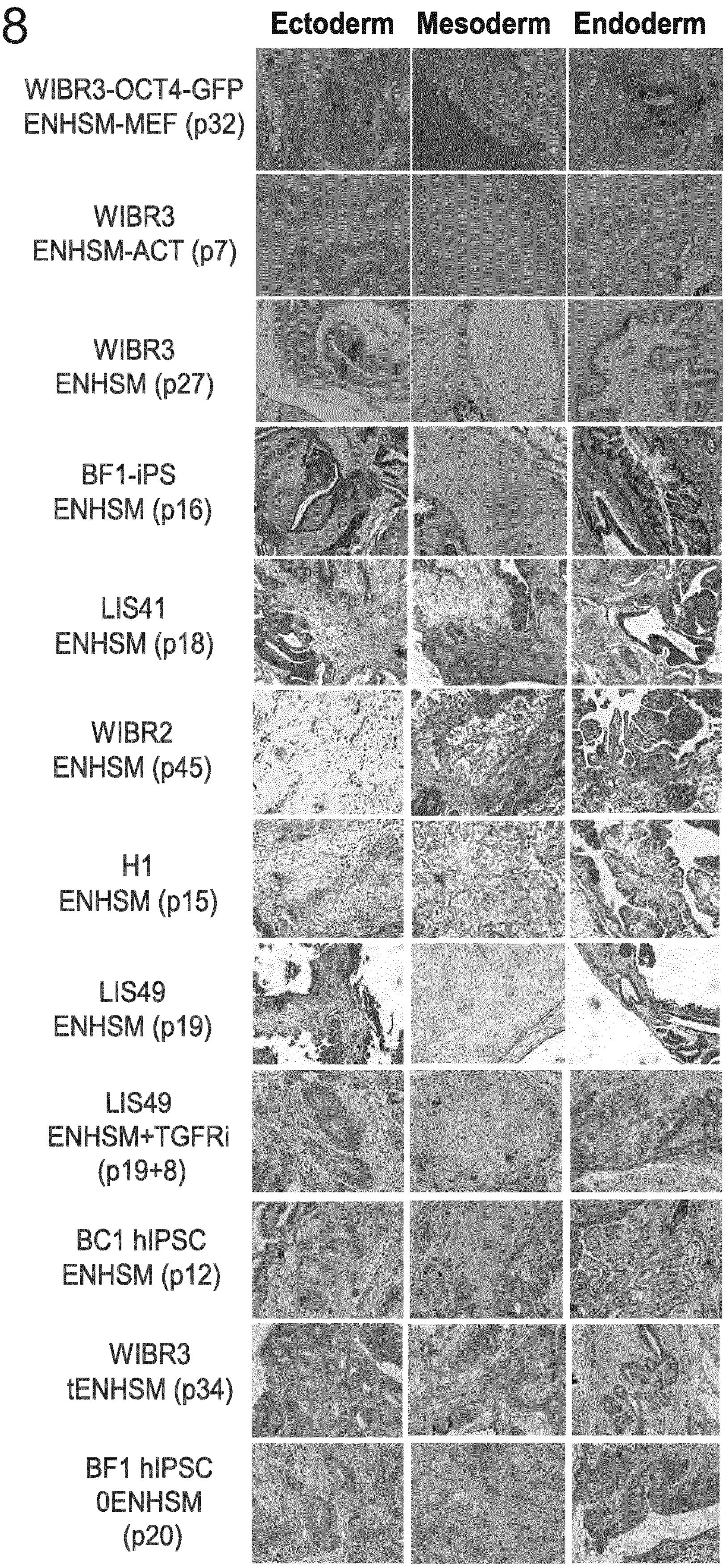
Ectoderm
Mesoderm
Endoderm
WIBR3-OCT4-GFP
ENHSM-MEF (p32)
WIBR3
ENHSM-ACT (p7)
WIBR3
ENHSM (p27)
BF1-iPS
ENHSM (p16)
LIS41
ENHSM (p18)
WIBR2
ENHSM (p45)
H1
ENHSM (p15)
LIS49
ENHSM (p19)
LIS49
ENHSM+TGFRi
(p19+8)
BC1 hIPSC
ENHSM (p12)
WIBR3
tENHSM (p34)
BF1 hIPSC
0ENHSM
(p20)

FIG. 9A

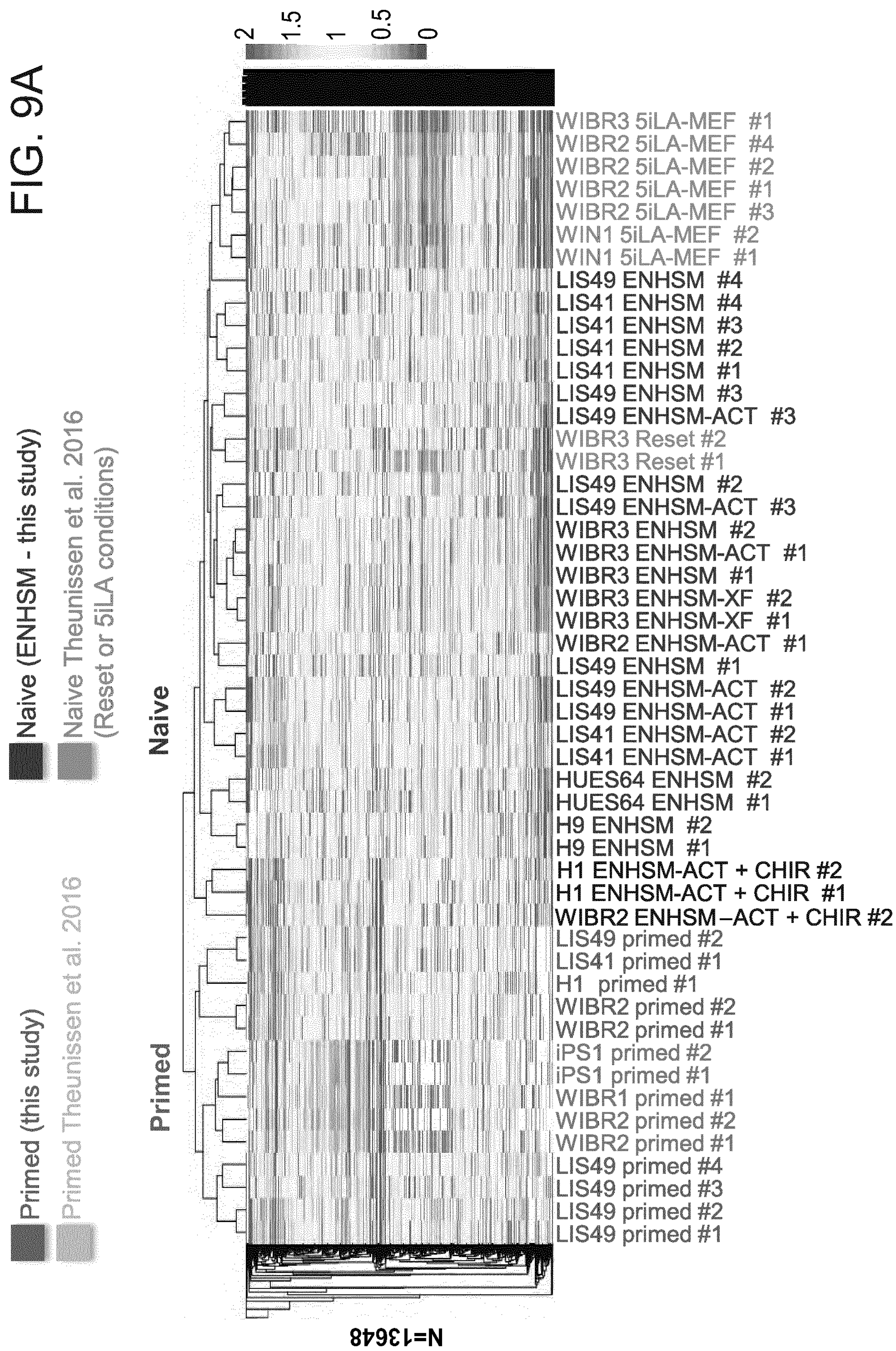
2
1.5
1
0.5
0
WIBR3 5iLA-MEF #1
WIBR2 5iLA-MEF #4
WIBR2 5iLA-MEF #2
WIBR2 5iLA-MEF #1
WIBR2 5iLA-MEF #3
WIN1 5iLA-MEF #2
WIN1 5iLA-MEF #1
LIS49 ENHSM #4
LIS41 ENHSM #4
LIS41 ENHSM #3
LIS41 ENHSM #2
LIS41 ENHSM #1
LIS49 ENHSM #3
LIS49 ENHSM-ACT #3
WIBR3 Reset #2
WIBR3 Reset #1
LIS49 ENHSM #2
LIS49 ENHSM-ACT #3
WIBR3 ENHSM #2
WIBR3 ENHSM-ACT #1
WIBR3 ENHSM #1
WIBR3 ENHSM-XF #2
WIBR3 ENHSM-XF #1
WIBR2 ENHSM-ACT #1
LIS49 ENHSM #1
LIS49 ENHSM-ACT #2
LIS49 ENHSM-ACT #1
LIS41 ENHSM-ACT #2
LIS41 ENHSM-ACT #1
HUES64 ENHSM #2
HUES64 ENHSM #1
H9 ENHSM #2
H9 ENHSM #1
H1 ENHSM-ACT + CHIR #2
H1 ENHSM-ACT + CHIR #1
WIBR2 ENHSM –ACT + CHIR #2
LIS49 primed #2
LIS41 primed #1
H1 primed #1
WIBR2 primed #2
WIBR2 primed #1
iPS1 primed #2
iPS1 primed #1
WIBR1 primed #1
WIBR2 primed #2
WIBR2 primed #1
LIS49 primed #4
LIS49 primed #3
LIS49 primed #2
LIS49 primed #1
Naive
Primed
Naive (ENHSM - this study)
Naive Theunissen et al. 2016 (Reset or 5iLA conditions)
Primed (this study)
Primed Theunissen et al. 2016
N=13648

FIG. 9B

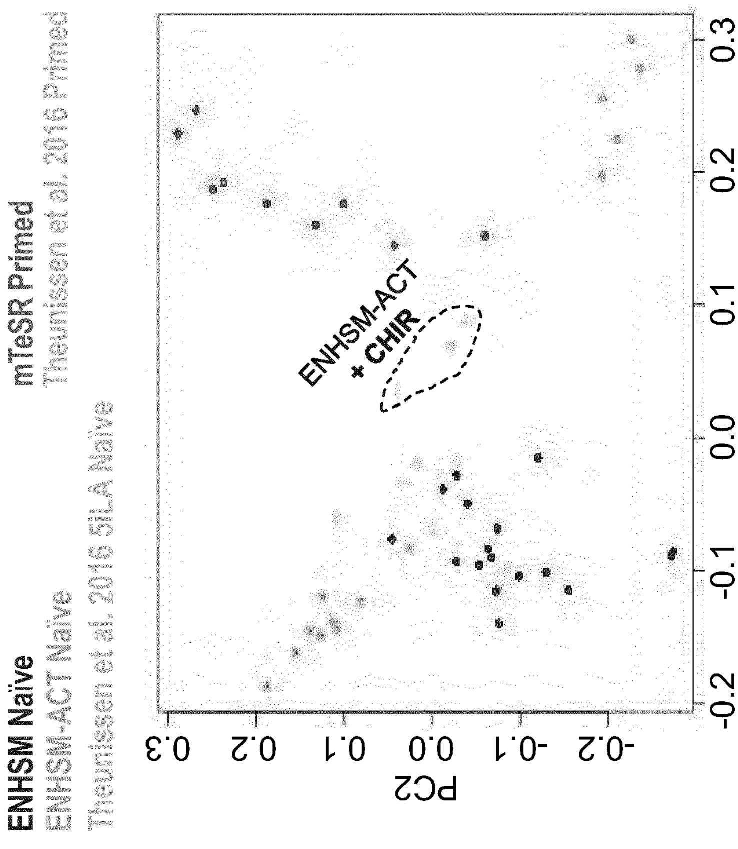


FIG. 9C

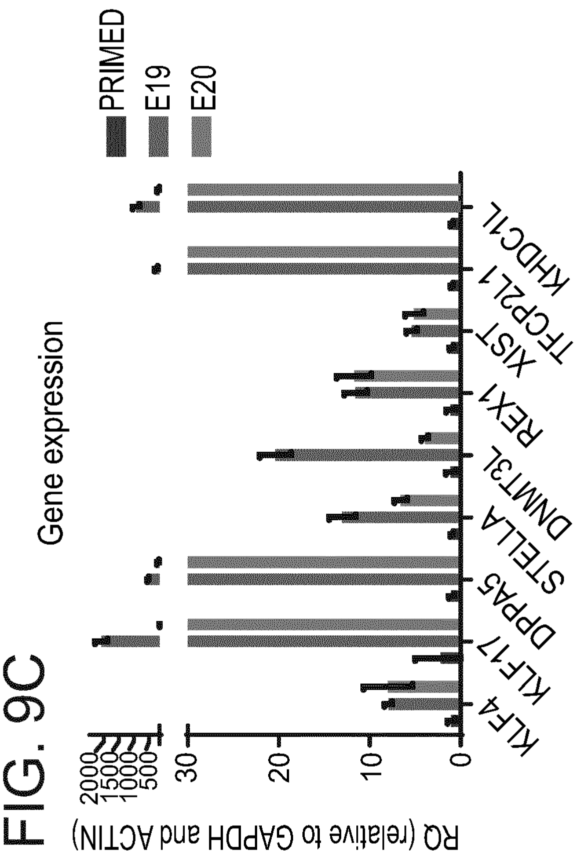


FIG. 9D

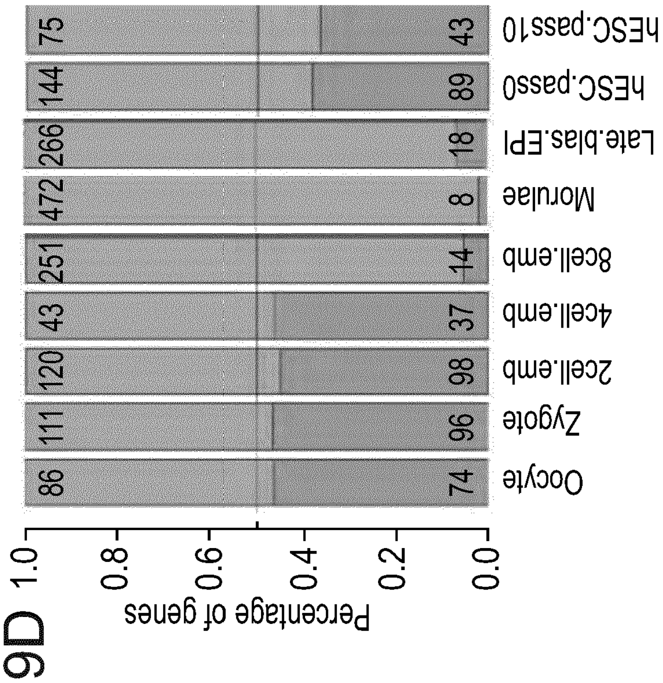

FIG. 10

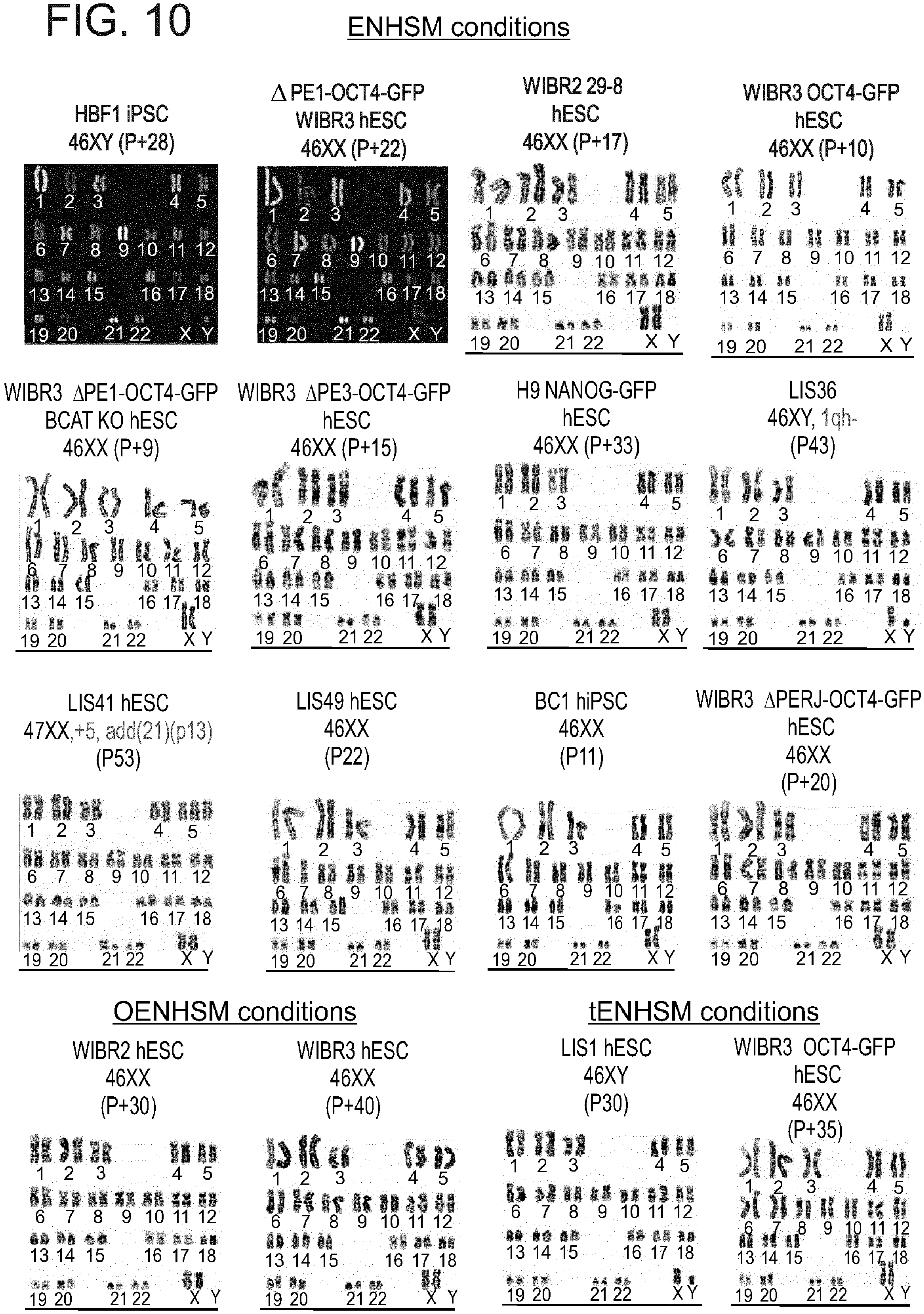
ENHSM conditions
HBF1 iPSC
46XY (P+28)
Δ PE1-OCT4-GFP
WIBR3 hESC
46XX (P+22)
WIBR2 29-8
hESC
46XX (P+17)
WIBR3 OCT4-GFP
hESC
46XX (P+10)
WIBR3 ΔPE1-OCT4-GFP
BCAT KO hESC
46XX (P+9)
WIBR3 ΔPE3-OCT4-GFP
hESC
46XX (P+15)
H9 NANOG-GFP
hESC
46XX (P+33)
LIS36
46XY, 1qh-
(P43)
LIS41 hESC
47XX,+5, add(21)(p13)
(P53)
LIS49 hESC
46XX
(P22)
BC1 hiPSC
46XX
(P11)
WIBR3 ΔPERJ-OCT4-GFP
hESC
46XX
(P+20)
OENHSM conditions
WIBR2 hESC
46XX
(P+30)
WIBR3 hESC
46XX
(P+40)
tENHSM conditions
LIS1 hESC
46XY
(P30)
WIBR3 OCT4-GFP
hESC
46XX
(P+35)

FIG. 11A

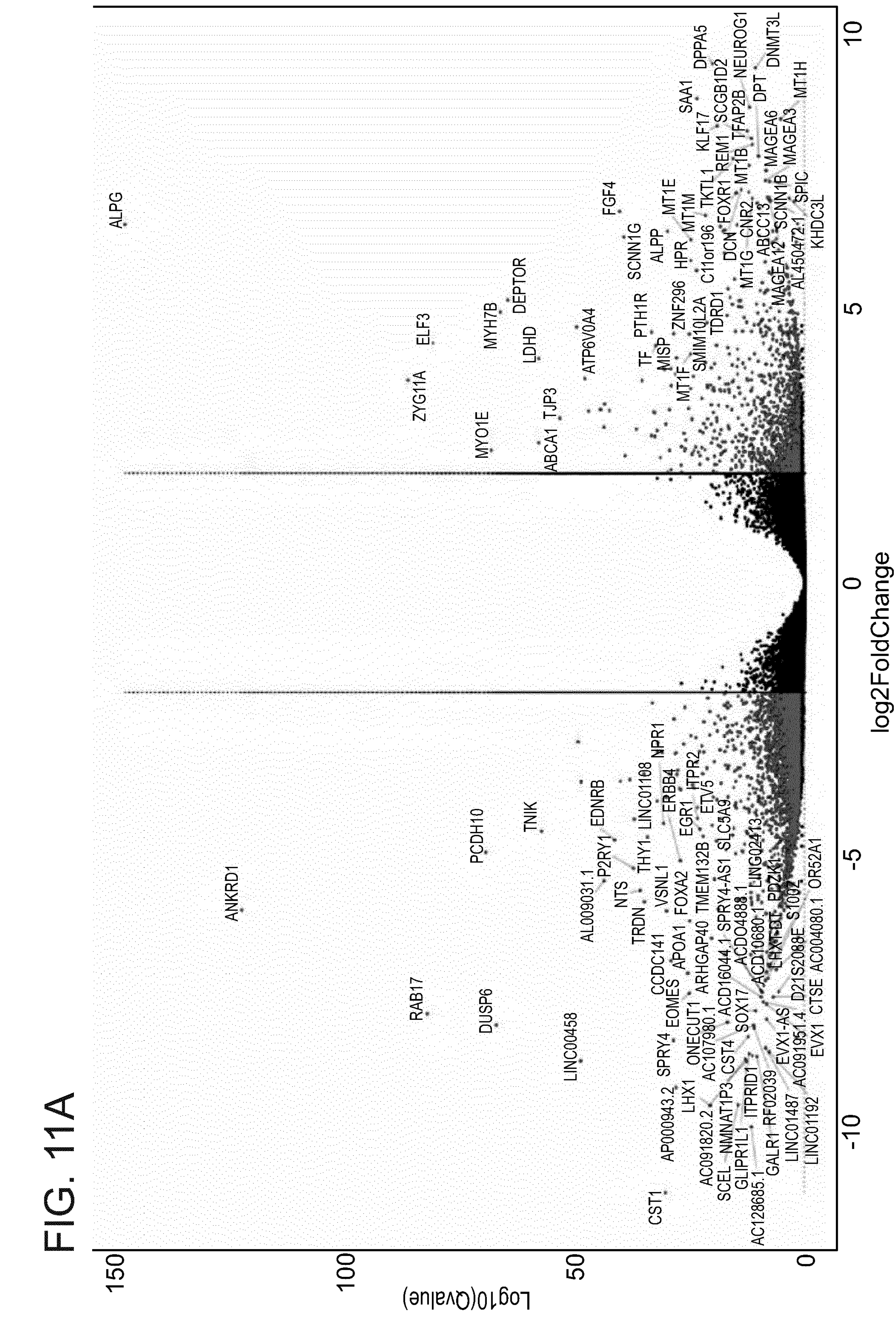
Log10(Qvalue)
150
100
50
0
log2FoldChange
-10
-5
0
5
10
ALPG
ANKRD1
RAB17
DUSP6
PCDH10
TNIK
LINC00458
EDNRB
ELF3
ZYG11A
MYH7B
DEPTOR
LDHD
MYO1E
ABCA1
TJP3
ATP6V0A4
FGF4
SCNN1G
PTH1R
TF
MISP
ALPP
MT1E
MT1M
HPR
ZNF296
SMIM10L2A
TDRD1
C11orf196
TKTL1
KLF17
SAA1
DPPA5
SCGB1D2
REM1
FOXR1
DCN
MT1G
CNR2
MT1B
TFAP2B
NEUROG1
DPT
DNMT3L
MAGEA6
MAGEA3
MT1H
SCNN1B
ABCC13
MAGEA12
SPIC
KHDC3L
CST1
AP000943.2
SPRY4
EOMES
CCDC141
LHX1
ONECUT1
APOA1
TRDN
NTS
AL009031.1
P2RY1
THY1
VSNL1
FOXA2
LINC01108
NPR1
ERBB4
EGR1
ITPR2
ETV5
TMEM132B
SLC5A9
ARHGAP40
CST4
SOX17
GALR1
RF02039
LINC01487
LINC01192
EVX1
CTSE
OR52A1
AC128685.1

FIG. 11B

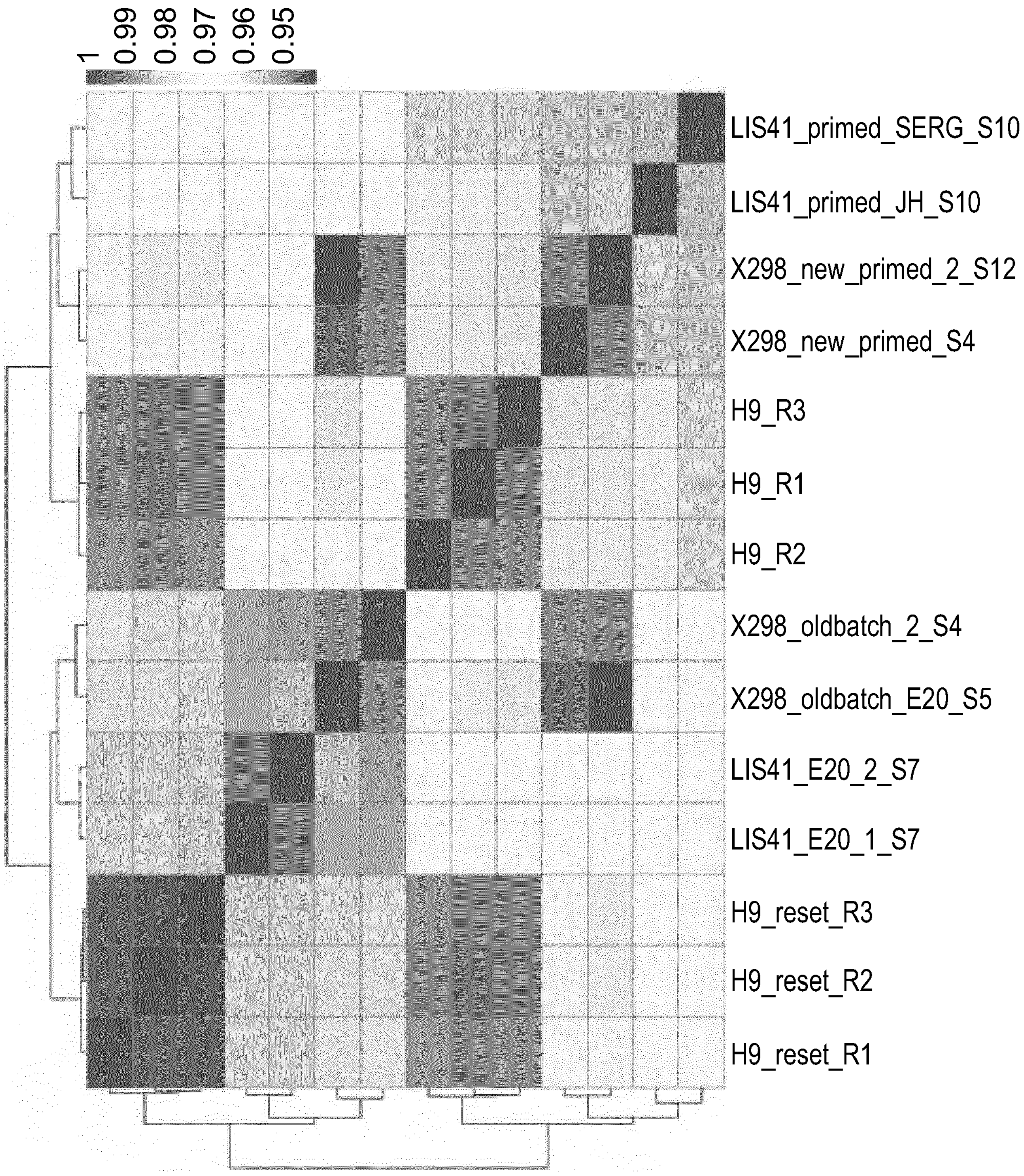
1
0.99
0.98
0.97
0.96
0.95
LIS41_primed_SERG_S10
LIS41_primed_JH_S10
X298_new_primed_2_S12
X298_new_primed_S4
H9_R3
H9_R1
H9_R2
X298_oldbatch_2_S4
X298_oldbatch_E20_S5
LIS41_E20_2_S7
LIS41_E20_1_S7
H9_reset_R3
H9_reset_R2
H9_reset_R1

FIG. 11C

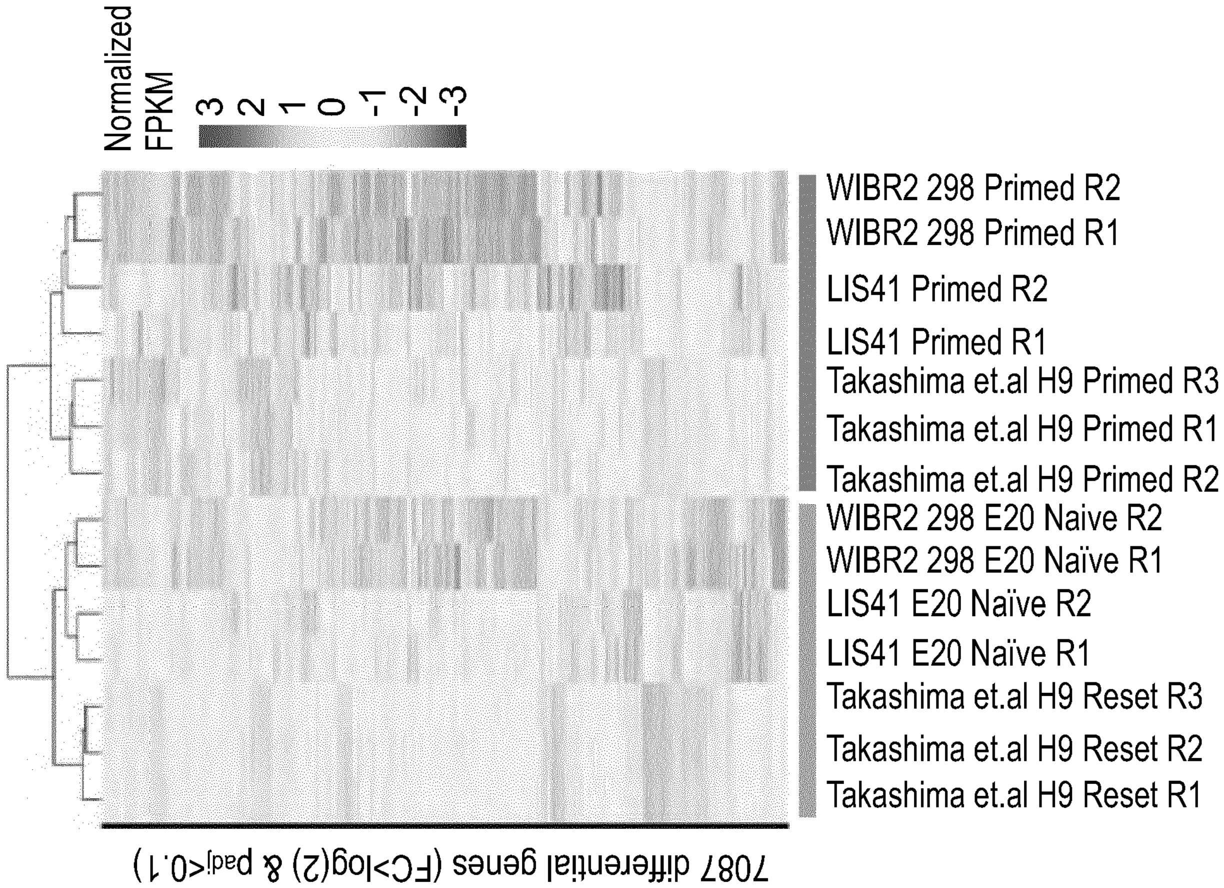


FIG. 11D

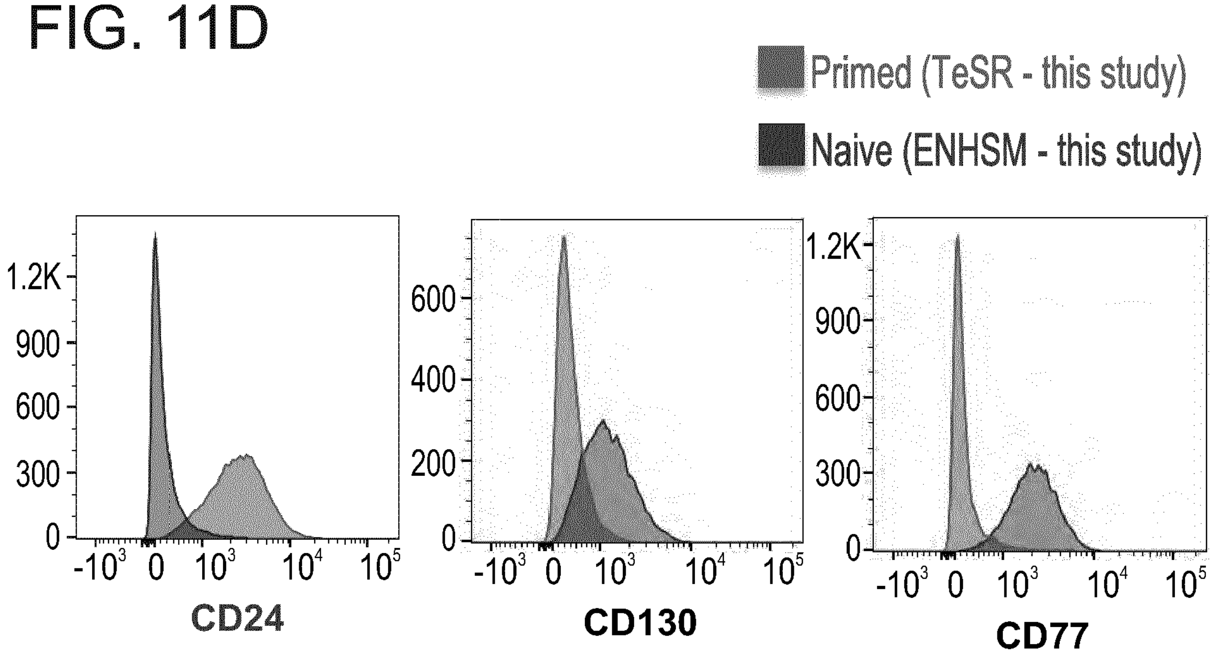

FIG. 12A

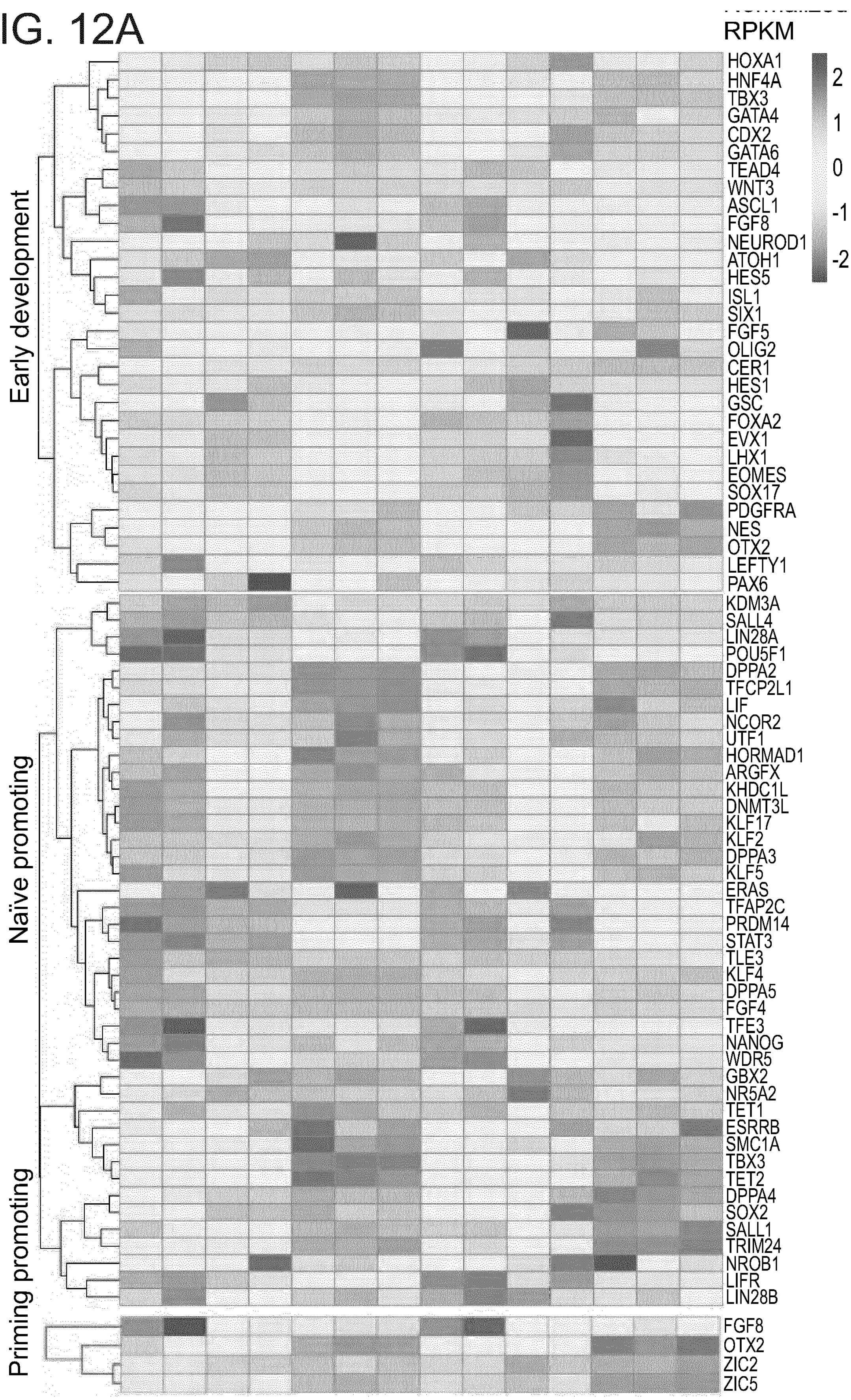
Normalized
RPKM
2
1
0
-1
-2
Early development
Naïve promoting
Priming promoting
HOXA1
HNF4A
TBX3
GATA4
CDX2
GATA6
TEAD4
WNT3
ASCL1
FGF8
NEUROD1
ATOH1
HES5
ISL1
SIX1
FGF5
OLIG2
CER1
HES1
GSC
FOXA2
EVX1
LHX1
EOMES
SOX17
PDGFRA
NES
OTX2
LEFTY1
PAX6
KDM3A
SALL4
LIN28A
POU5F1
DPPA2
TFCP2L1
LIF
NCOR2
UTF1
HORMAD1
ARGFX
KHDC1L
DNMT3L
KLF17
KLF2
DPPA3
KLF5
ERAS
TFAP2C
PRDM14
STAT3
TLE3
KLF4
DPPA5
FGF4
TFE3
NANOG
WDR5
GBX2
NR5A2
TET1
ESRRB
SMC1A
TBX3
TET2
DPPA4
SOX2
SALL1
TRIM24
NROB1
LIFR
LIN28B
FGF8
OTX2
ZIC2
ZIC5

FIG. 12A continued

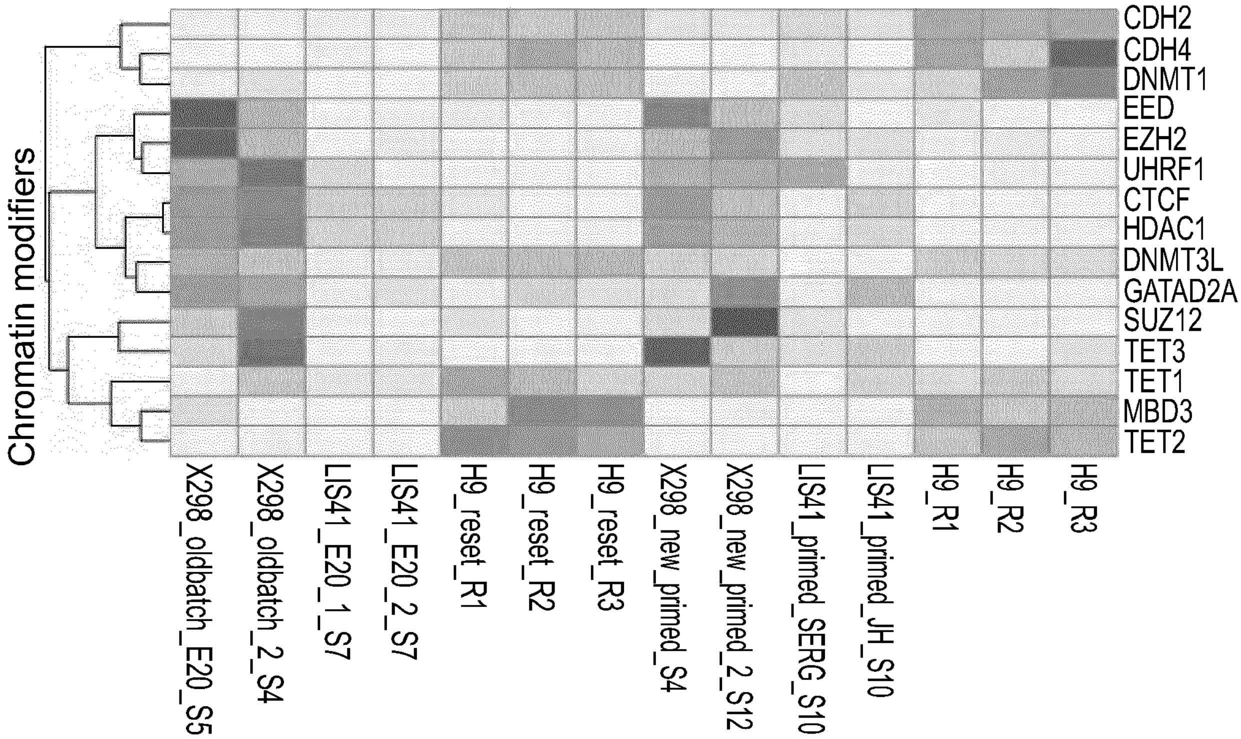


FIG. 12B

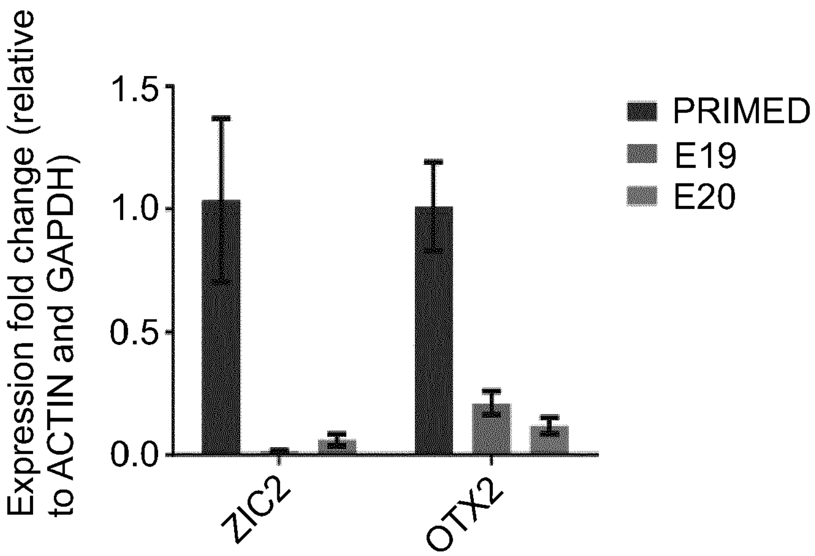

FIG. 13A

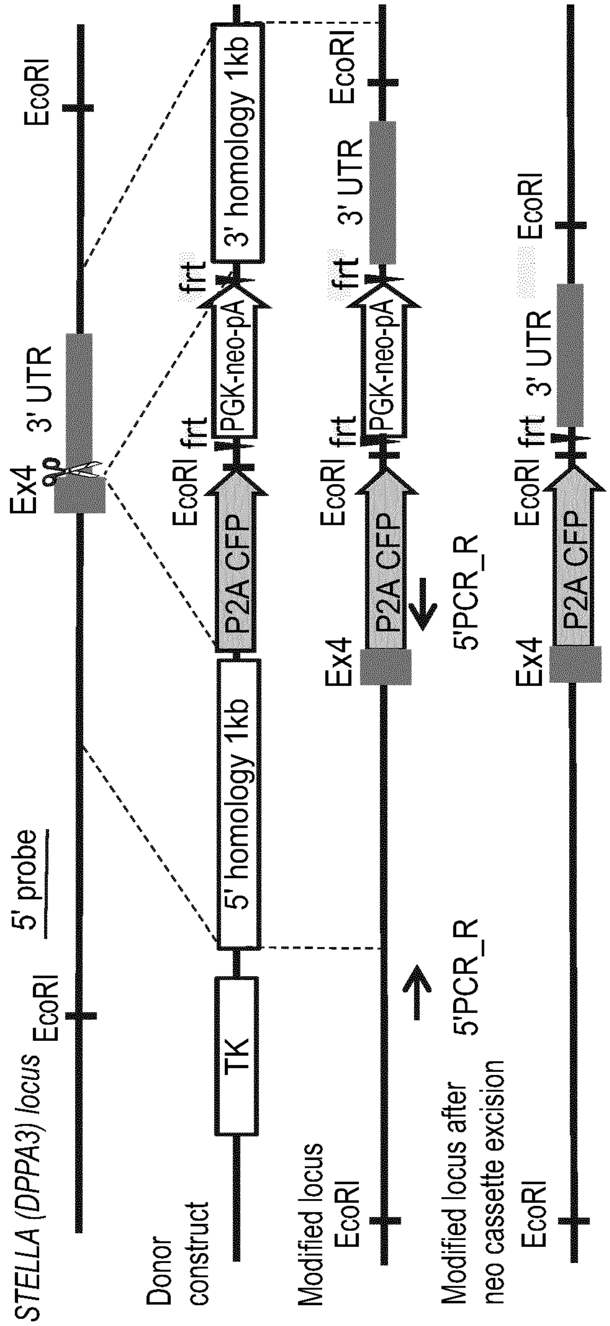


FIG. 13B

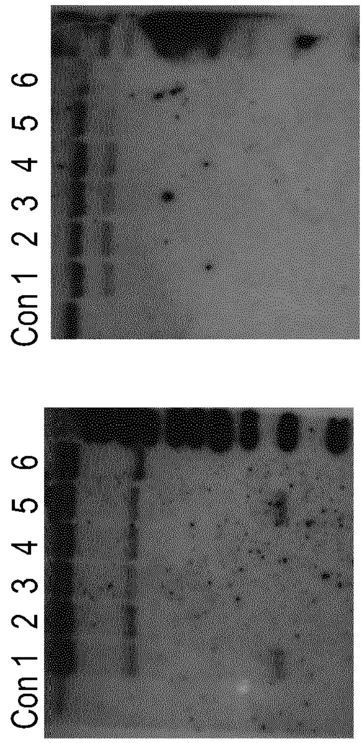


FIG. 13C

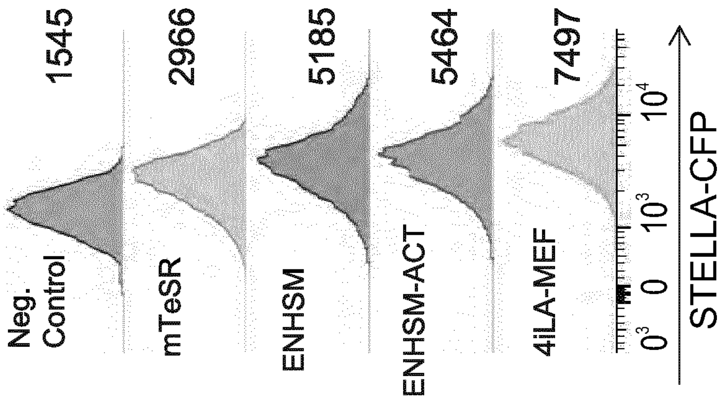

FIG. 13D

**TFAP2C knockout in WIBR3-ΔPE-O4G hESC:**

sgRNA sequences:
sgRNA #1
CGTTGTAAGCAAAGAGTGCG
sgRNA #2
CTACCACAAATGTCCCACGC

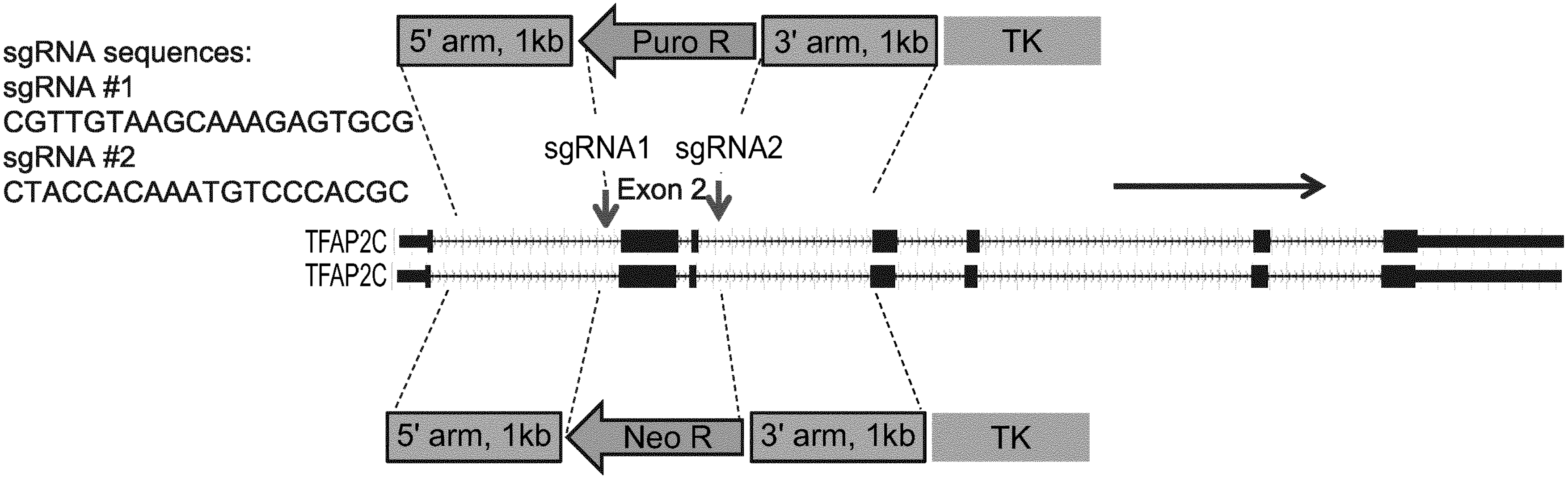


FIG. 13E

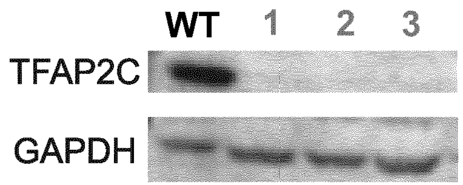

FIG. 14A

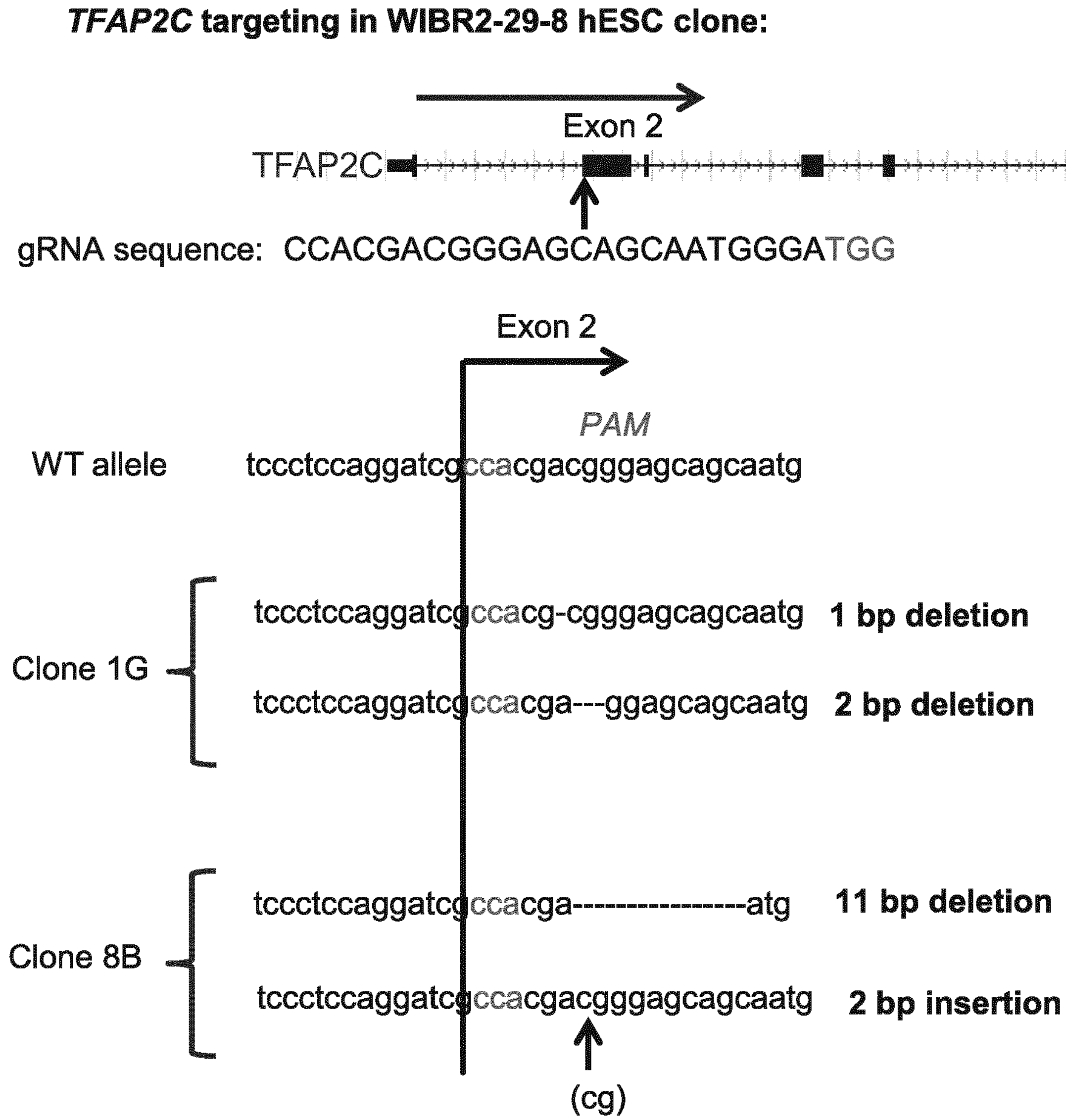


FIG. 14B

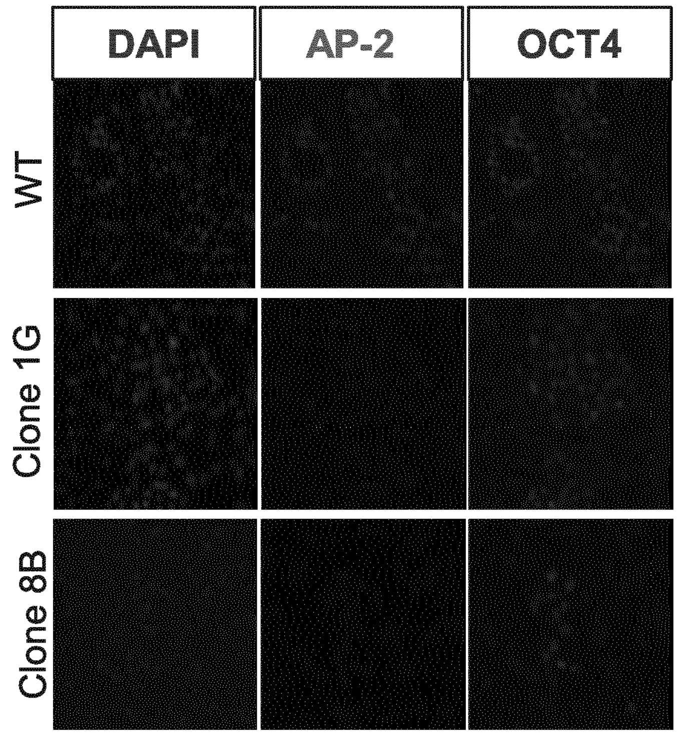

FIG. 15A

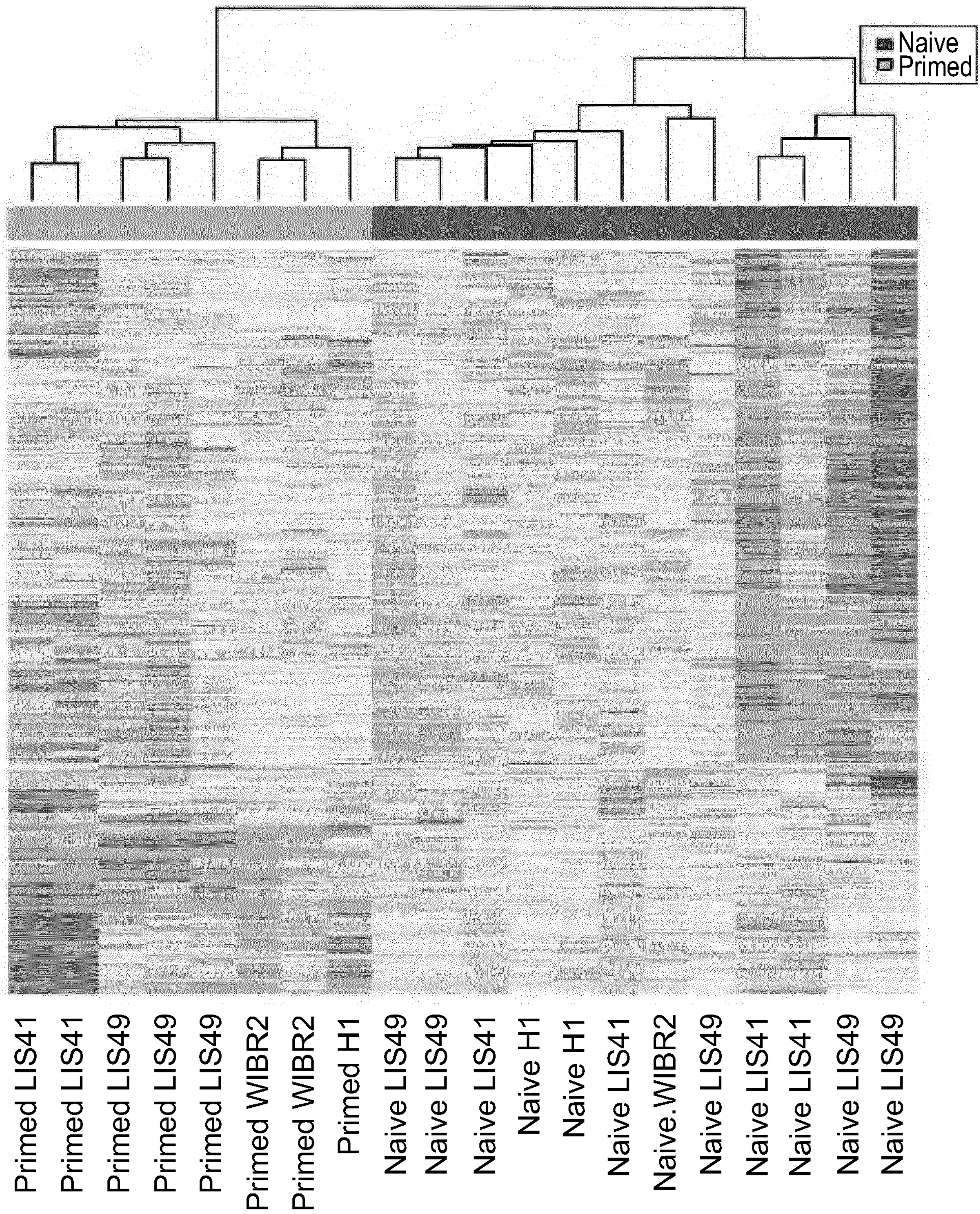
Naive
Primed
Primed LIS41
Primed LIS41
Primed LIS49
Primed LIS49
Primed LIS49
Primed WIBR2
Primed WIBR2
Primed H1
Naive LIS49
Naive LIS49
Naive LIS41
Naive H1
Naive H1
Naive LIS41
Naive.WIBR2
Naive LIS49
Naive LIS41
Naive LIS41
Naive LIS49
Naive LIS49

FIG. 15B

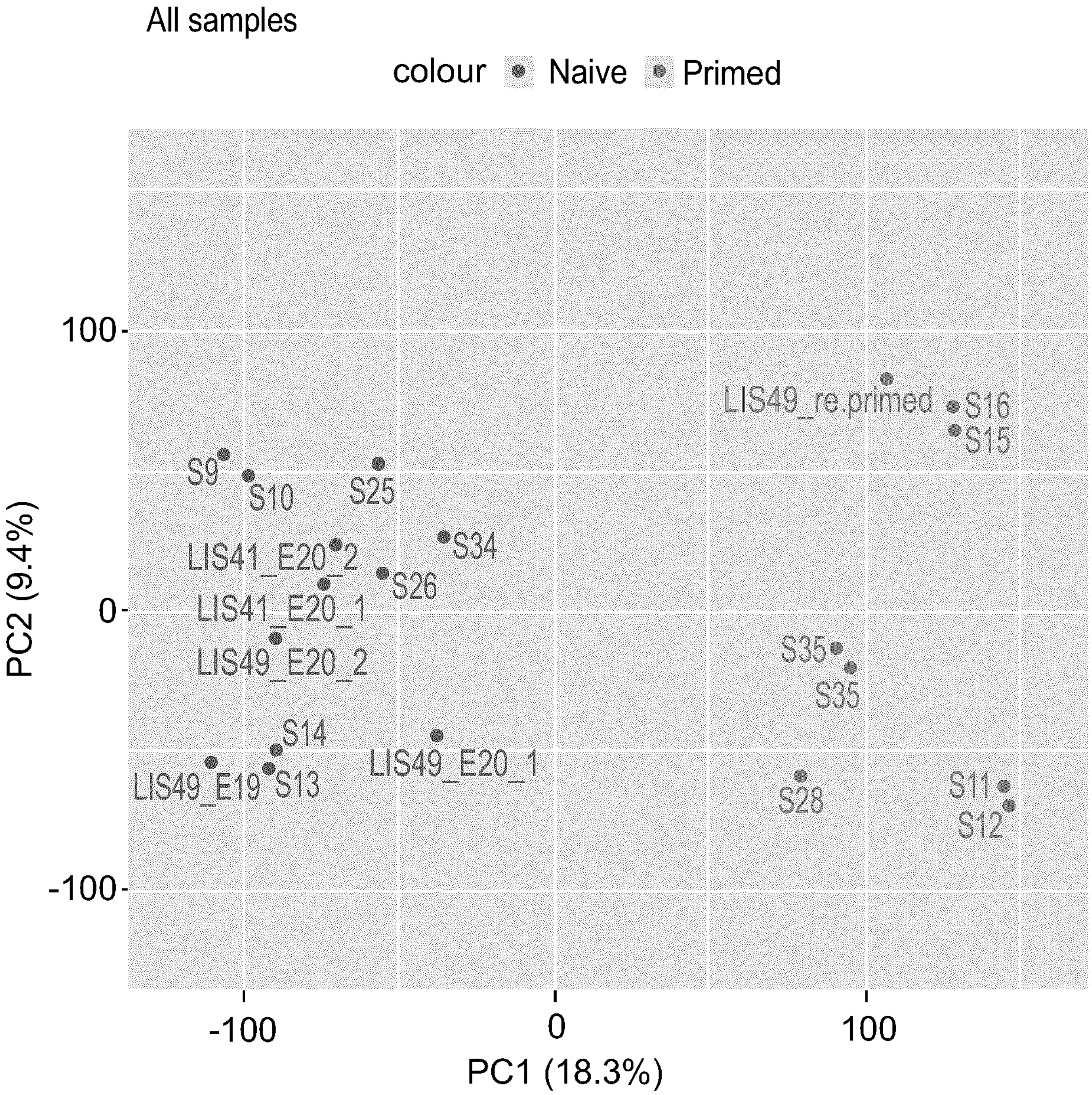
All samples
colour
Naive
Primed
100
0
-100
-100
0
100
PC2 (9.4%)
PC1 (18.3%)
S9
S10
S25
LIS41_E20_2
S34
S26
LIS41_E20_1
LIS49_E20_2
S14
LIS49_E19
S13
LIS49_E20_1
LIS49_re.primed
S16
S15
S35
S35
S28
S11
S12

FIG. 16

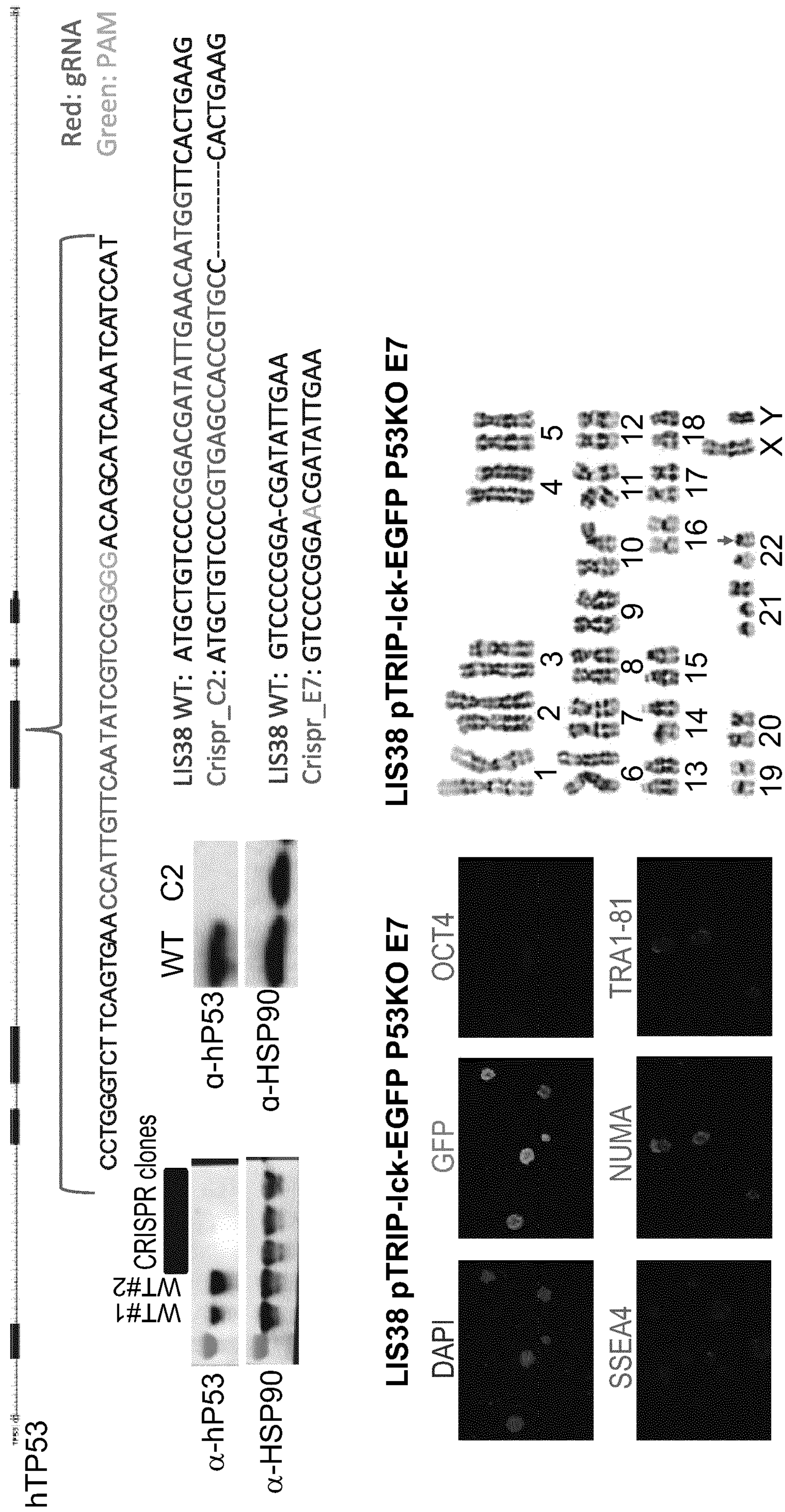
hTP53
hTP53 targeting scheme
Exon 4
CCTGGGTCTTCAGTGAACCATTGTTCAATATCGTCCGGGGACAGCATCAAATCATCCAT
Red: gRNA
Green: PAM
LIS38 WT: ATGCTGTCCCCGGACGATATTGAACAATGGTTCACTGAAG
Crispr_C2: ATGCTGTCCCCGTGAGCCACCGTGCC----------CACTGAAG
LIS38 WT: GTCCCCGGA-CGATATTGAA
Crispr_E7: GTCCCCGGAACGATATTGAA
WT#1
WT#2
CRISPR clones
α-hP53
α-HSP90
WT
C2
LIS38 pTRIP-lck-EGFP P53KO E7
DAPI
GFP
OCT4
SSEA4
NUMA
TRA1-81
1
2
3
4
5
6
7
8
9
10
11
12
13
14
15
16
17
18
19
20
21
22
X
Y

FIG. 16 continued

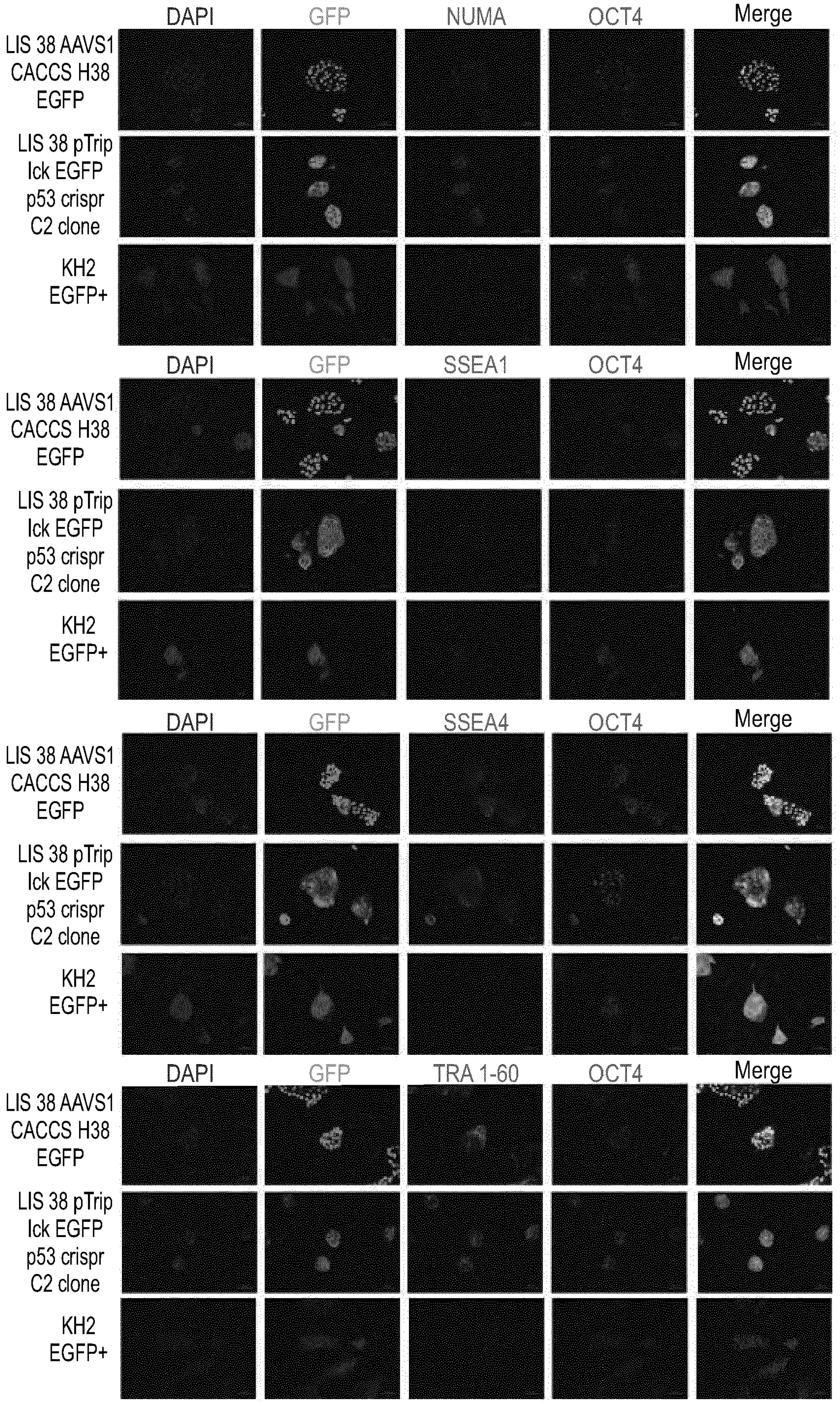

FIG. 17A

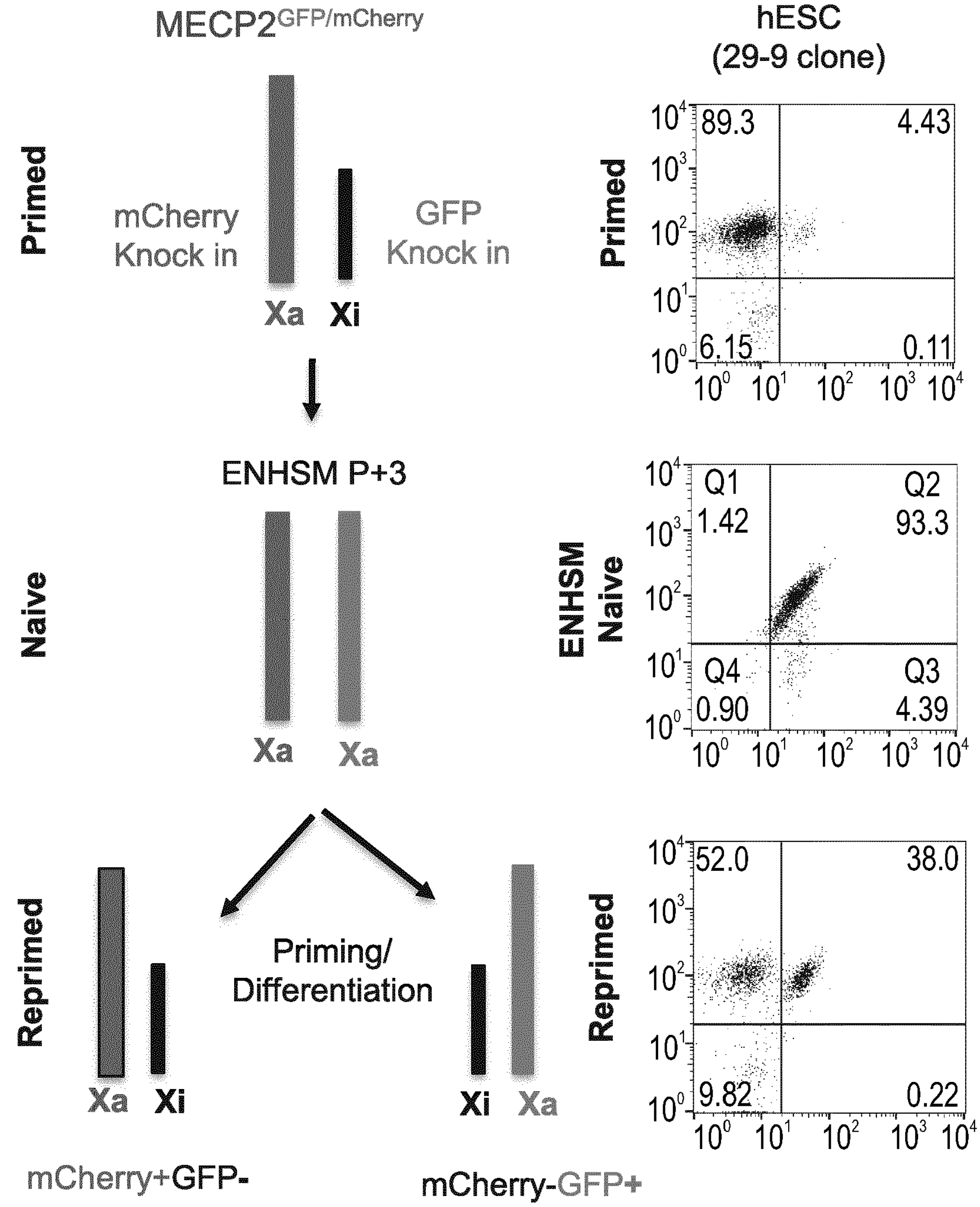


FIG. 17B

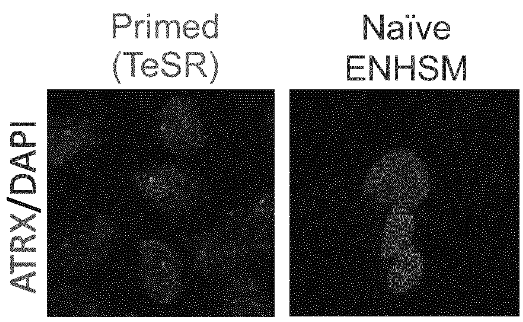


FIG. 17C

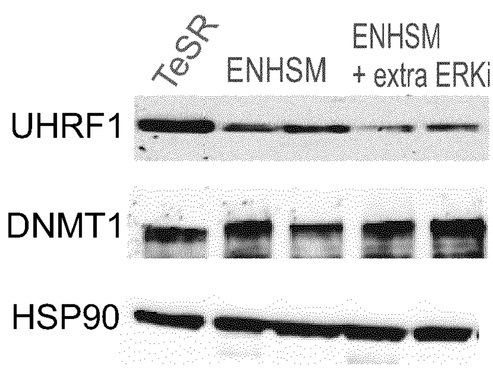

FIG. 17D

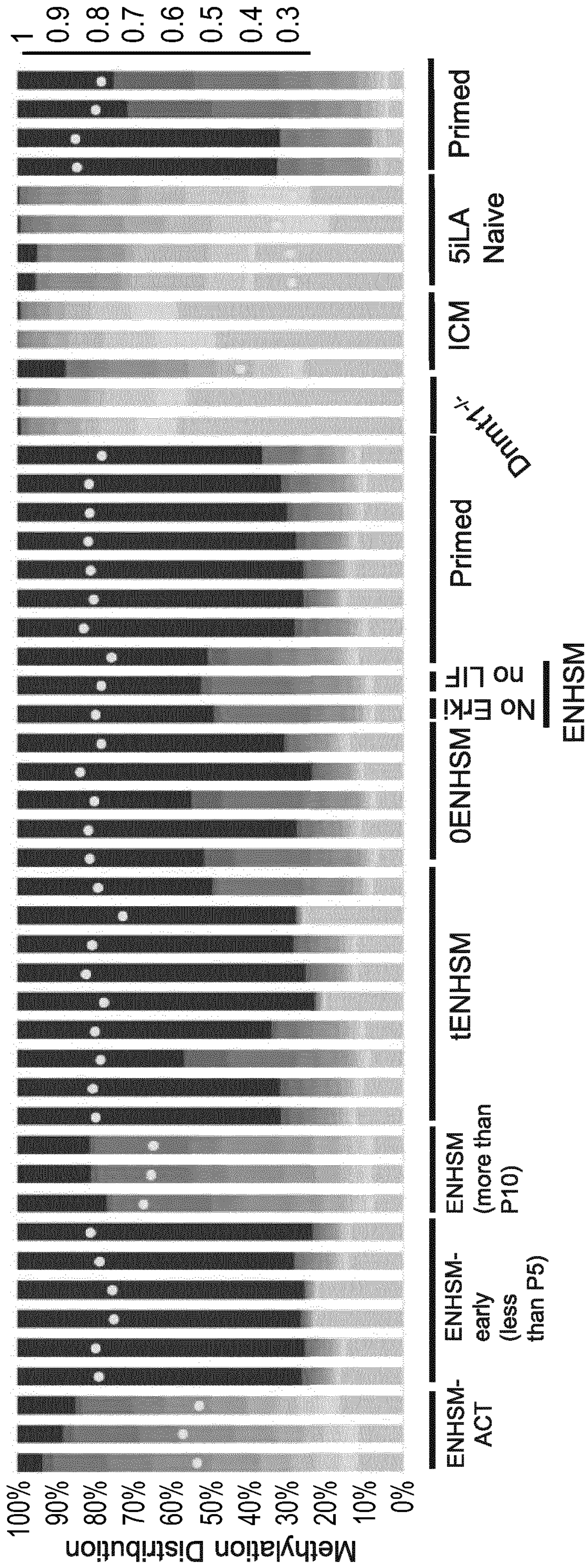
Methylation Distribution
100%
90%
80%
70%
60%
50%
40%
30%
20%
10%
0%
1
0.9
0.8
0.7
0.6
0.5
0.4
0.3
ENHSM-ACT
ENHSM-early (less than P5)
ENHSM (more than P10)
tENHSM
0ENHSM
No ERKi
no LIF
ENHSM
Primed
Dnmt1-/-
ICM
5iLA Naive
Primed

FIG. 18

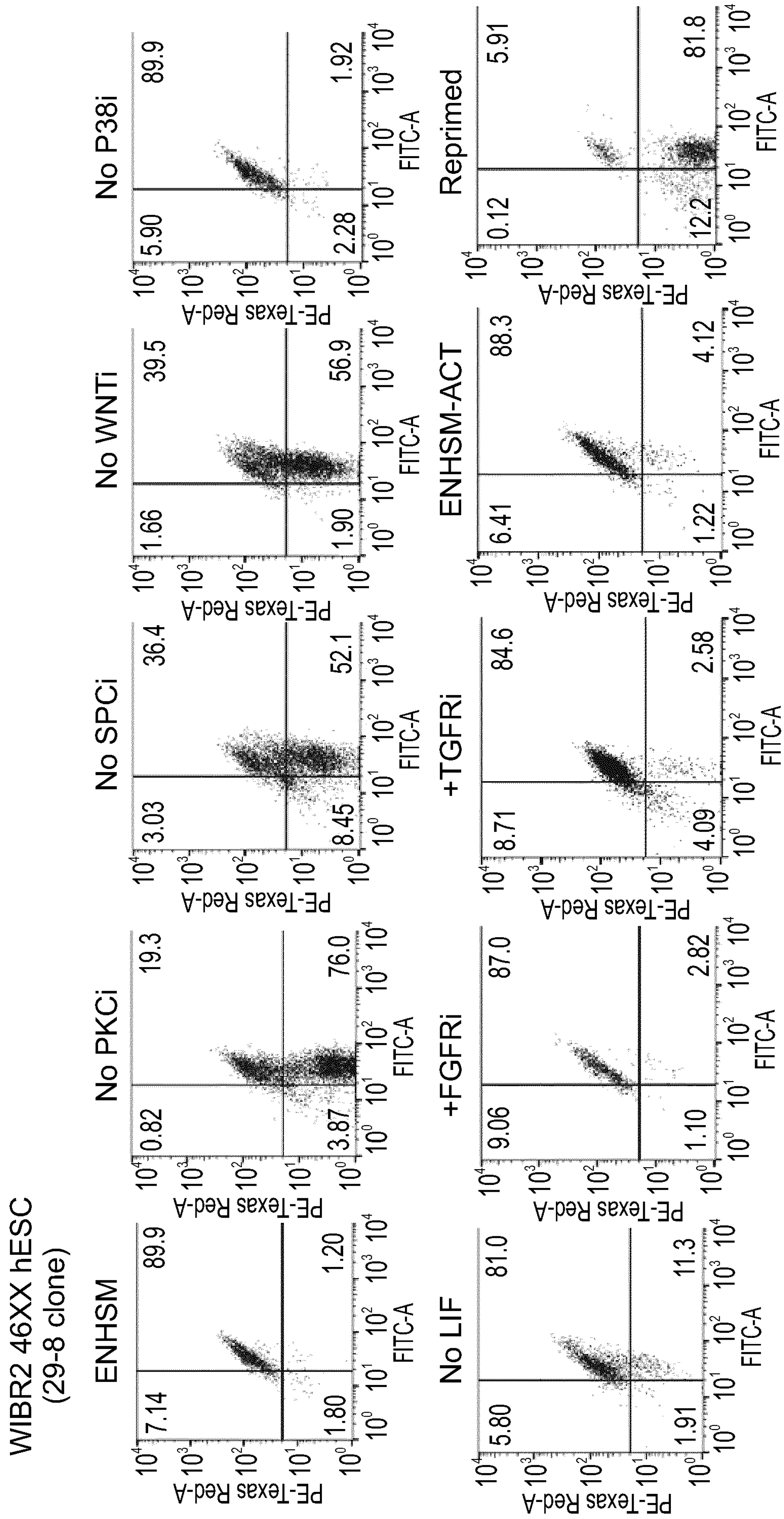
WIBR2 46XX hESC
(29-8 clone)
ENHSM
7.14
89.9
1.80
1.20
No PKCi
0.82
19.3
3.87
76.0
No SPCi
3.03
36.4
8.45
52.1
No WNTi
1.66
39.5
1.90
56.9
No P38i
5.90
89.9
2.28
1.92
No LIF
5.80
81.0
1.91
11.3
+FGFRi
9.06
87.0
1.10
2.82
+TGFRi
8.71
84.6
4.09
2.58
ENHSM-ACT
6.41
88.3
1.22
4.12
Reprimed
0.12
5.91
12.2
81.8
PE-Texas Red-A
FITC-A

FIG. 19A

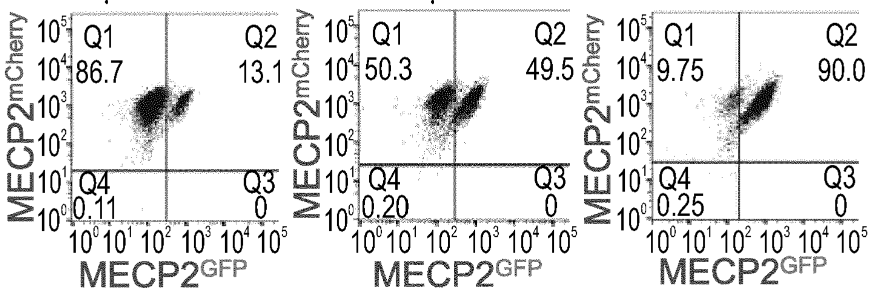


FIG. 19C

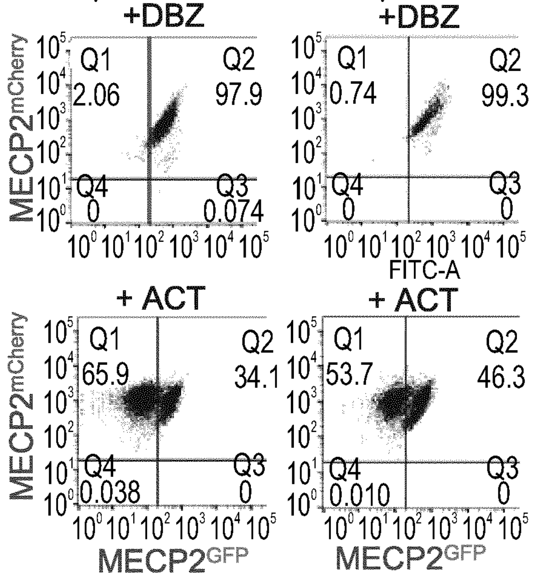


FIG. 19B

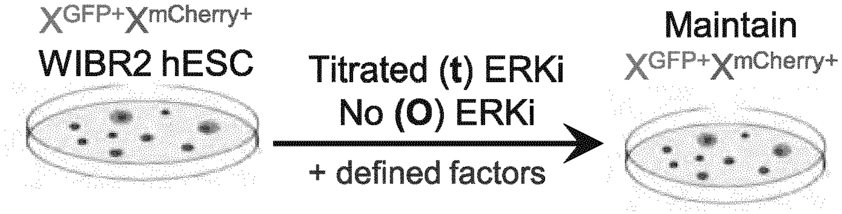


**ENHSM**

LIF (20ng/ml)
MEKi (PD0325901 1μM)
P38i/JNKi (BIRB0796 0.9μM)
PKCi (Go6983 2μM)
ROCKi (Y27632 1.2μM)
TNKi (XAV939 3μM)
SRCi (CGP77675 1.2μM)

**Factors tested**

| | |
|---|---|
| BMP4 | SCF |
| BMPRi | SRCi |
| IGF1 | ACTIVIN |
| Forskolin | NOTCHi |
| FGFRi | GSK3i |
| TGFRi | ERK5i |
| Kenpaullone | SHHi |
| DOT1Li | G9ai |

FIG. 19D

**tENHSM**

LIF (20ng/ml)
MEKi (PD0325901 0.3μM)
P38i/JNKi (BIRB0796 0.9μM)
PKCi (Go6983 2μM)
ROCKi (Y27632 1.2μM)
TNKi (XAV939 3μM)
SRCi (CGP77675 1.2μM)
NOTCHi (DBZ 0.25μM)

**0ENHSM**

LIF (20ng/ml)
P38i/JNKi (BIRB0796 0.9μM)
PKCi (Go6983 2μM)
ROCKi (Y27632 1.2μM)
TNKi (XAV939 3μM)
SRCi (CGP77675 1.2μM)
NOTCHi (DBZ 0.25μM)

FIG. 19E

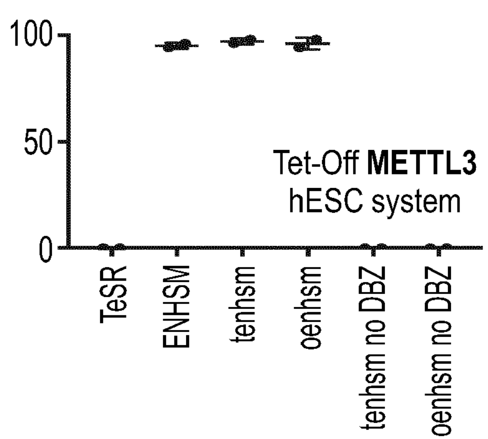


FIG. 19F

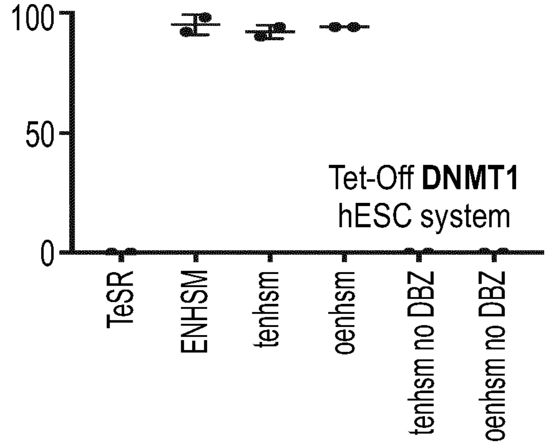


FIG. 19G

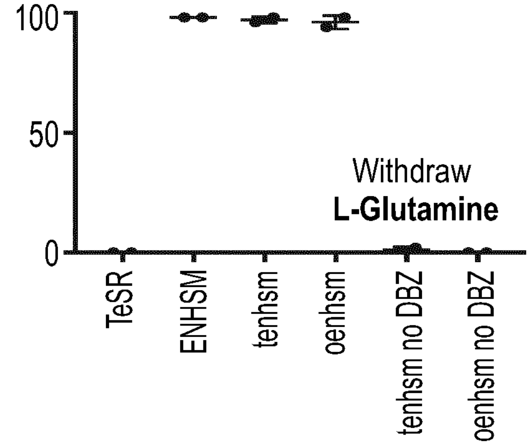


FIG. 19H

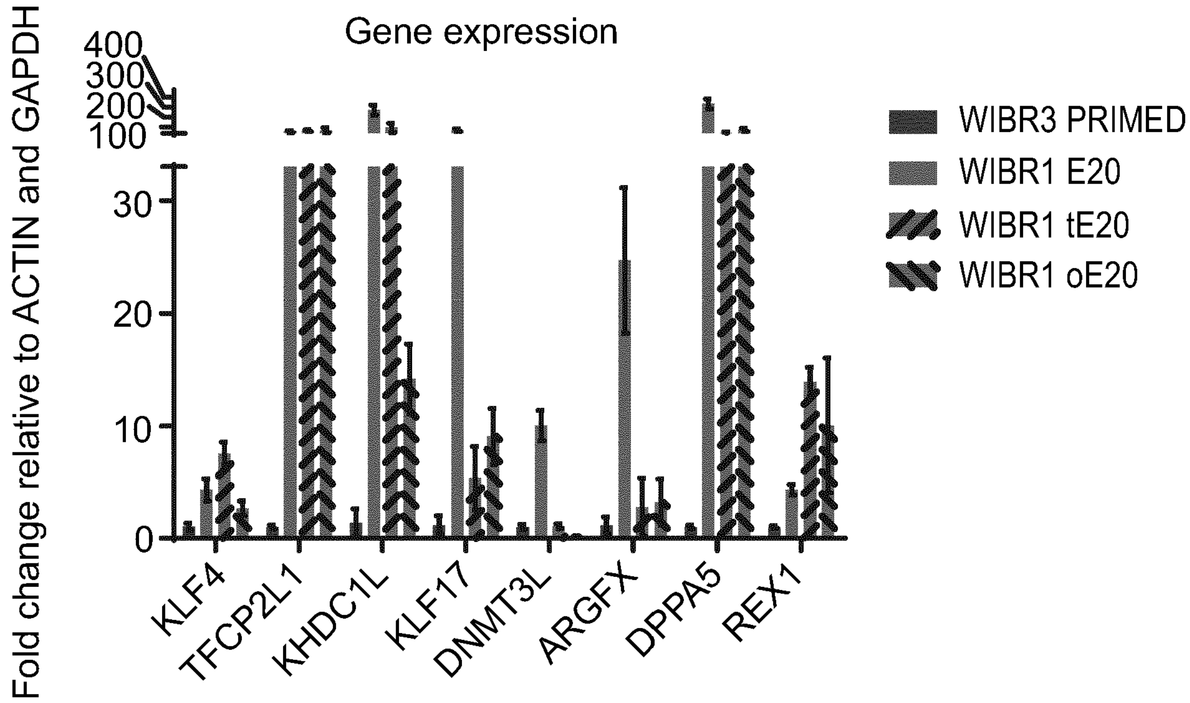

FIG. 20A

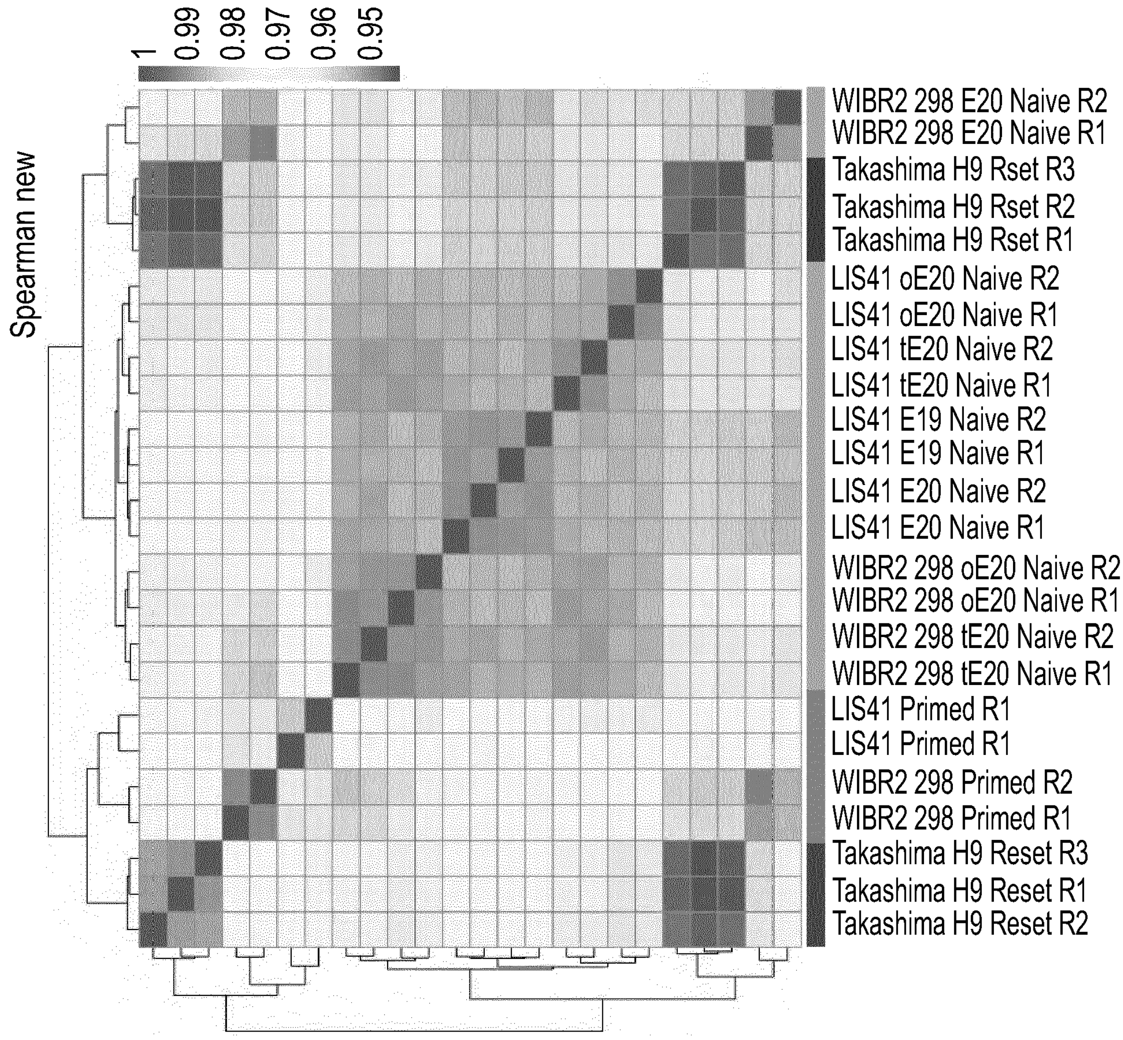


FIG. 20B

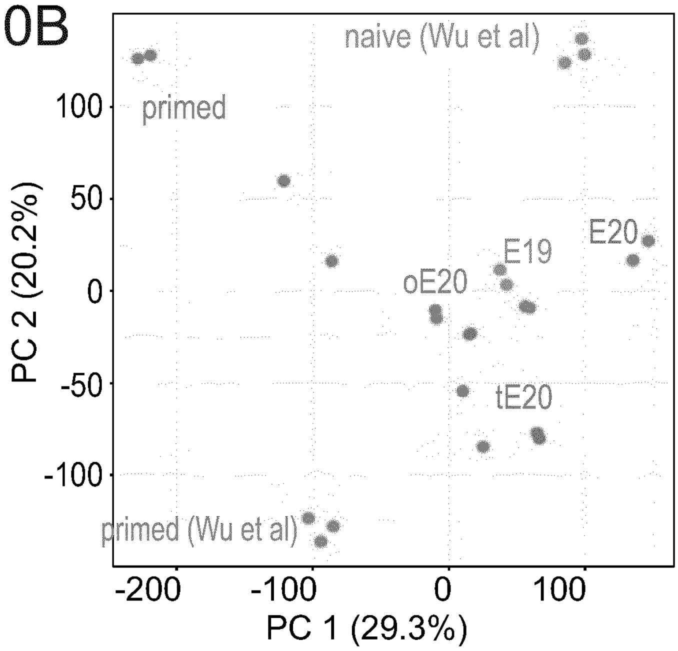

FIG. 21

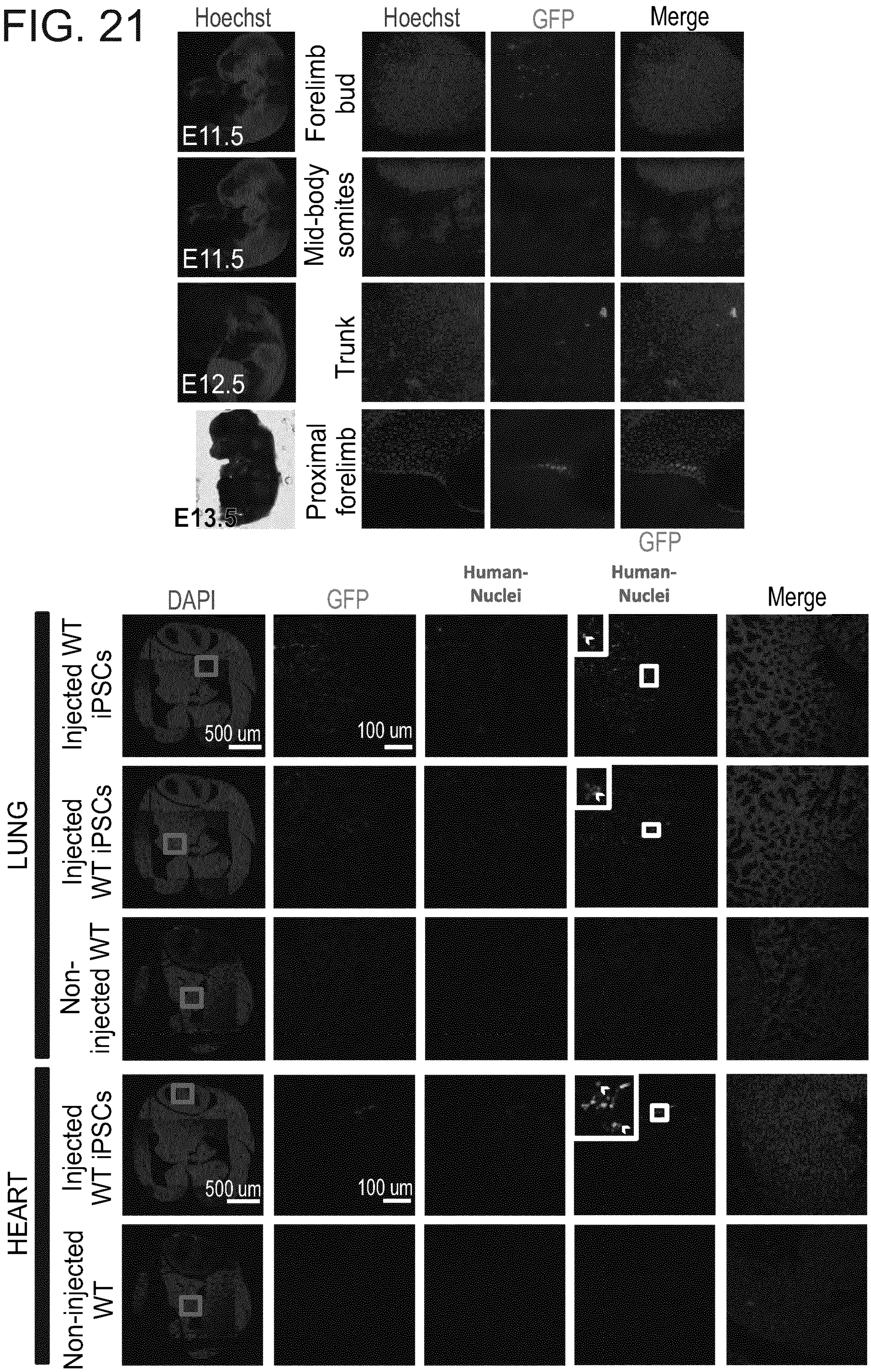
Hoechst
Hoechst
GFP
Merge
E11.5
Forelimb bud
E11.5
Mid-body somites
E12.5
Trunk
E13.5
Proximal forelimb
DAPI
GFP
Human-Nuclei
GFP Human-Nuclei
Merge
LUNG
Injected WT iPSCs
500 um
100 um
Injected WT iPSCs
Non-injected WT
HEART
Injected WT iPSCs
500 um
100 um
Non-injected WT

FIG. 22

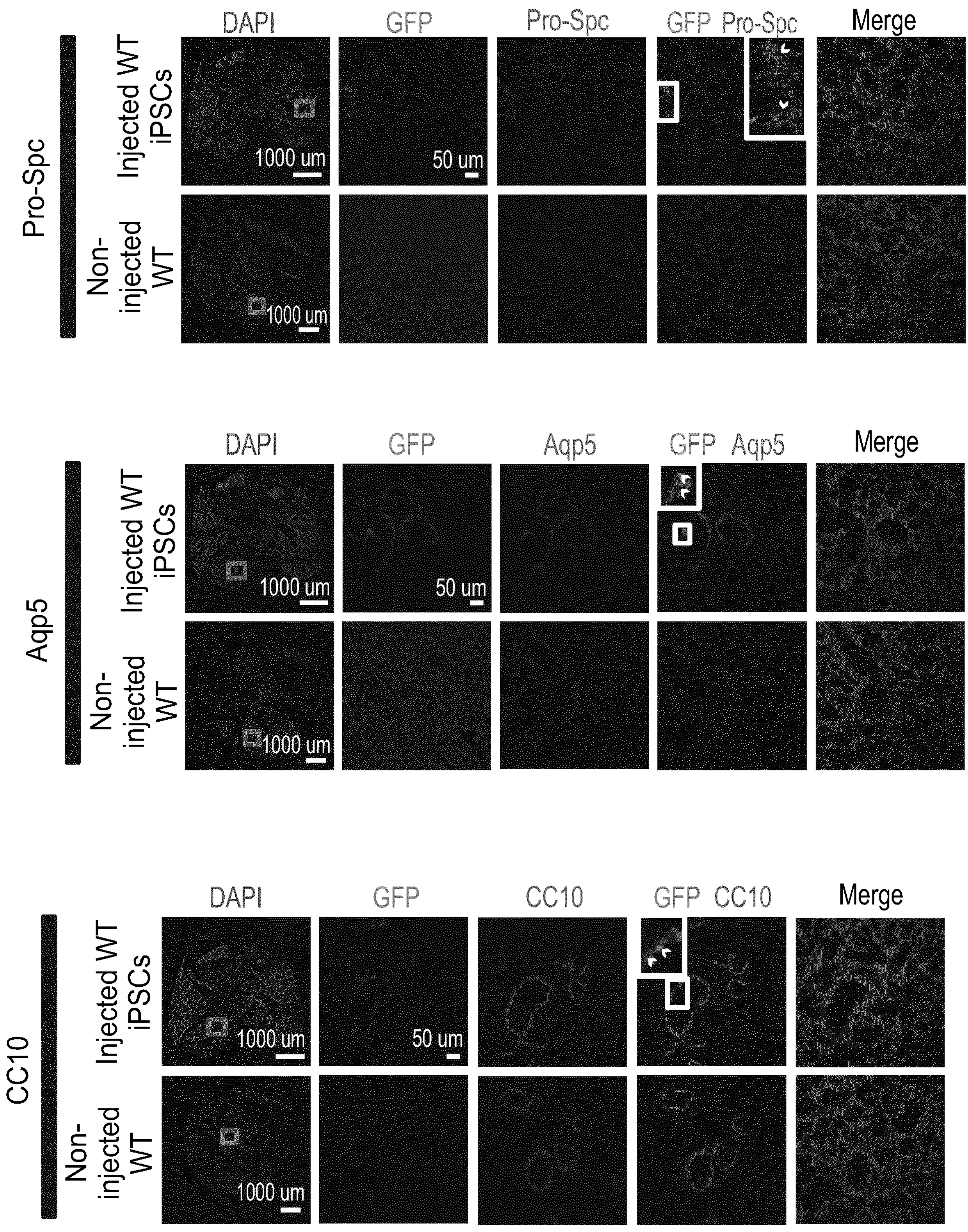

DAPI
GFP
Pro-Spc
GFP Pro-Spc
Merge
Pro-Spc
Injected WT iPSCs
Non-injected WT
1000 um
50 um
1000 um
DAPI
GFP
Aqp5
GFP Aqp5
Merge
Aqp5
Injected WT iPSCs
Non-injected WT
1000 um
50 um
1000 um
DAPI
GFP
CC10
GFP CC10
Merge
CC10
Injected WT iPSCs
Non-injected WT
1000 um
50 um
1000 um

FIG. 23

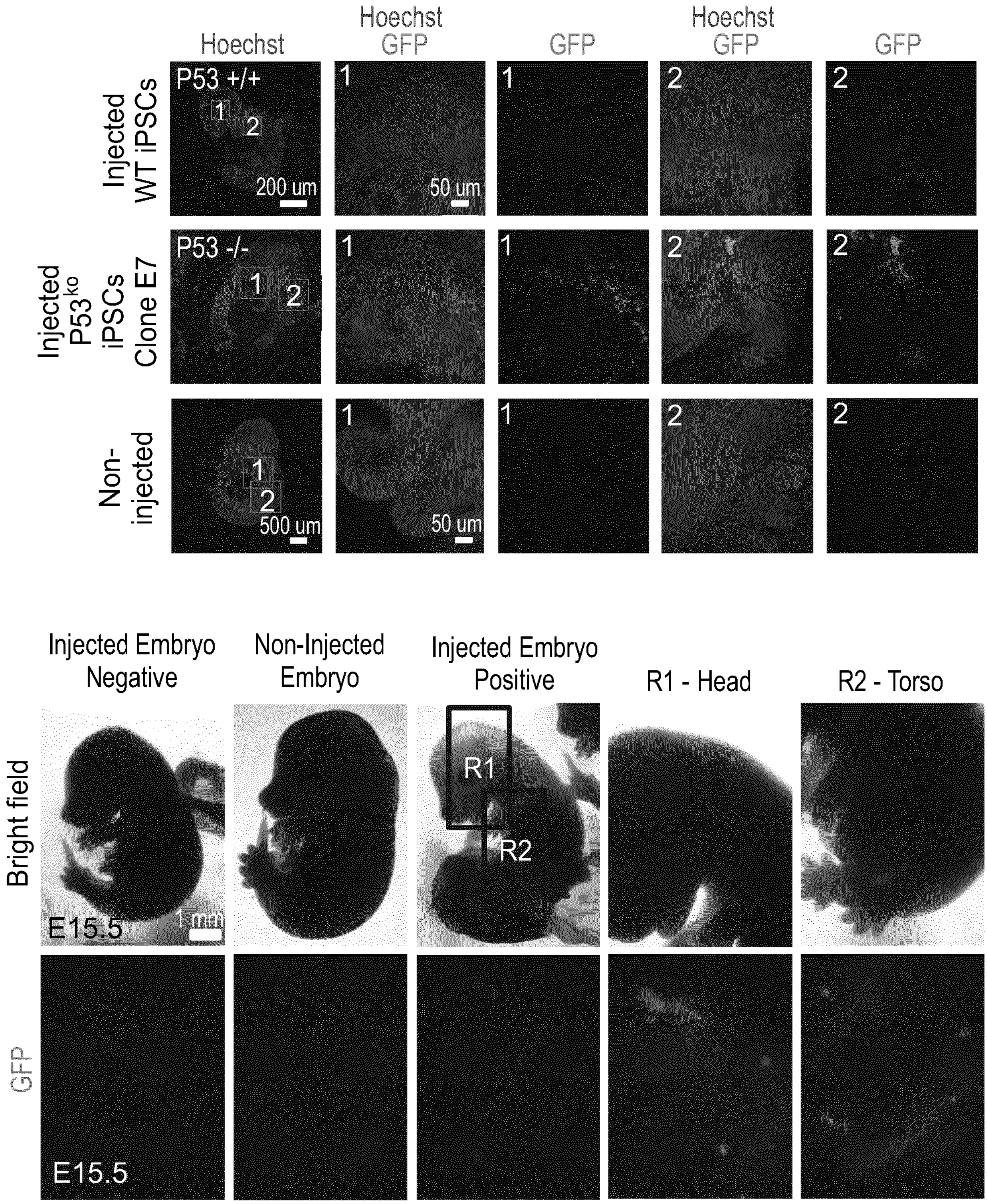
Hoechst
Hoechst GFP
GFP
Hoechst GFP
GFP
Injected WT iPSCs
P53 +/+
200 um
50 um
Injected P53ko iPSCs Clone E7
P53 -/-
Non-injected
500 um
50 um
Injected Embryo Negative
Non-Injected Embryo
Injected Embryo Positive
R1 - Head
R2 - Torso
Bright field
GFP
R1
R2
E15.5
1 mm
E15.5

FIG. 24

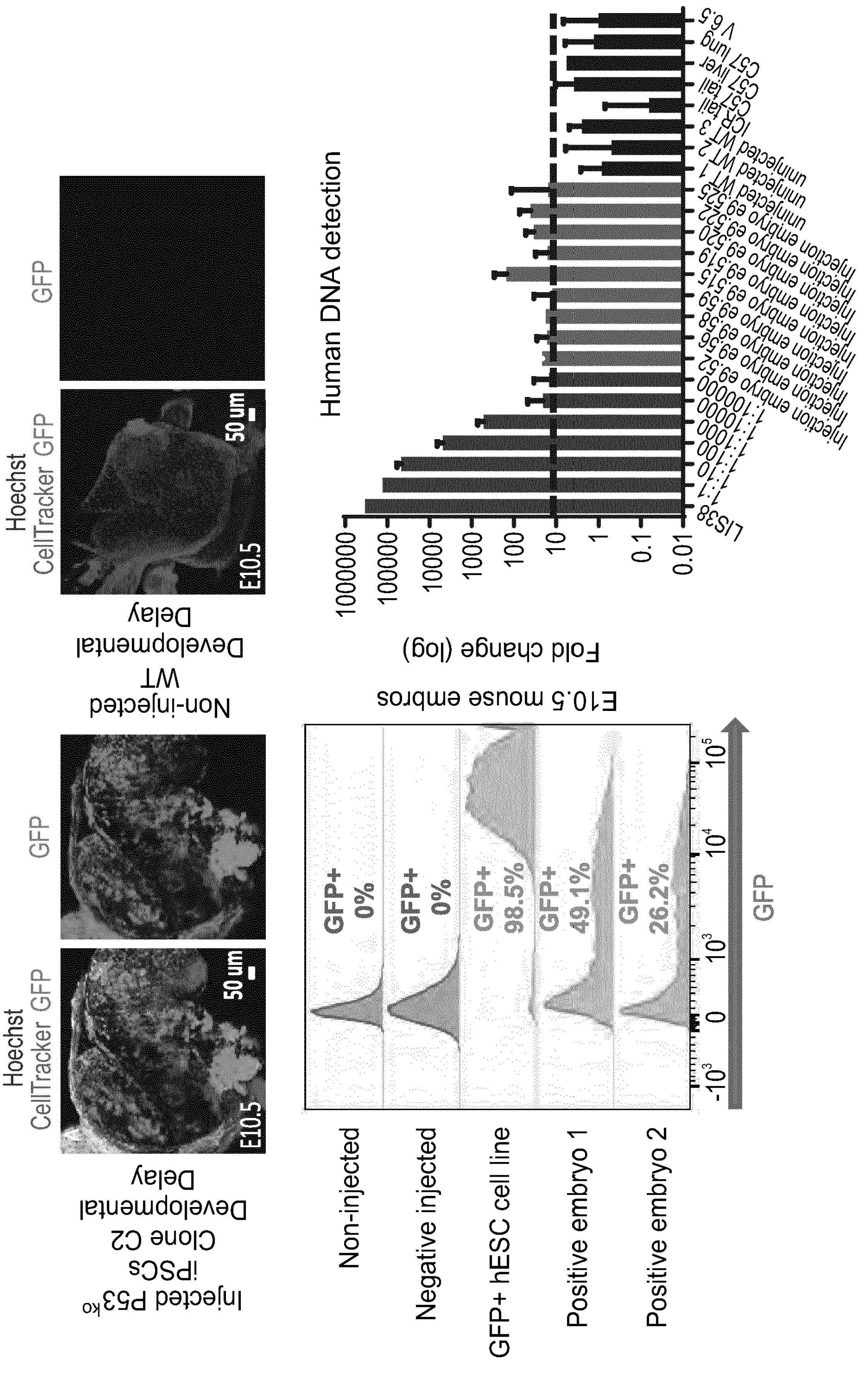

Injected P53ko iPSCs Clone C2 Developmental Delay
Hoechst CellTracker GFP
GFP
E10.5
50 um
Non-injected WT Developmental Delay
Hoechst CellTracker GFP
GFP
E10.5
50 um
Non-injected
Negative injected
GFP+ hESC cell line
Positive embryo 1
Positive embryo 2
GFP+ 0%
GFP+ 0%
GFP+ 98.5%
GFP+ 49.1%
GFP+ 26.2%
-10^3
0
10^3
10^4
10^5
GFP
E10.5 mouse embros
Human DNA detection
Fold change (log)
1000000
100000
10000
1000
100
10
1
0.1
0.01
LIS38
1:1
1:10
1:100
1:1000
1:10000
1:100000
Injection embryo e9.52
Injection embryo e9.56
Injection embryo e9.58
Injection embryo e9.59
Injection embryo e9.515
Injection embryo e9.519
Injection embryo e9.520
Injection embryo e9.522
Injection embryo e9.525
uninjected WT 1
uninjected WT 2
uninjected WT 3
ICR tail
C57 tail
C57 liver
C57 lung
V 6.5

FIG. 25

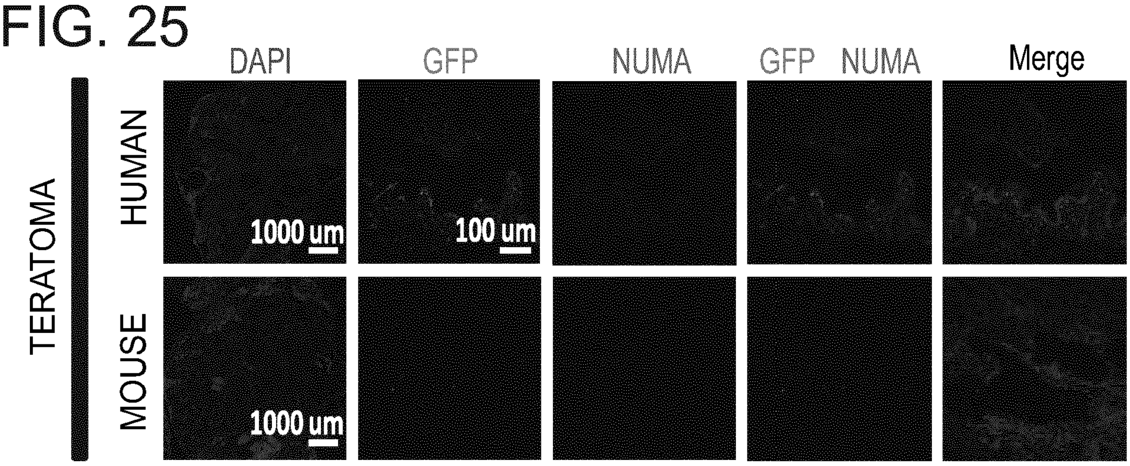
DAPI
GFP
NUMA
GFP NUMA
Merge
TERATOMA
HUMAN
MOUSE
1000 um
100 um
1000 um

FIG. 26

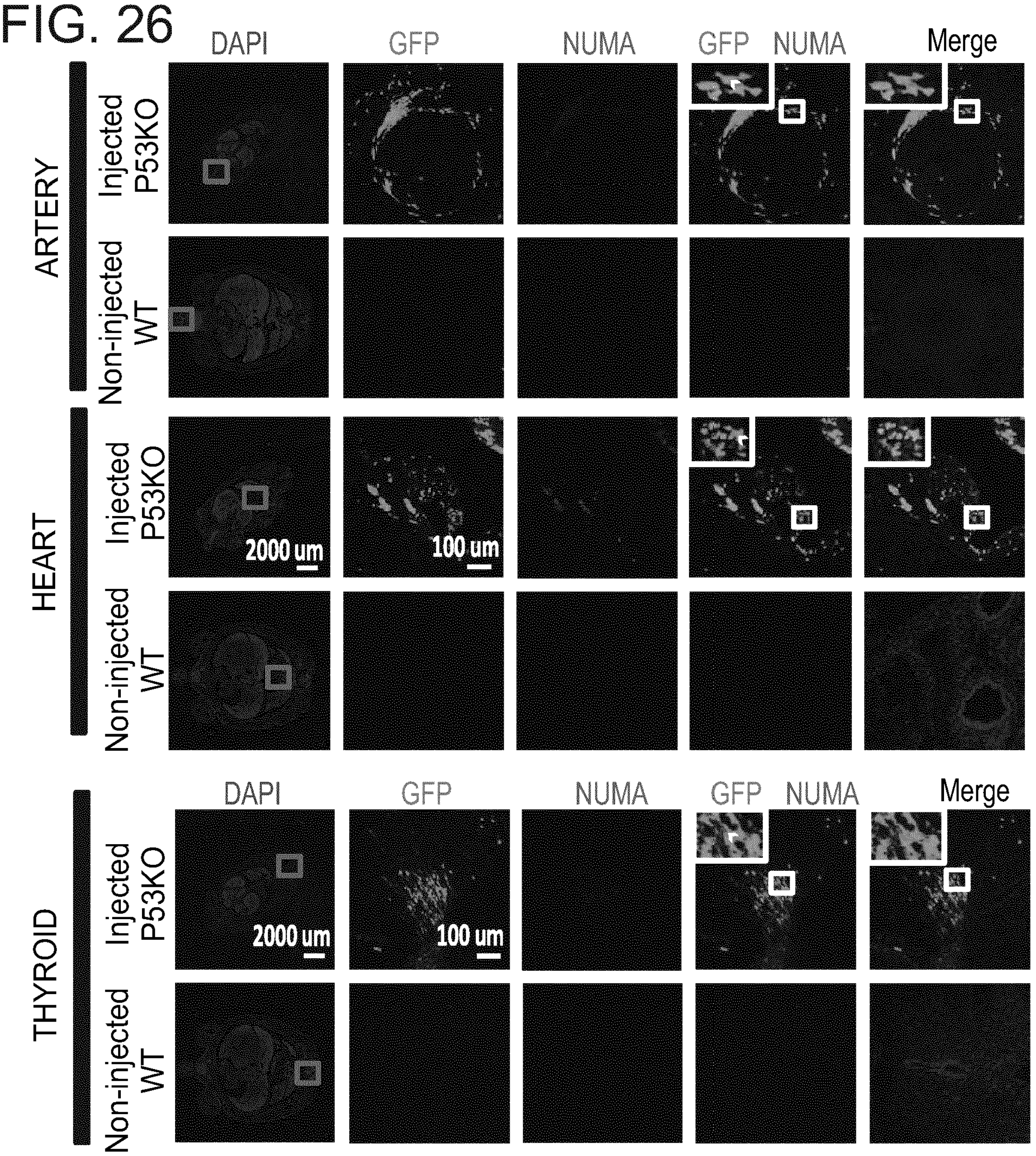
DAPI
GFP
NUMA
GFP NUMA
Merge
ARTERY
Injected P53KO
Non-injected WT
HEART
Injected P53KO
Non-injected WT
2000 um
100 um
THYROID
Injected P53KO
Non-injected WT
2000 um
100 um

FIG. 27

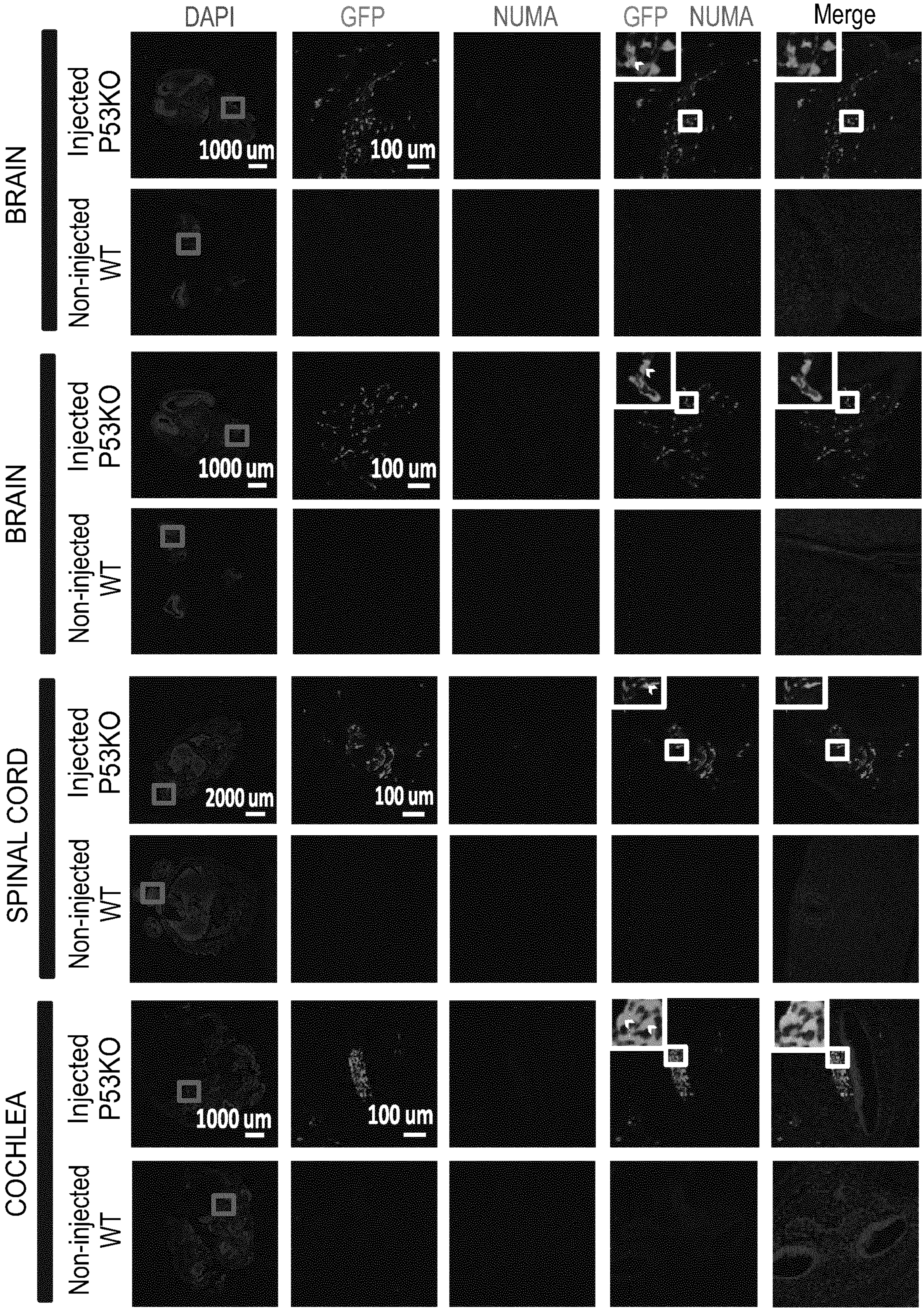
DAPI
GFP
NUMA
GFP NUMA
Merge
BRAIN
Injected P53KO
Non-injected WT
BRAIN
Injected P53KO
Non-injected WT
SPINAL CORD
Injected P53KO
Non-injected WT
COCHLEA
Injected P53KO
Non-injected WT
1000 um
100 um
1000 um
100 um
2000 um
100 um
1000 um
100 um

FIG. 28

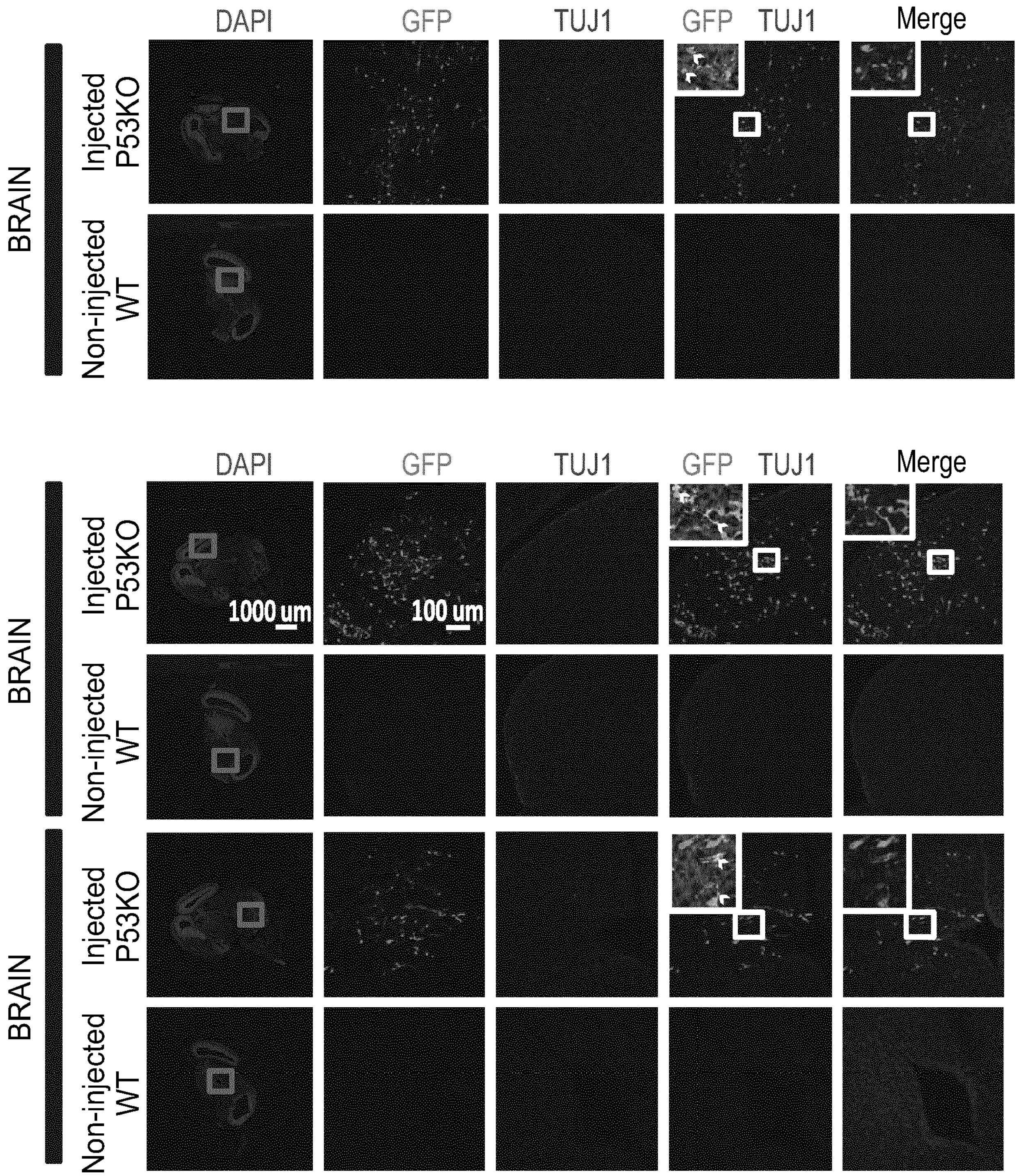
DAPI
GFP
TUJ1
GFP TUJ1
Merge
BRAIN
Injected P53KO
Non-injected WT
1000 um
100 um

FIG. 29

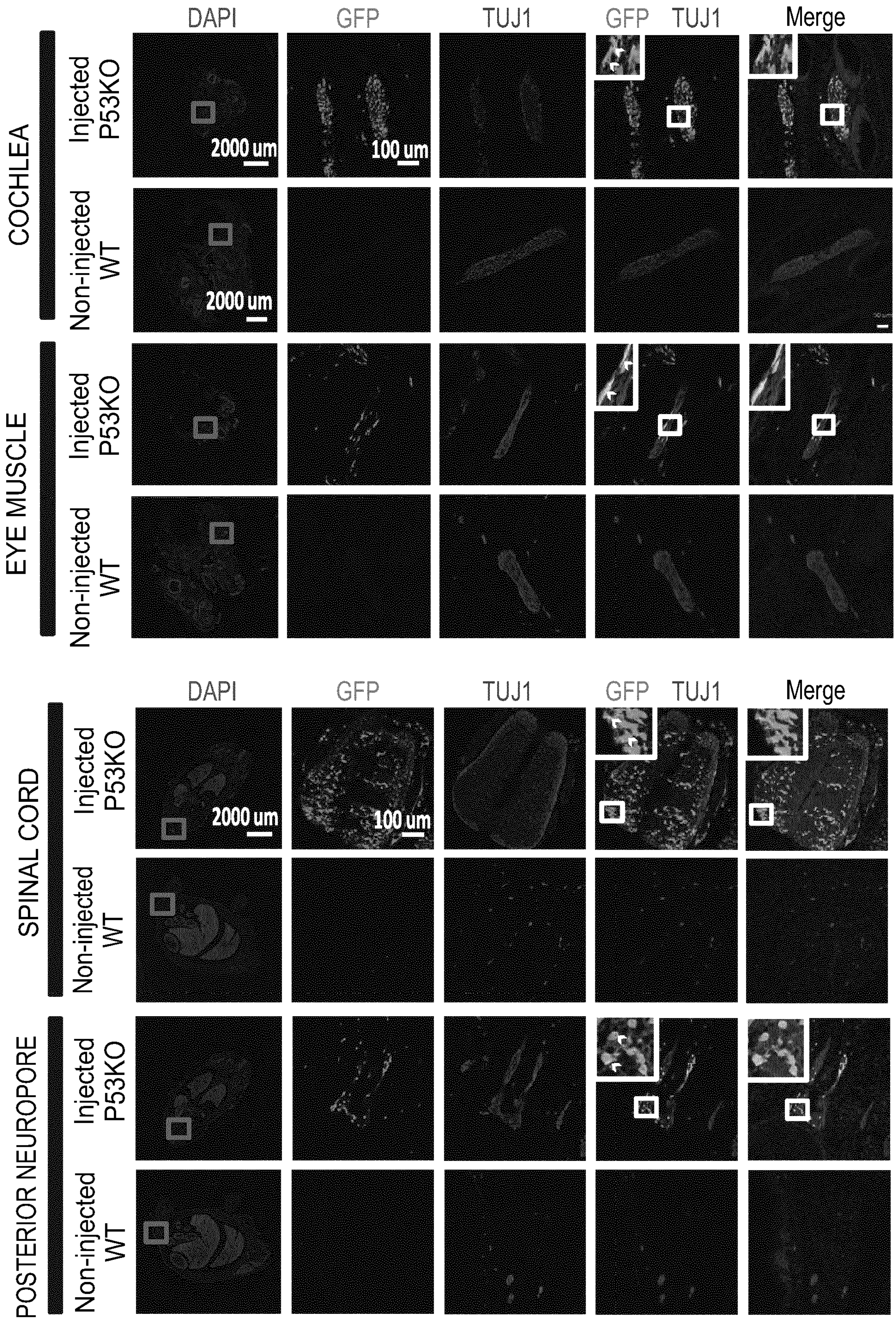
DAPI
GFP
TUJ1
GFP TUJ1
Merge
COCHLEA
Injected P53KO
Non-injected WT
EYE MUSCLE
Injected P53KO
Non-injected WT
2000 um
100 um
2000 um
DAPI
GFP
TUJ1
GFP TUJ1
Merge
SPINAL CORD
Injected P53KO
Non-injected WT
POSTERIOR NEUROPORE
Injected P53KO
Non-injected WT
2000 um
100 um

FIG. 30

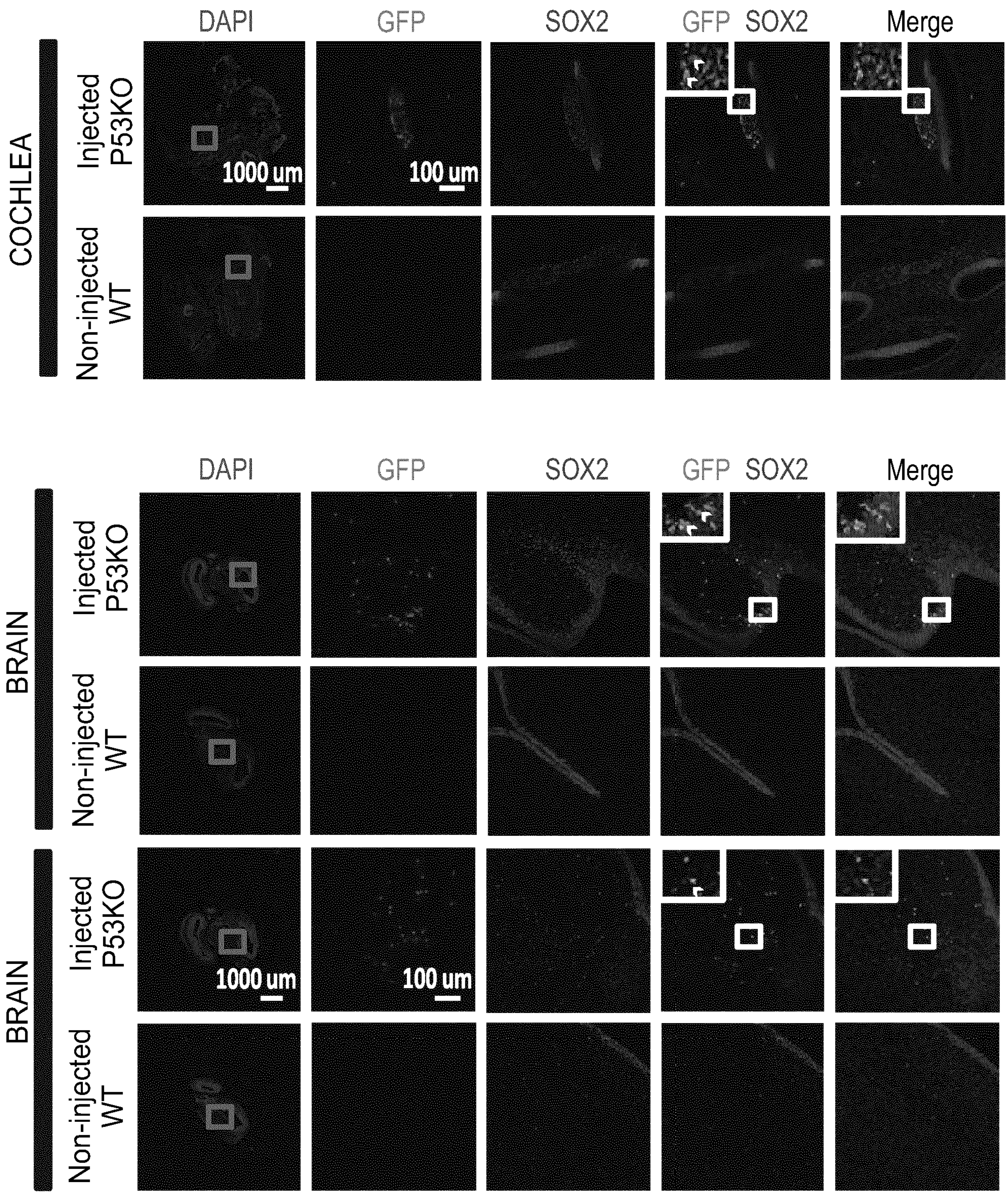
DAPI
GFP
SOX2
GFP SOX2
Merge
COCHLEA
Injected P53KO
Non-injected WT
1000 um
100 um
DAPI
GFP
SOX2
GFP SOX2
Merge
BRAIN
Injected P53KO
Non-injected WT
BRAIN
Injected P53KO
Non-injected WT
1000 um
100 um

FIG. 31

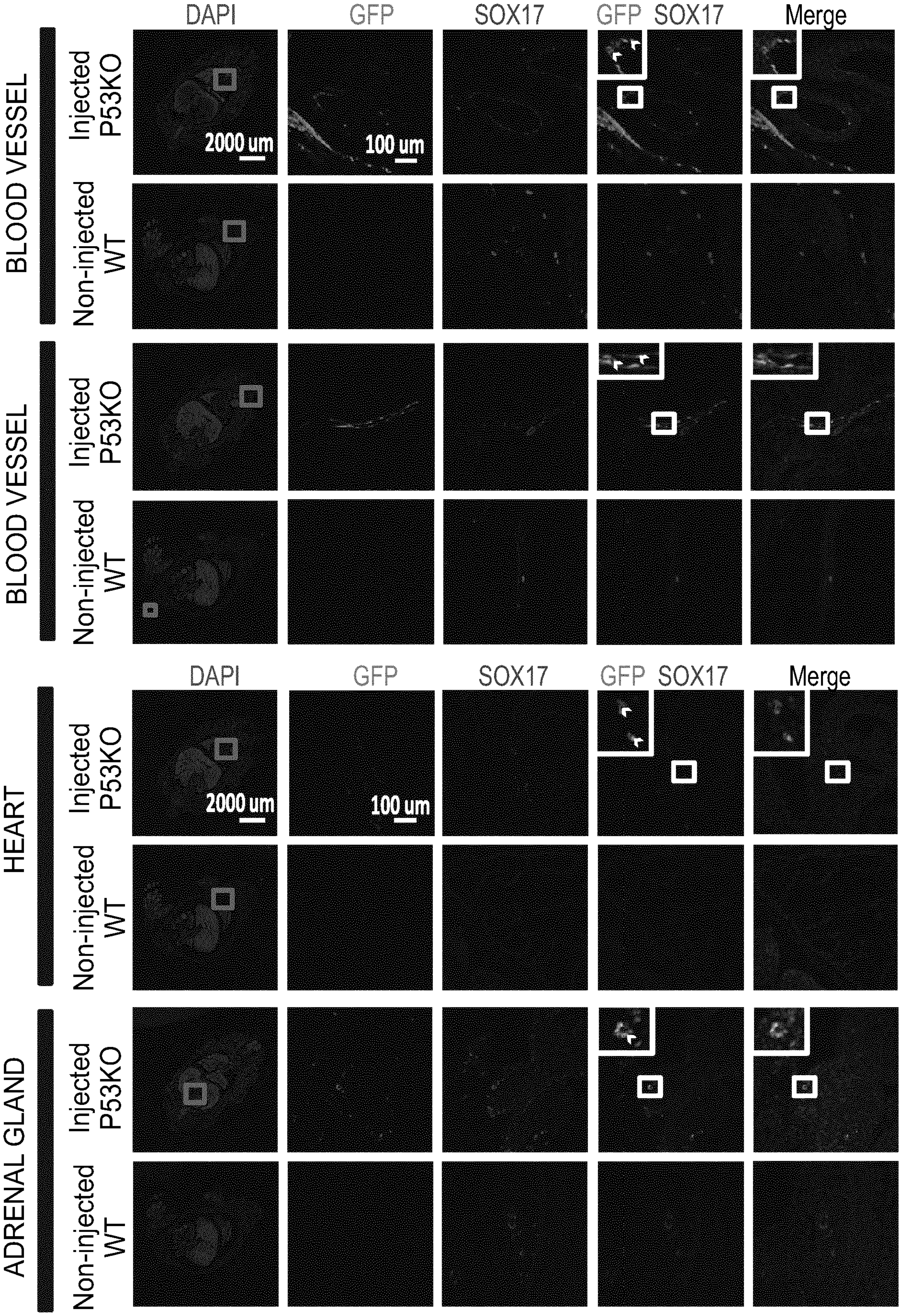
DAPI
GFP
SOX17
GFP SOX17
Merge
BLOOD VESSEL
Injected P53KO
Non-injected WT
2000 um
100 um
BLOOD VESSEL
Injected P53KO
Non-injected WT
DAPI
GFP
SOX17
GFP SOX17
Merge
HEART
Injected P53KO
Non-injected WT
2000 um
100 um
ADRENAL GLAND
Injected P53KO
Non-injected WT

FIG. 32

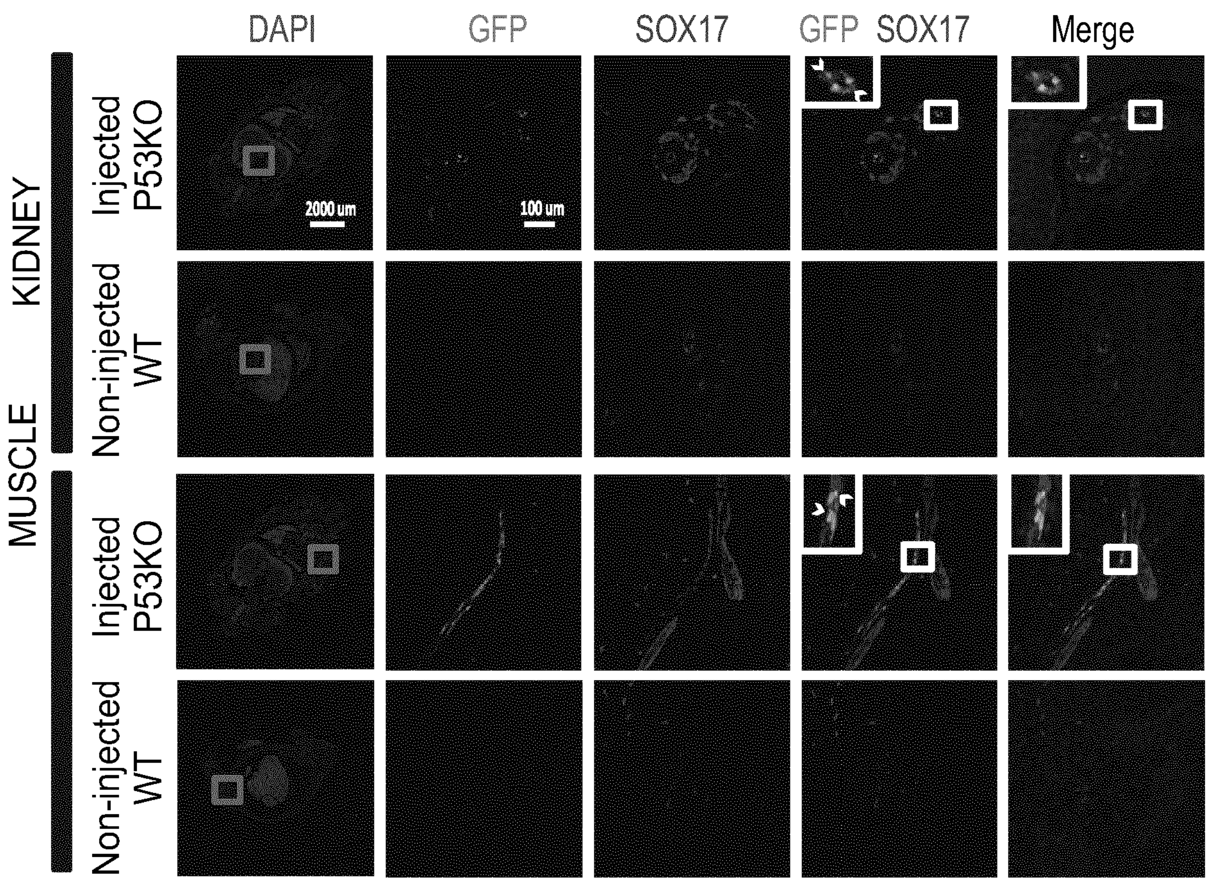
DAPI
GFP
SOX17
GFP SOX17
Merge
KIDNEY
MUSCLE
Injected P53KO
Non-injected WT
Injected P53KO
Non-injected WT
2000 um
100 um

FIG. 33

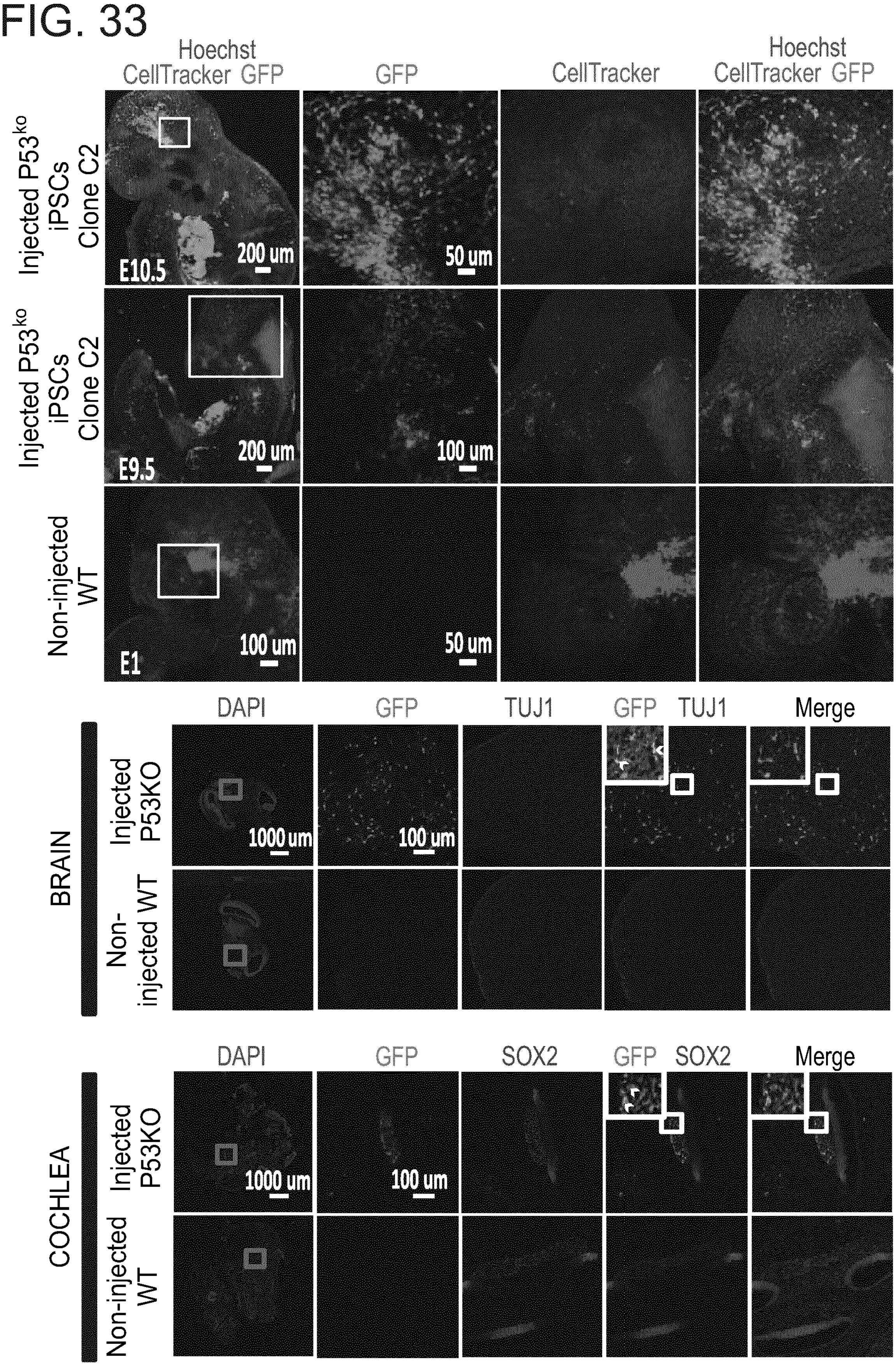
Hoechst CellTracker GFP
GFP
CellTracker
Hoechst CellTracker GFP
Injected P53ko iPSCs Clone C2
E10.5
200 um
50 um
Injected P53ko iPSCs Clone C2
E9.5
200 um
100 um
Non-injected WT
E1
100 um
50 um
DAPI
GFP
TUJ1
GFP TUJ1
Merge
BRAIN
Injected P53KO
1000 um
100 um
Non-injected WT
DAPI
GFP
SOX2
GFP SOX2
Merge
COCHLEA
Injected P53KO
1000 um
100 um
Non-injected WT

FIG. 34

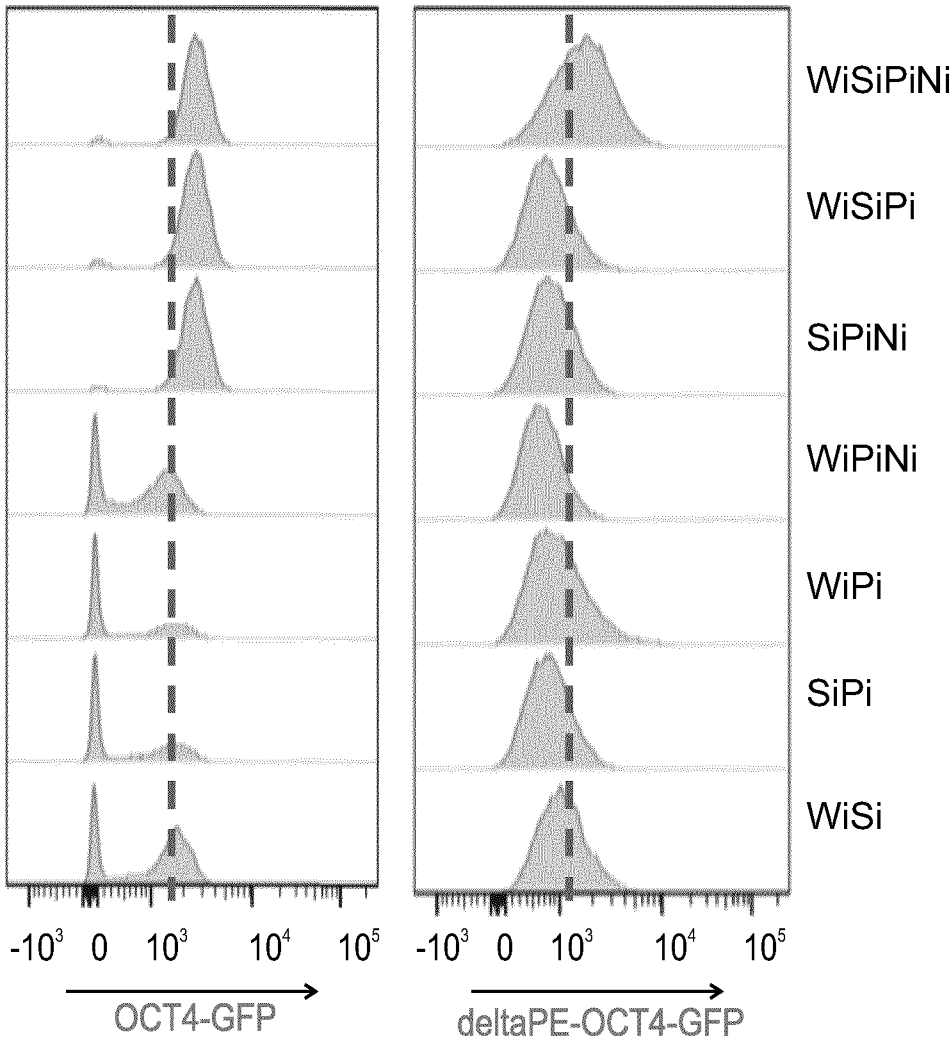
WiSiPiNi
WiSiPi
SiPiNi
WiPiNi
WiPi
SiPi
WiSi
-10³ 0 10³ 10⁴ 10⁵
OCT4-GFP
-10³ 0 10³ 10⁴ 10⁵
deltaPE-OCT4-GFP

FIG. 35

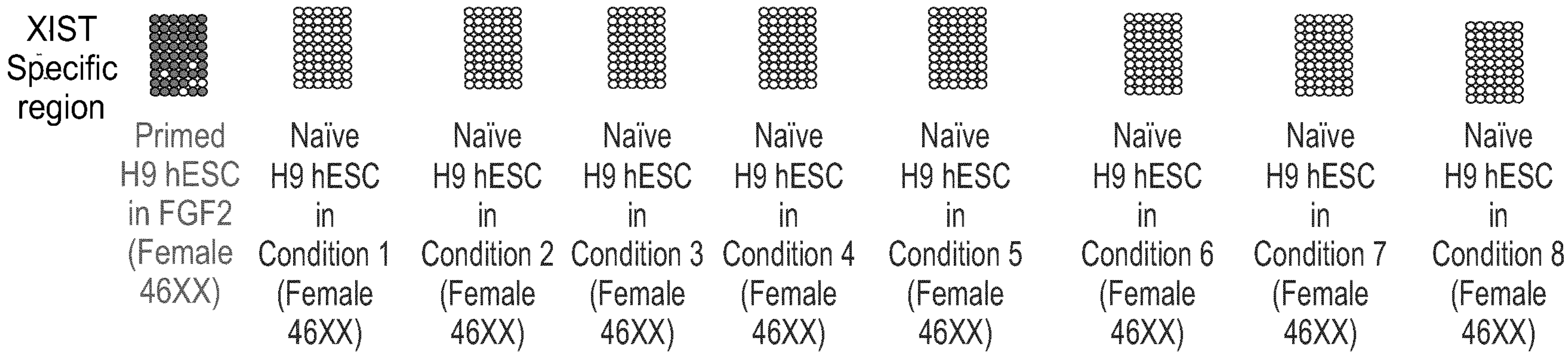
XIST
Specific
region
Primed
H9 hESC
in FGF2
(Female
46XX)
Naïve
H9 hESC
in
Condition 1
(Female
46XX)
Naïve
H9 hESC
in
Condition 2
(Female
46XX)
Naïve
H9 hESC
in
Condition 3
(Female
46XX)
Naïve
H9 hESC
in
Condition 4
(Female
46XX)
Naïve
H9 hESC
in
Condition 5
(Female
46XX)
Naïve
H9 hESC
in
Condition 6
(Female
46XX)
Naïve
H9 hESC
in
Condition 7
(Female
46XX)
Naïve
H9 hESC
in
Condition 8
(Female
46XX)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016016894 A
- WO 2014174470 A
- WO 0194341 A
- WO 0216352 A
- WO 0230924 A
- WO 0230926 A
- WO 0234744 A
- WO 02085895 A
- WO 02092577 A
- GB 0202117 W
- WO 02092578 A
- GB 0202124 W
- WO 02092579 A
- GB 0202128 W
- WO 03008409 A
- GB 0203177 W
- WO 03047584 A
- WO 03048159 A
- EP 02292736
- EP 03290900
- WO 9610028 A
- WO 9707131 A
- WO 9708193 A
- WO 9716452 A
- WO 9728161 A
- WO 9732879 A
- WO 9749706 A
- WO 02079192 A
- WO 03000188 A
- WO 03000266 A
- WO 03000705 A
- WO 02083668 A
- WO 02092573 A
- WO 03004492 A
- WO 0049018 A
- WO 03013541 A
- WO 0100207 A
- WO 0100213 A
- WO 0100214 A
- WO 2006040763 A
- US 5843780 A, Thomson
- US 6090622 A
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A


### Non-patent literature cited in the description


- **THEUNISSEN et al.** *Cell Stem Cell,* 2014, 1-17
- **THEUNISSEN.** *Cell Stem Cell,* 2016, 1-49
- **LIU et al.** *Nat. Methods,* 2017, vol. 14, 1-14
- **GUO et al.** *Stem Cell Reports,* 2016, 1-19
- **TAKASHIMA et al.** *Cell,* 2014, vol. 158, 1254-1269
- **THOROLD W. THEUNISSEN et al.** Systematic identification of Culture Conditions for Induction and Maintenance of Naive Human Pluripotency. *CELL STEM CELL,* 01 October 2014, vol. 15 (4
- **VORONKOV et al.** Wnt/beta-Catenin Signaling and Small Molecule Inhibitors. *CURRENT PHARMACEUTICAL DESIGN,* 01 December 2012, vol. 19, 634-664
- **MASAKI KINOSHITA et al.** Pluripotency Deconstructed. *DEVELOPMENT GROWTH AND DIFFERENTIATION.,* 01 January 2018, vol. 60 (1
- Establishment of porcine and human expanded potential stem cells. **GAO XUEFEI et al.** NATURE CELL BIOLOGY. MACMILLAN MAGAZINES LTD, vol. 21, 687-699
- **THEUNISSEN et al.** *Cell Stem Cell,* 2016
- **TAKASHIMA et al.** *Cell,* 2014

- **COLLIER et al.** *Cell Stem Cell,* 2017
- **SMITH et al.** *Nature,* 2014
- **GUO et al.** *Nature,* 2014
- **HURTADO et al.** *Scientific Reports,* 2019, vol. 9 (10811
- *Journal Medicinal Chemistry,* 2001, vol. 44, 822-833, 3965-3977
- *Cancer Research,* 2003, vol. 63, 375
- *J Bone Mineral Research,* 1999, vol. 14 (1), S487
- *Molecular Cell,* 1999, vol. 3, 639-647
- *Journal Medicinal Chemistry,* 1997, vol. 40, 2296-2303
- *Journal Medicinal Chemistry,* 1998, vol. 41, 3276-3292
- *Bioorganic & Medicinal Chemistry Letters,* 2002, vol. 12, 1361-3153
- *Molecular Cell,* 1999, vol. 3, 639-648
- *Journal Medicinal Chemistry,* 2002, vol. 45, 3394
- *Journal Medicinal Chemistry,* 2001, vol. 44, 1915
- *J. Pharmacol. Exp. Ther.,* 1997, vol. 283, 1433-1444
- *Mol. Biol. Cell,* 2000, vol. 11, 51-64
- *Biochem Pharmacol.,* 2000, vol. 60, 885-898
- *Cancer Research,* 1999, vol. 59, 6145-6152
- *CHEMICAL ABSTRACTS,* 133053-19-7
- *Science,* 1998, vol. 282, 1145
- *Curr. Top. Dev. Biol.,* 1998, vol. 38, 133
- *Proc. Natl. Acad. Sci. USA,* 1995, vol. 92, 7844
- **BONGSO et al.** *Hum Reprod,* 1989, vol. 4, 706
- **GARDNER et al.** *Fertil. Steril.,* 1998, vol. 69 (84
- **CHUNG et al.** *Cell Stem Cell,* 07 February 2008, vol. 2 (2), 113-117
- **DOETSCHMAN et al.** *Dev Biol.,* 1988, vol. 127, 224-7
- **IANNACCONE et al.** *Dev Biol.,* 1994, vol. 163, 288-92
- **GILES et al.** *Mol Reprod Dev.,* 1993, vol. 36, 130-8
- **GRAVES ; MOREADITH.** *Mol Reprod Dev.,* 1993, vol. 36, 424-33
- **NOTARIANNI et al.** *J Reprod Fertil Suppl.,* 1991, vol. 43, 255-60
- **WHEELER.** *Reprod Fertil Dev.,* 1994, vol. 6, 563-8
- **MITALIPOVA et al.** *Cloning,* 2001, vol. 3, 59-67
- **THOMSON et al.** *Proc Natl Acad Sci U S A.,* 1995, vol. 92, 7844-8
- **THOMSON et al.** *Biol Reprod.,* 1996, vol. 55, 254-9
- **SHAMBLOTT et al.** *Proc. Natl. Acad. Sci. USA,* 1998, vol. 95, 13726
- **YAMANAKA S.** *Cell Stem Cell,* 2007, vol. 1 (1), 39-49
- **AOI T et al.** Generation of Pluripotent Stem Cells from Adult Mouse Liver and Stomach Cells. *Science,* 14 February 2008
- **IH PARK ; ZHAO R ; WEST JA et al.** Reprogramming of human somatic cells to pluripotency with defined factors. *Nature,* 2008, vol. 451, 141-146
- **K TAKAHASHI ; TANABE K ; OHNUKI M et al.** Induction of pluripotent stem cells from adult human fibroblasts by defined factors. *Cell,* 2007, vol. 131, 861-872
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY.** Culture of Animal Cells - A Manual of Basic Technique. Wiley-Liss, 1994
- Current Protocols in Immunology. 1994, vol. I-III
- Basic and Clinical Immunology. Appleton & Lange, 1994
- Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** *A Practical Guide to Molecular Cloning,* 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **GAFNI et al.** Naive hiPSCs can contribute to interspecies chimaerism with highly variable and limited efficiency. *Nature,* 2013
- **BETSCHINGER, J. ; NICHOLS, J. ; DIETMANN, S. ; CORRIN, P.D. ; PADDISON, P.J. ; SMITH, A.** Exit from Pluripotency Is Gated by Intracellular Redistribution of the bHLH Transcription Factor Tfe3. *Cell,* 2013, vol. 153, 335-347

- **BRONS, I. ; CLARKSON, A. ; AHRLUND-RICHTER, L. ; PEDERSEN, R.A.** Derivation of pluripotent epiblast stem cells from mammalian embryos. *Nature,* 2007
- **CAREY, B.W. ; FINLEY, L.W.S. ; CROSS, J.R. ; ALLIS, C.D. ; THOMPSON, C.B.** Intracellular $\alpha$-ketoglutarate maintains the pluripotency of embryonic stem cells. *Nature,* 2015, vol. 518, 413-416
- **CHIA, N.-Y. ; CHAN, Y.-S. ; FENG, B. ; LU, X. ; ORLOV, Y.L. ; MOREAU, D. ; KUMAR, P. ; YANG, L. ; JIANG, J. ; LAU, M.-S. et al.** *A genome-wide RNAi screen reveals determinants of human embryonic stem cell identity,* 2010, vol. 468, 316-320
- **CHOI, J. ; HUEBNER, A.J. ; CLEMENT, K. ; WALSH, R.M. ; SAVOL, A. ; LIN, K. ; GU, H. ; DI STEFANO, B. ; BRUMBAUGH, J. ; KIM, S.Y. et al.** Prolonged Mek1/2 suppression impairs the developmental potential of embryonic stem cells. *Nature,* 2017, vol. 548, 219-223
- **COLLIER, A.J. ; PANULA, S.P. ; SCHELL, J.P. ; CHOVANEC, P. ; REYES, A.P. ; PETROPOULOS, S. ; CORCORAN, A.E. ; WALKER, R. ; DOUAGI, I. ; LANNER, F. et al.** Comprehensive Cell Surface Protein Profiling Identifies Specific Markers of Human Naive and Primed Pluripotent States. *Cell Stem Cell,* 2017, 1-41
- **GAFNI, O. ; WEINBERGER, L. ; MANSOUR, A.A. ; MANOR, Y.S. ; CHOMSKY, E. ; BEN-YOSEF, D. ; KALMA, Y. ; VIUKOV, S. ; MAZA, I. ; ZVIRAN, A. et al.** Derivation of novel human ground state naive pluripotent stem cells. *Nature,* 2013, vol. 504, 282-286
- **GEULA, S. ; MOSHITCH-MOSHKOVITZ, S. ; DOMINISSINI, D. ; MANSOUR, A.A. ; KOL, N. ; HERSHKOVITZ, V. ; PEER, E. ; MOR, N. ; MANOR, Y.S. ; BEN-HAIM, M.S. et al.** m6 mRNA methylation facilitates resolution of naive pluripotence toward differentiation. *Science,* 2015, vol. 3, 1002-1006
- **GEULA, S. ; MOSHITCH-MOSHKOVITZ, S. ; DOMINISSINI, D. ; MANSOUR, A.A.F. ; KOL, N. ; SALMON-DIVON, M. ; HERSHKOVITZ, V. ; PEER, E. ; MOR, N. ; MANOR, Y.S. et al.** m6A mRNA methylation facilitates resolution of naive pluripotency toward differentiation. *Science,* 2015, vol. 347, 1002-1006
- **GUO, G. ; VON MEYENN, F. ; SANTOS, F. ; CHEN, Y. ; REIK, W. ; BERTONE, P. ; SMITH, A. ; NICHOLS, J.** Naive Pluripotent Stem Cells Derived Directly from Isolated Cells of the Human Inner Cell Mass. *Stem Cell Reports,* 2016, 1-19
- **GUO, G. ; VON MEYENN, F. ; ROSTOVSKAYA, M. ; CLARKE, J. ; DIETMANN, S. ; BAKER, D. ; SAHAKYAN, A. ; MYERS, S. ; BERTONE, P. ; REIK, W. et al.** Epigenetic resetting of human pluripotency. *Development,* 2017, vol. 144, 2748-2763
- **HACKETT, J.A. ; SURANI, M.A.** Regulatory principles of pluripotency: from the ground state up. *Cell Stem Cell,* 2014, vol. 15, 416-430
- **HANNA, J. ; MARKOULAKI, S. ; MITALIPOVA, M. ; CHENG, A.W. ; CASSADY, J.P. ; STAERK, J. ; CAREY, B.W. ; LENGNER, C.J. ; FOREMAN, R. ; LOVE, J. et al.** Metastable Pluripotent States in NOD-Mouse-Derived ESCs. *Cell Stem Cell,* 2009, vol. 4, 513-524
- **HANNA, J.H. ; SAHA, K. ; JAENISCH, R.** Pluripotency and cellular reprogramming: Facts, hypotheses, unresolved issues. *Cell,* 2010, vol. 143, 508-525
- **HAYASHI, K. ; OHTA, H. ; KURIMOTO, K. ; ARAMAKI, S. ; SAITOU, M.** Reconstitution of the mouse germ cell specification pathway in culture by pluripotent stem cells. *Cell,* 2011, vol. 146, 519-532
- **IRIE, N. ; WEINBERGER, L. ; TANG, W.W.C. ; KOBAYASHI, T. ; VIUKOV, S. ; MANOR, Y.S. ; DIETMANN, S. ; HANNA, J.H. ; SURANI, M.A.** SOX17 is a critical specifier of human primordial germ cell fate. *Cell,* 2015, vol. 160, 253-268
- **KIM, H. ; WU, J. ; YE, S. ; TAI, C.-I. ; ZHOU, X. ; YAN, H. ; LI, P. ; PERA, M. ; YING, Q.-L.** Modulation of $\beta$-catenin function maintains mouse epiblast stem cell and human embryonic stem cell self-renewal. *Nat. Commun.,* 2013, vol. 4, 2403
- **KOJIMA, Y. ; KAUFMAN-FRANCIS, K. ; STUDDERT, J.B. ; STEINER, K.A. ; POWER, M.D. ; LOEBEL, D.A.F. ; JONES, V. ; HOR, A. ; DE ALENCASTRO, G. ; LOGAN, G.J. et al.** The transcriptional and functional properties of mouse epiblast stem cells resemble the anterior primitive streak. *Cell Stem Cell,* 2014, vol. 14, 107-120
- **LIAO, J. ; KARNIK, R. ; GU, H. ; ZILLER, M.J. ; CLEMENT, K. ; TSANKOV, A.M. ; AKOPIAN, V. ; GIFFORD, C.A. ; DONAGHEY, J. ; GALONSKA, C. et al.** Targeted disruption of DNMT1, DNMT3A and DNMT3B in human embryonic stem cells. *Nat. Genet.,* 2015
- **LIU, X. ; NEFZGER, C.M. ; ROSSELLO, F.J. ; CHEN, J. ; KNAUPP, A.S. ; FIRAS, J. ; FORD, E. ; PFLUEGER, J. ; PAYNTER, J.M. ; CHY, H.S. et al.** Comprehensive characterization of distinct states of human naive pluripotency generated by reprogramming. *Nat. Methods,* 2017, vol. 14, 1-14
- **MARKS, H. ; KALKAN, T. ; MENAFRA, R. ; DENISSOV, S. ; JONES, K. ; HOFEMEISTER, H. ; NICHOLS, J. ; KRANZ, A. ; FRANCIS STEWART, A. ; SMITH, A. et al.** The transcriptional and epigenomic foundations of ground state pluripotency. *Cell,* 2012, vol. 149, 590-604
- **NICHOLS, J. ; SMITH, A.** Naive and Primed Pluripotent States. *Cell Stem Cell,* 2009, vol. 4, 487-492
- **PASTOR, W.A. ; LIU, W. ; CHEN, D. ; HO, J. ; KIM, R. ; HUNT, T.J. ; LUKIANCHIKOV, A. ; LIU, X. ; POLO, J.M. ; JACOBSEN, S.E. et al.** TFAP2C regulates transcription in human naive pluripotency by opening enhancers. *Nat. Cell Biol.,* 2018

- **RAIS, Y. ; ZVIRAN, A. ; GEULA, S. ; GAFNI, O. ; CHOMSKY, E. ; VIUKOV, S. ; MANSOUR, A.A. ; CASPI, I. ; KRUPALNIK, V. ; ZERBIB, M. et al.** Deterministic direct reprogramming of somatic cells to pluripotency. *Nature,* 2013, vol. 502, 65-70
- **SAHAKYAN, A. ; KIM, R. ; CHRONIS, C. ; SABRI, S. ; BONORA, G. ; THEUNISSEN, T.W. ; KUOY, E. ; LANGERMAN, J. ; CLARK, A.T. ; JAENISCH, R. et al.** Human Naive Pluripotent Stem Cells Model X Chromosome Dampening and X Inactivation. *Cell Stem Cell,* 2016, 1-36
- **SHAKIBA, N. ; WHITE, C.A. ; LIPSITZ, Y.Y. ; YACHIE-KINOSHITA, A. ; TONGE, P.D. ; HUSSEIN, S.M.I. ; PURI, M.C. ; ELBAZ, J. ; MORRISSEY-SCOOT, J. ; LI, M. et al.** CD24 tracks divergent pluripotent states in mouse and human cells. *Nat. Commun.,* 2015, 6
- **TAKASHIMA, Y. ; GUO, G. ; LOOS, R. ; NICHOLS, J. ; FICZ, G. ; KRUEGER, F. ; OXLEY, D. ; SANTOS, F. ; CLARKE, J. ; MANSFIELD, W. et al.** Resetting Transcription Factor Control Circuitry toward Ground-State Pluripotency in Human. *Cell,* 2014, vol. 158, 1254-1269
- **TESAR, P.J. ; CHENOWETH, J.G. ; BROOK, F.A. ; DAVIES, T.J. ; EVANS, E.P. ; MACK, D.L. ; GARDNER, R.L. ; MCKAY, R.D.G.** *New cell lines from mouse epiblast share defining features with human embryonic stem cells,* 2007, vol. 448, 196-199
- **THEUNISSEN, T.W.** *Molecular Criteria for Defining the Naive Human Pluripotent State,* 2016, 1-50
- **THEUNISSEN, T.W. ; POWELL, B.E. ; WANG, H. ; MITALIPOVA, M. ; FADDAH, D.A. ; REDDY, J. ; FAN, Z.P. ; MAETZEL, D. ; GANZ, K. ; SHI, L. et al.** Systematic Identification of Defined Conditions for Induction and Maintenanceof Naive Human Pluripotency. *Cell Stem Cell,* 2014, 1-17
- **THEUNISSEN, T.W. ; POWELL, B.E. ; WANG, H. ; MITALIPOVA, M. ; FADDAH, D.A. ; REDDY, J. ; FAN, Z.P. ; MAETZEL, D. ; GANZ, K. ; SHI, L. et al.** Systematic Identification of Culture Conditions for Induction and Maintenance of Naive Human Pluripotency. *Cell Stem Cell,* 2014, vol. 15, 471-487
- **THEUNISSEN, T.W. ; FRIEDLI, M. ; HE, Y. ; PLANET, E. ; O'NEIL, R.C. ; MARKOULAKI, S. ; PONTIS, J. ; WANG, H. ; IOURANOVA, A. ; IMBEAULT, M. et al.** Molecular Criteria for Defining the Naive Human Pluripotent State. *Cell Stem Cell,* 2016, 1-49
- **WEINBERGER, L. ; AYYASH, M. ; NOVERSHTERN, N. ; HANNA, J.H.** Dynamic stem cell states: Naive to primed pluripotency in rodents and humans. *Nat. Rev. Mol. Cell Biol.,* 2016, vol. 17, 155-169
- **YANG, Y. ; LIU, B. ; XU, J. ; WANG, J. ; WU, J. ; SHI, C. ; XU, Y. ; DONG, J. ; WANG, C. ; LAI, W. et al.** Derivation of Pluripotent Stem Cells with In~Vivo Embryonic and Extraembryonic Potency. *Cell,* 2017, vol. 169, 243-257
- **YING, Q.-L. ; WRAY, J. ; NICHOLS, J. ; BATLLE-MORERA, L. ; DOBLE, B. ; WOODGETT, J. ; COHEN, P. ; SMITH, A.** *The ground state of embryonic stem cell self-renewal,* 2008, vol. 453, 519-523
- **ZIMMERLIN, L. ; PARK, T.S. ; HUO, J.S. ; VERMA, K. ; PATHER, S.R. ; TALBOT JR, C.C. ; AGARWAL, J. ; STEPPAN, D. ; ZHANG, Y.W. ; CONSIDINE, M. et al.** Tankyrase inhibition promotes a stable human naiive pluripotent state with improved functionality. *Development,* 2016